# EUROPEAN PATENT SPECIFICATION

(11) **EP 2 723 773 B1**
(45) Date of publication and mention of the grant of the patent: **28.02.2018**
(21) Application number: 12728091.5
(22) Date of filing: 19.06.2012
(51) Int. Cl.: C07K 14/74, C07K 16/30, C07K 14/005, C07K 16/28, C12P 21/00

(54) **REMOVAL OF TARGET CELLS BY CIRCULATING VIRUS-SPECIFIC CYTOTOXIC T-CELLS USING MHC CLASS I COMPRISING COMPLEXES**
TÖTEN VON TARGETZELLEN MITTELS ZIRKULATION VIRENSPEZIFISCHER CYTOTOXISCHER T-ZELLEN MIT MHC-KLASSE-I-HALTIGEN KOMPLEXEN
ÉLIMINATION DES CELLULES CIBLES PAR LA CIRCULATION DE LYMPHOCYTES T CYTOTOXIQUES SPÉCIFIQUES AU VIRUS À L'AIDE DE PROTÉINES DE FUSION CHIMÈRIQUES COMPRENANT UNE MOLÉCULE DU CMH DE CLASSE I

(30) Priority: 22.06.2011 EP 11171027
(43) Date of publication of application: 30.04.2014
(73) Proprietor: F. Hoffmann-La Roche AG, 4070 Basel (CH)
(72) Inventor: KNOETGEN, Hendrik, 79650 Schopfheim (DE)
(74) Representative: Burger, Alexander
(86) International application number: PCT/EP2012/061734
(87) International publication number: WO 2012/175508

(56) References cited:
- WO-A1-99/64597
- WO-A2-2005/099361
- US-A1- 2004 091 488
- US-B2- 7 521 197

## Description

Herein is reported a fusion polypeptide comprising an antibody and a MHC class I component and its use for removal of tumor cells by targeted attraction of circulating virus-specific cytotoxic T-cells.

### Background of the Invention

The MHC Class I protein consists of an α-chain (α-1 to 3 and a transmembrane domain) and β2-microglobulin. It is polygenic (3 gene loci for MHC-class I protein in the haploid genome) giving rise to six different MHC class I protein α-chains (in humans two HLA-A, two HLA-B, two HLA-C). The MHC is further polymorphic. The human HLA-A allele A*0201 is prevalent in about 30 % to 50 % of the caucasian population (Player, et al., J Immunother. Emphasis Tumor Immunol. 19 (1996) 357-363).

Human cytomegalovirus HCMV (= Human herpesvirus 5, HHV-5) is one of the largest human viruses. Its genome comprises around 230,000 bp linear double stranded DNA and encodes more than 160 proteins (Davison, A.J., et al., J. Gen. Virol. 84 (2003) 17-28).

The CMV has evolved to become a sublime parasite of the human genome and it is a potent immunogen and triggers strong immune responses from all arms of the immune system. This virus appears to be among the most immunodominant antigens known to the human immune system and stimulates CD8⁺-T-cell responses of unprecedented magnitude.

The CMV "latency" depends on chronic immune suppression of CMV viruses rather than a change in the pattern of viral transcription (Moss & Khan, Human Immunology 65 (2004) 456-464).

CD8⁺-T-cell immune responses are not directed evenly against all CMV proteins but are focused. The CMV proteins pp65 and IE-1 are the predominant targets (McLaughlin-Taylor, E., et al., J. Med. Virol. 43 (1994) 103-110; Moss & Khan, Human Immunology 65 (2004) 456-464).

The true frequency of CMV-specific T-cells is very high with frequencies for individual peptides in the order of up to 1 to 2 % of the total CD8⁺-T-cell repertoire (Moss & Khan, Human Immunology supra; Wills, M.R., et al., J. Virol. 70 (1996) 7569-7579).

The CMV-specific CD8⁺-T-cell response increases markedly with age and individual HLA-peptide tetramers frequently stain in excess of 10 % of the total CD8⁺-T-cell pool (Khan, N., et al., J. Immunol. 169 (2002) 1984-1992).

The total CD8⁺-T-cell response in healthy elderly donors could constitute approximately 50 % of the CD8⁺-T-cell repertoire.

The enormous CD8⁺-T-cell expansions are often very clonally restricted, and it is estimated that CMV is the cause of at least 30 % of the clonal CD8⁺-T-cell expansions that are seen in peripheral blood with aging. The total CD8⁺-T-cell count is twice as high in CMV-seropositive donors older than age 60 years in comparison to a CMV-seronegative cohort (Looney, R.J., et al., Clin. Immunol. 90 (1999) 213-219).

A fusion of soluble HLA and β-2-microglobulin is reported by Mottez et al. (Eur. J. Immunol. 21 (1991) 467-471); Godeau et al. (J. Biol. Chem. 267 (1992) 24223-24229) and Mage et al. (Proc. Natl. Acad. Sci. 89 (1992) 10658-10662). A fusion of viral-derived peptide with soluble HLA and β-2-microglobulin is reported by Mottez et al. (J. Exp. Med. 181 (1995) 493-502). A fusion of an immunoglobulin heavy chain with soluble HLA and co-expressed β-2-microglobulin is reported by Dal Porto et al. (Proc. Natl. Acad. Sci. USA 90 (1993) 6671-6675). A tetrameric complex of biotinylated peptide-soluble HLA and β-2-microglobulin with streptavidin chemically coupled to a Fab is described by Robert et al. (Eur. J. Immun. 30 (2000) 3165-3170). A chemically coupled Fab with a fusion of viral-derived peptide with soluble HLA and β-2-microglobulin is reported by Robert et al. (Cancer Immunity 1 (2001) 2). A fusion of a viral-derived peptide with soluble HLA and β-2-microglobulin to a murine monoclonal antibody heavy chain is reported by Greten et al. (J. Immunol. Methods 271 (2002) 125-135). An E. coli expression of scFv fusions without peptide, in vitro refolding and peptide loading is reported by Lev et al. (J. Immunol. 169 (2002) 2988-2996), Lev et al. (Proc. Natl. Acad. Sci. 101 (2004) 9051-9056), and Novak et al. (Int. J. Cancer 120 (2006) 329-336). The use of biotinylated soluble MHC loaded with peptides and coupled to streptavidin fused Fab or scFv antibodies is reported by Mous et al. (Leukemia 20 (2006) 1096-1102).

In WO 2005/099361 are reported MHC class I - peptide-antibody conjugates with modified beta-2-microglobulin. Exemplary conjugates as reported in WO 2005/099361 are obtained by in vitro conjugation of the alpha chain of the MHC-complex (HLA) or by the co-expression from separate genes in the same cell.

In US 2004/0091488 antigenic constructs of major histocompatibility complex class I antigens with specific carrier molecules is reported. Herein fusion polypeptides are reported lacking a hinge region.

### Summary of the Invention

Herein is reported a method for recombinantly producing a complex comprising as first part an antibody derived part that specifically binds to a target antigen, and as second part a virus-derived peptide covalently linked to a MHC class I protein complex as well as the complex itself.

With the complex as reported herein existing virus-specific circulating cytotoxic T-cells (T-memory-cells and/or T-effector-cells) of an individual can be directed to cells expressing the target antigen, to which the antibody derived part of the complex specifically binds to, by dressing these cells with a MHC class I complexes mimicking an acute viral infection by the virus-derived peptide linked to the MHC class I protein complex.

One aspect of the invention is a method for the recombinant production of a complex comprising i) a fusion polypeptide comprising in N- to C-terminal direction a T-cell response eliciting peptide, β2-microglobulin and the extracellular domains α1, α2, and α3 of a class I MHC molecule with a relative frequency of 1 % or more; ii) a pair of disulfide-linked polypeptide chains derived from an antibody hinge region, and wherein each disulfide-linked polypeptide chain comprises a CH2 domain and a CH3 domain of human origin, wherein one CH3 domain comprises the mutation T366W and the other CH3 domain comprises the mutations T366S, L368A and Y407V (numberings according to EU index of Kabat); and iii) at least one pair of an antibody light chain variable domain and an antibody heavy chain variable domain in a eukaryotic cell. The method comprises the following steps: (i) cultivating a eukaryotic cell comprising one or more nucleic acids encoding the complex, and (ii) recovering the complex from the cell or the cultivation medium. According to the invention the complex comprises exactly one fusion polypeptide of a T-cell response eliciting peptide, β2-microglobulin and the extracellular domains α1, α2, and α3 of a class I MHC molecule. Within the complex the fusion polypeptide is (a) covalently bound either to the C-terminus or the N-terminus of one of the disulfide-linked polypeptide chains, or (b) covalently bound to the N-terminus of an antibody variable domain that is the complementary heavy or light chain variable domain to that comprised in the first disulfide-linked polypeptide chain, or (c) covalently bound to the C-terminus of an antibody constant domain that is the complementary heavy or light chain constant domain to that comprised in the first disulfide-linked polypeptide chain.

In one embodiment the complex comprises exactly one MHC-derived polypeptide or exactly one fusion polypeptide comprising an MHC-derived molecule.

In one embodiment the complex is obtained with a concentration of 1 mg/ml or more in the cultivation medium. In one embodiment the complex is obtained with a concentration of 4 mg/ml or more in the cultivation medium.

In one embodiment the eukaryotic cell is a mammalian cell. In one embodiment the mammalian cell is a human embryonic kidney cell, or a chinese hamster ovary cell, or a baby hamster kidney cell, or a mouse myeloma cell.

In one embodiment the polypeptides of the pair of disulfide-linked polypeptide chains derived from an antibody hinge region i) are linked by one or more disulfide bonds, ii) the first disulfide-linked polypeptide chain comprises in N- to C-terminal direction an immunoglobulin light or heavy chain variable domain, an immunoglobulin light or heavy chain constant domain, and an antibody heavy chain hinge region polypeptide, and the second disulfide-linked polypeptide chain comprises an antibody heavy chain hinge region polypeptide.

In one embodiment the T-cell response eliciting peptide is a virus-derived peptide.

In one embodiment the fusion polypeptide comprises in N- to C-terminal direction
(i) a virus-derived peptide that has an amino acid sequence selected from SEQ ID NO: 01 to SEQ ID NO: 09,
(ii) a first linker peptide that has an amino acid sequence selected from SEQ ID NO: 16, 17, 18, 21, 22, and 23,
(iii) a β2-microglobulin that has an amino acid sequence of SEQ ID NO: 10,
(iv) a second linker peptide that has an amino acid sequence selected from SEQ ID NO: 16, 17, 18, 21, 22, and 23,
(v) the extracellular domains α1, α2 and α3 of a class I MHC molecule that has an amino acid sequence of SEQ ID NO: 11, and
(vi) a third linker peptide that has an amino acid sequence selected from SEQ ID NO: 12, 16, 17, 18, 21, 22, and 23.

In one embodiment the first disulfide-linked polypeptide chain and the second disulfide-linked polypeptide chain comprise i) a human IgG1 CH2 domain comprising an amino acid sequence selected from SEQ ID NO: 31, 32, and 33, and a human IgG1 CH3 domain comprising an amino acid sequence selected from SEQ ID NO: 34, 35, and 36.

In one embodiment the complex comprises i) a first linker peptide that has the amino acid sequence of SEQ ID NO: 21, and/or ii) a second linker peptide that has the amino acid sequence of SEQ ID NO: 22, and/or iii) a third linker peptide that has the amino acid sequence of SEQ ID NO: 12, and/or iv) a human IgG1 CH2 domain that has the amino acid sequence of SEQ ID NO: 32 or 33, and/or v) in the first disulfide-linked polypeptide a human IgG1 CH3 domain that has the amino acid sequence of SEQ ID NO: 35 and in the second disulfide-linked polypeptide a human IgG1 CH3 domain that has the amino acid sequence of SEQ ID NO: 36.

One aspect of the invention is a complex, characterized in that it comprises
- one fusion polypeptide that comprises in N- to C-terminal direction
   (i) a T-cell response eliciting peptide,
   (ii) a β2-microglobulin, and
   (iii) the extracellular domains α1, α2, and α3 of a class I MHC molecule with a relative frequency of 1 % or more,
   and
- two polypeptide chains, which are linked by one or more disulfide bonds,
   wherein the first disulfide-linked polypeptide chain comprises in N- to C-terminal direction
   (i) an immunoglobulin light or heavy chain variable domain,
   (ii) an immunoglobulin light or heavy chain constant domain, and
   (iii) an antibody heavy chain hinge region polypeptide,
   and
   wherein the second disulfide-linked polypeptide chain comprises an antibody heavy chain hinge region polypeptide, wherein the first disulfide-linked polypeptide chain and the second disulfide-linked polypeptide chain both comprise a CH2 domain and a CH3 domain of human origin, wherein one CH3 domain comprises the mutation T366W and the other CH3 domain comprises the mutations T366S, L368A and Y407V (numberings according to EU index of Kabat);
wherein the fusion polypeptide is
- covalently bound either to the C-terminus or the N-terminus of one of the disulfide-linked polypeptide chains, or
- covalently bound to the N-terminus of an antibody variable domain that is the complementary heavy or light chain variable domain to that comprised in the first disulfide-linked polypeptide chain, or
- covalently bound to the C-terminus of an antibody constant domain that is the complementary heavy or light chain constant domain to that comprised in the first disulfide-linked polypeptide chain
with the proviso that the complex comprises exactly one fusion polypeptide.

In one embodiment of all aspects the complex is an antigen binding complex.

In one embodiment of all aspects the complex is a covalent complex.

In one embodiment of all aspects the class I MHC molecule with a relative frequency of 1 % or more has a relative frequency of 10 % or more. In one embodiment the class I MHC molecule with a relative frequency of 10 % or more is HLA-A*0201, or HLA-A*1101, or HLA-A*2402, or HLA-A*340101, or HLA-C*0304, or HLA-C*0401, or HLA-C*0702.

In one embodiment of all aspects the class I MHC molecule with a relative frequency of 10 % or more is selected depending on the region of the individual to whom the complex is to be administered as follows:
- for an individual of European origin the class I MHC molecule is selected from the group comprising HLA-A*0101, HLA-A*0201, HLA-A*0301, HLA-B*0702, HLA-B*0801, HLA-B*4402, HLA-C*0401, HLA-C*0501, HLA-C*0701, and HLA-C*0702,
- for an individual of Australian origin the class I MHC molecule is selected from the group comprising HLA-A*0201, HLA-A*1101, HLA-A*2402, HLA-A*340101, HLA-B*1301, HLA-B*1521, HLA-B*5601, HLA-B*5602, HLA-C*0102, HLA-C*0401, HLA-C*0403, and HLA-C*1502,
- for an individual of North American origin the class I MHC molecule is selected from the group comprising HLA-A*0201, HLA-A*2402, HLA-C*0202, HLA-C*0304, HLA-C*0401, and HLA-C*0702, and
- for an individual of South-East-Asian origin the class I MHC molecule is selected from the group comprising HLA-A*1101, HLA-A*2402, HLA-B*1504, HLA-C*0102, HLA-C*0304, HLA-C*0702, and HLA-C*0801.

In one embodiment of all aspects the class I MHC molecule with a relative frequency of 10 % or more is selected depending on the region of the individual to whom the complex is to be administered as follows:
- for an individual of European origin the class I MHC molecule is HLA-A*0201,
- for an individual of Australian origin the class I MHC molecule is selected from the group comprising HLA-A*2402, HLA-B*1301, HLA-C*0102, and HLA-C*0401,
- for an individual of North American origin the class I MHC molecule is selected from the group comprising HLA-A*2402, and HLA-C*0304, and
- for an individual of South-East-Asian origin the class I MHC molecule is HLA-A*2402.

In one embodiment of all aspects the T-cell response eliciting peptide is a CD8⁺-T-cell response eliciting peptide. In one embodiment the T-cell response eliciting peptide is a virus-derived peptide.

In one embodiment of all aspects the fusion polypeptide comprises
(i) a virus-derived peptide,
(ii) β2-microglobulin, and
(iii) the soluble HLA-A allele A*0201.

In one embodiment of all aspects the virus is selected from adenovirus, human herpesvirus 1, human herpesvirus 2, human herpesvirus 4 (Epstein-Barr virus), hepatitis-B-virus, hepatitis-C-virus, human cytomegalovirus, human immunodeficiency virus, human papillomavirus type 16, human papillomavirus type 18, human papillomavirus type 31, human papillomavirus type 33, human papillomavirus type 35, human papillomavirus type 39, human papillomavirus type 45, human papillomavirus type 51, human papillomavirus type 52, human papillomavirus type 56, human papillomavirus type 58, human papillomavirus type 59, human papillomavirus type 68, human papillomavirus type 73, human papillomavirus type 82, human T-cell lymphotropic virus type I, human influenza A virus, human influenza B virus, vaccinia virus, dengue virus.

In one embodiment of all aspects the virus-derived peptide is selected from NLVPMVATV (SEQ ID NO: 01), SLYNTVATL (SEQ ID NO: 02), GLCTLVAML (SEQ ID NO: 03), GILGFVFTL (SEQ ID NO: 04), STNRQSGRQ (SEQ ID NO: 05), LLFGYPVYV (SEQ ID NO: 06), FAEGFVRAL (SEQ ID NO: 07), LIVIGILIL (SEQ ID NO: 08), or ILHTPGCV (SEQ ID NO: 09), WYAQIQPHW (SEQ ID NO: 52), AFSGVSWTM (SEQ ID NO: 53), ILIGVVITW (SEQ ID NO: 54), MMIPTVVAF (SEQ ID NO: 55), PFPQSNAPI (SEQ ID NO: 56), LLLTLLATV (SEQ ID NO: 57), IVLEHGSCV (SEQ ID NO: 58), LLFKTENGV (SEQ ID NO: 59), PLNEAIMAV (SEQ ID NO: 60), NLVRLQSGV (SEQ ID NO: 61), LVISGLFPV (SEQ ID NO: 62), LLLVAHYAI (SEQ ID NO: 63), LALLAAFKV (SEQ ID NO: 64), VILAGPMPV (SEQ ID NO: 65), HVLGRLITV (SEQ ID NO: 66), VTEHDTLLY (SEQ ID NO: 67), NTDFRVLEL (SEQ ID NO: 68), CVETMCNEY (SEQ ID NO: 69), VLEETSVML (SEQ ID NO: 70), NLVPMVATV (SEQ ID NO: 71), RIFAELEGV (SEQ ID NO: 72), IIYTRNHEV (SEQ ID NO: 73), VLAELVKQI (SEQ ID NO: 74), AVGGAVASV (SEQ ID NO: 75), TVRSHCVSK (SEQ ID NO: 76), IMREFNSYK (SEQ ID NO: 77), GPISHGHVLK (SEQ ID NO: 78), ATVQGQNLK (SEQ ID NO: 79), VYALPLKML (SEQ ID NO: 80), AYAQKIFKIL (SEQ ID NO: 81), QYDPVAALF (SEQ ID NO: 82), YVKVYLESF (SEQ ID NO: 83), DIYRIFAEL (SEQ ID NO: 84), VFETSGGLVV (SEQ ID NO: 85), KARDHLAVL (SEQ ID NO: 86), QARLTVSGL (SEQ ID NO: 87), KARAKKDEL (SEQ ID NO: 88), QIKVRVDMV (SEQ ID NO: 89), RRRHRQDAL (SEQ ID NO: 90), ARVYEIKCR (SEQ ID NO: 91), KMQVIGDQY (SEQ ID NO: 92), NVRRSWEEL (SEQ ID NO: 93), CPSQEPMSIYVY (SEQ ID NO: 94), KPGKISHIMLDVA (SEQ ID NO: 95), ELRRKMMYM (SEQ ID NO: 96), IPSINVHHY (SEQ ID NO: 97), FEQPTETPP (SEQ ID NO: 98), YAYIYTTYL (SEQ ID NO: 99), QEFFWDANDIY (SEQ ID NO: 100), YEQHKITSY (SEQ ID NO: 101), QEPMSIYVY (SEQ ID NO: 102), SEHPTFTSQY (SEQ ID NO: 103), QAIRETVEL (SEQ ID NO: 104), TRATKMQVI (SEQ ID NO: 105), DALPGPCI (SEQ ID NO: 106), CEDVPSGKL (SEQ ID NO: 107), HERNGFTVL (SEQ ID NO: 108), PTFTSQYRIQGKL (SEQ ID NO: 109), QMWQARLTV (SEQ ID NO: 110), HELLVLVKKAQL (SEQ ID NO: 111), or DDYSNTHSTRYV (SEQ ID NO: 112), or a variant thereof comprising of from 1 to 3 amino acid exchanges, additions, and/or deletions.

In one embodiment of all aspects the β2-microglobulin is human β2-microglobulin. In one embodiment the β2-microglobulin is wild-type human β2-microglobulin. In one embodiment the β2-microglobulin is consisting of the amino acid sequence of SEQ ID NO: 10 or is a variant thereof comprising of from 1 to 10 amino acid exchanges, additions, and/or deletions.

In one embodiment of all aspects the β2-microglobulin is human β2-microglobulin and the class I MHC molecule with a relative frequency of 10 % or more is human LA-A*0201. In one embodiment the extracellular domains α1, α2 and α3 of a class I MHC molecule is consisting of the amino acid sequence of SEQ ID NO: 11 or is a variant thereof comprising of from 1 to 10 amino acid exchanges, additions, and/or deletions.

In one embodiment of all aspects the virus-derived peptide is fused to the β2-microglobulin via a first linker peptide. In one embodiment the virus-derived peptide is fused to the N-terminus of the β2-microglobulin.

In one embodiment of all aspects the β2-microglobulin is fused to the extracellular domain α1 of a class I MHC molecule via a second linker peptide.

In one embodiment of all aspects the extracellular domains α3 of a class I MHC molecule is fused to one of the disulfide-linked polypeptide chains via a third linker peptide.

In one embodiment of all aspects the first, second, and third linker peptide is the same or different.

In one embodiment of all aspects the first linker peptide, the second linker peptide, and the third linker peptide are selected independently from each other from the amino acid sequences GS (SEQ ID NO: 12), GGS (SEQ ID NO: 13), GGGS (SEQ ID NO: 14), GGGSGGGS (SEQ ID NO: 15), GGGSGGGSGGGS (SEQ ID NO: 16), GGGSGGGSGGGSGGGS (SEQ ID NO: 17), GGGSGGGSGGGSGGGSGGGS (SEQ ID NO: 18), GGGGS (SEQ ID NO: 19), GGGGSGGGGS (SEQ ID NO: 20), GGGGSGGGGSGGGGS (SEQ ID NO: 21), GGGGSGGGGSGGGGSGGGGS (SEQ ID NO: 22), and GGGGSGGGGSGGGGSGGGGSGGGGS (SEQ ID NO: 23).

In one embodiment of all aspects the first linker peptide comprises the amino acid sequence of SEQ ID NO: 21.

In one embodiment of all aspects the second linker peptide comprises the amino acid sequence of SEQ ID NO: 22.

In one embodiment of all aspects the third linker peptide comprises the amino acid sequence of SEQ ID NO: 12.

In one embodiment of all aspects the antibody heavy chain hinge region polypeptide is selected from an antibody heavy chain hinge region polypeptide of a human antibody of the class IgG or the class IgE.

In one embodiment of all aspects the antibody heavy chain hinge region polypeptide is selected from an antibody heavy chain hinge region polypeptide of a human antibody of the subclass IgG1, or IgG2, or IgG3, or IgG4.

In one embodiment of all aspects the antibody heavy chain hinge region polypeptide comprises or is consisting of the amino acid sequence of EPKSCDKTHTCPPCP (SEQ ID NO: 24), EPKSADKTHTCPPCP (SEQ ID NO: 25), ERKCCVECPPCP (SEQ ID NO: 26), ERKCAVECPPCP (SEQ ID NO: 27), ERKACVECPPCP (SEQ ID NO: 28), ELKTPLGDTTHTCPRCP (EPKSCDTPPPCPRCP)₃ (SEQ ID NO: 29), ESKYGPPCPSCP (SEQ ID NO: 30), DKTHTCPPCP (SEQ ID NO: 47), VECPPCP (SEQ ID NO: 48), AVECPPCP (SEQ ID NO: 49), DTTHTCPRCP (SEQ ID NO: 50), or PPCPSCP (SEQ ID NO: 51).

In one embodiment of all aspects the first disulfide-linked polypeptide and/or the second disulfide-linked polypeptide comprises a CH2 domain and/or a CH3 domain of human origin. In one embodiment the CH2 domain and the CH3 of human origin is of a human antibody of the class IgG or IgE. In one embodiment the CH2 domain and the CH3 domain is of a human antibody of the subclass IgG1, or IgG2, or IgG3, or IgG4. In one embodiment the CH2 domain comprises the amino acid sequence of SEQ ID NO: 31. In one embodiment the CH2 domain is of a human antibody of the subclass IgG1 or IgG2 and comprises at least one mutation in E233, L234, L235, G236, D265, D270, N297, E318, K320, K322, A327, P329, A330, and/or P331 (numbering according to the EU index of Kabat). In one embodiment the CH2 domain is of a human antibody of the subclass IgG1 or the human subclass IgG2 with the mutations L234A and L235A, and/or the mutations D265A and N297A, and/or contains the PVA236 mutation, and/or contains the mutation P329G. In one embodiment the CH2 domain is of a human antibody of the subclass IgG1 with the mutations L234A and L235A and/or P329G. In one embodiment the CH2 domain is of a human antibody of the subclass IgG4 with the mutation S228P and/or L235E. In one embodiment the CH2 domain comprises the amino acid sequence of SEQ ID NO: 32 or SEQ ID NO: 33. In one embodiment the CH3 domain comprises the amino acid sequence of SEQ ID NO: 34.

In one embodiment of all aspects the first disulfide-linked polypeptide comprises the amino acid sequence of SEQ ID NO: 35 and the second disulfide-linked polypeptide comprises the amino acid sequence of SEQ ID NO: 36.

In one embodiment of all aspects the first and the second disulfide-linked polypeptide comprise the amino acid sequence of SEQ ID NO: 37 or SEQ ID NO: 38.

In one embodiment of all aspects the first disulfide-linked polypeptide or the second disulfide-linked polypeptide is consisting of the amino acid sequence of SEQ ID NO: 37 or SEQ ID NO: 38.

In one embodiment of all aspects the first disulfide-linked polypeptide comprises the amino acid sequence of SEQ ID NO: 39 and the second disulfide-linked polypeptide comprises the amino acid sequence of SEQ ID NO: 40.

In one embodiment of all aspects the polypeptide chains, which are linked by one or more disulfide bonds, are linked by two, or three, or four disulfide bonds.

In one embodiment of all aspects the complex is characterized in that the fusion polypeptide comprises in N- to C-terminal direction
(i) a virus-derived peptide that has an amino acid sequence selected from the group comprising SEQ ID NO: 01 to SEQ ID NO: 09,
(ii) a first linker peptide that has an amino acid sequence selected from the group comprising SEQ ID NO: 16, 17, 18, 21, 22, and 23.
(iii) a β2-microglobulin that has an amino acid sequence of SEQ ID NO: 10 or is a variant thereof comprising of from 1 to 10 amino acid exchanges, additions, and/or deletions,
(iv) a second linker peptide that has an amino acid sequence selected from the group comprising SEQ ID NO: 16, 17, 18, 21, 22, and 23.
(v) the extracellular domains α1, α2 and α3 of a class I MHC molecule that has an amino acid sequence of SEQ ID NO: 11 or is a variant thereof comprising of from 1 to 10 amino acid exchanges, additions, and/or deletions, and
(vi) a third linker peptide that has an amino acid sequence selected from the group comprising SEQ ID NO: 12, 16, 17, 18, 21, 22, and 23.

In one embodiment of all aspects the first disulfide-linked polypeptide and the second disulfide-linked polypeptide further comprise
- a human IgG1 CH2 domain comprising an amino acid sequence selected from SEQ ID NO: 31, 32, and 33, and
- a human IgG1 CH3 domain comprising an amino acid sequence selected from SEQ ID NO: 34, 35, and 36.

In one embodiment of all aspects the complex is characterized in that it comprises
- one fusion polypeptide that comprises in N- to C-terminal direction
   (i) a virus-derived peptide that has an amino acid sequence selected from SEQ ID NO: 01 to SEQ ID NO: 09,
   (ii) a first linker peptide that has an amino acid sequence selected from SEQ ID NO: 16, 17, 18, 21, 22, and 23.
   (iii) a β2-microglobulin that has an amino acid sequence of SEQ ID NO: 10 or is a variant thereof comprising of from 1 to 10 amino acid exchanges, additions, and/or deletions,
   (iv) a second linker peptide that has an amino acid sequence selected from SEQ ID NO: 16, 17, 18, 21, 22, and 23.
   (v) the extracellular domains α1, α2 and α3 of a class I MHC molecule that has an amino acid sequence of SEQ ID NO: 11 or is a variant thereof comprising of from 1 to 10 amino acid exchanges, additions, and/or deletions, and
   (vi) a third linker peptide that has an amino acid sequence selected from SEQ ID NO: 12, 16, 17, 18, 21, 22, and 23,
   and
- two polypeptide chains, which are linked by one or more disulfide bonds, wherein the first disulfide-linked polypeptide chain comprises in N- to C-terminal direction
   (i) an immunoglobulin light or heavy chain variable domain,
   (ii) an immunoglobulin light or heavy chain constant domain,
   (iii) an antibody heavy chain hinge region polypeptide comprising an amino acid sequence selected from SEQ ID NO: 24 to SEQ ID NO: 30 and SEQ ID NO: 47-51,
   (iv) a human IgG1 CH2 domain comprising an amino acid sequence selected from SEQ ID NO: 31, 32, and 33, and
   (v) a human IgG1 CH3 domain comprising an amino acid sequence selected from SEQ ID NO: 34, 35, and 36,
   and the second disulfide-linked polypeptide chain comprises in N- to C-terminal direction
   (i) an antibody heavy chain hinge region polypeptide comprising an amino acid sequence selected from SEQ ID NO: 24 to SEQ ID NO: 30 and SEQ ID NO: 47-51,
   (ii) a human IgG1 CH2 domain comprising an amino acid sequence selected from SEQ ID NO: 31, 32, and 33, and
   (iii) a human IgG1 CH3 domain comprising an amino acid sequence selected from SEQ ID NO: 34, 35, and 36,
wherein the fusion polypeptide is
- covalently bound either to the C-terminus or the N-terminus of the second disulfide-linked polypeptide chain.

In one embodiment of all aspects the first and second disulfide-linked polypeptide chain comprise the same antibody heavy chain hinge region polypeptide.

In one embodiment of all aspects the virus-derived polypeptide comprises the amino acid sequence of SEQ ID NO: 01, the first linker peptide comprises the amino acid sequence of SEQ ID NO: 21, the second linker peptide comprises the amino acid sequence of SEQ ID NO: 22, the third linker peptide comprises the amino acid sequence of SEQ ID NO: 12, the human IgG1 CH2 domain comprises the amino acid sequence of SEQ ID NO: 32 or 33, and the human IgG1 CH3 domain of one disulfide-linked polypeptide comprises the amino acid sequence of SEQ ID NO: 35 and the human IgG1 CH3 domain of the other disulfide-linked polypeptide comprises the amino acid sequence of SEQ ID NO: 36.

In one embodiment of all aspects the complex is characterized in that it comprises
- one fusion polypeptide that comprises in N- to C-terminal direction
   (i) a virus-derived peptide that has an amino acid sequence selected from SEQ ID NO: 01 to SEQ ID NO: 09,
   (ii) a first linker peptide that has an amino acid sequence selected from SEQ ID NO: 16, 17, 18, 21, 22, and 23.
   (iii) a β2-microglobulin that has an amino acid sequence of SEQ ID NO: 10 or is a variant thereof comprising of from 1 to 10 amino acid exchanges, additions, and/or deletions,
   (iv) a second linker peptide that has an amino acid sequence selected from SEQ ID NO: 16, 17, 18, 21, 22, and 23.
   (v) the extracellular domains α1, α2 and α3 of a class I MHC molecule that has an amino acid sequence of SEQ ID NO: 11 or is a variant thereof comprising of from 1 to 10 amino acid exchanges, additions, and/or deletions, and
   (vi) a third linker peptide that has an amino acid sequence selected from SEQ ID NO: 12, 16, 17, 18, 21, 22, and 23,
   and
- two polypeptide chains, which are linked by one or more disulfide bonds,
   wherein the first disulfide-linked polypeptide chain comprises in N- to C-terminal direction
   (i) an immunoglobulin light or heavy chain variable domain,
   (ii) an immunoglobulin light or heavy chain constant domain,
   (iii) an antibody heavy chain hinge region polypeptide comprising an amino acid sequence selected from SEQ ID NO: 24 to SEQ ID NO: 30 and SEQ ID NO: 47-51,
   (iv) a human IgG1 CH2 domain comprising an amino acid sequence selected from SEQ ID NO: 31, 32, and 33, and
   (v) a human IgG1 CH3 domain comprising an amino acid sequence selected from SEQ ID NO: 34, 35, and 36, and the second disulfide-linked polypeptide chain comprises an antibody heavy chain hinge region polypeptide comprising an amino acid sequence selected from SEQ ID NO: 24 to SEQ ID NO: 30 and SEQ ID NO: 47-51,
wherein the fusion polypeptide is
- covalently bound either to the C-terminus or the N-terminus of the first disulfide-linked polypeptide chain, or
- covalently bound to the N-terminus of an antibody variable domain that is the complementary heavy or light chain variable domain to that comprised in the first disulfide-linked polypeptide chain, or
- covalently bound to the C-terminus of an antibody constant domain that is the complementary heavy or light chain constant domain to that comprised in the first disulfide-linked polypeptide chain.

In one embodiment of all aspects the virus-derived polypeptide comprises the amino acid sequence of SEQ ID NO: 01, the first linker peptide comprises the amino acid sequence of SEQ ID NO: 21, the second linker peptide comprises the amino acid sequence of SEQ ID NO: 22, the third linker peptide comprises the amino acid sequence of SEQ ID NO: 12, the human IgG1 CH2 domain comprises the amino acid sequence of SEQ ID NO: 32 or 33, and the human IgG1 CH3 domain of one disulfide-linked polypeptide comprises the amino acid sequence of SEQ ID NO: 35 and the human IgG1 CH3 domain of the other disulfide-linked polypeptide comprises the amino acid sequence of SEQ ID NO: 36.

In one embodiment of all aspects the complex is characterized in that it comprises
- one fusion polypeptide that comprises in N- to C-terminal direction
   (i) a virus-derived peptide that has an amino acid sequence selected from SEQ ID NO: 01 to SEQ ID NO: 09,
   (ii) a first linker peptide that has an amino acid sequence selected from SEQ ID NO: 16, 17, 18, 21, 22, and 23.
   (iii) a β2-microglobulin that has an amino acid sequence of SEQ ID NO: 10 or is a variant thereof comprising of from 1 to 10 amino acid exchanges, additions, and/or deletions,
   (iv) a second linker peptide that has an amino acid sequence selected from SEQ ID NO: 16, 17, 18, 21, 22, and 23.
   (v) the extracellular domains α1, α2 and α3 of a class I MHC molecule that has an amino acid sequence of SEQ ID NO: 11 or is a variant thereof comprising of from 1 to 10 amino acid exchanges, additions, and/or deletions, and
   (vi) a third linker peptide that has an amino acid sequence selected from SEQ ID NO: 12, 16, 17, 18, 21, 22, and 23,
   and
- two polypeptide chains, which are linked by one or more disulfide bonds, wherein the first and the second disulfide-linked polypeptide chain each comprise in N- to C-terminal direction
   (i) an immunoglobulin light or heavy chain variable domain,
   (ii) an immunoglobulin light or heavy chain constant domain,
   (iii) an antibody heavy chain hinge region polypeptide comprising an amino acid sequence selected from SEQ ID NO: 24 to SEQ ID NO: 30 and SEQ ID NO: 47-51,
   (iv) a human IgG1 CH2 domain comprising an amino acid sequence selected from SEQ ID NO: 31, 32, and 33, and
   (v) a human IgG1 CH3 domain comprising an amino acid sequence selected from SEQ ID NO: 34, 35, and 36,
wherein the fusion polypeptide is
- covalently bound either to the C-terminus or the N-terminus of the second disulfide-linked polypeptide chain, or
- covalently bound to the N-terminus of an antibody variable domain that is the complementary heavy or light chain variable domain to that comprised in the first disulfide-linked polypeptide chain, or
- covalently bound to the C-terminus of an antibody constant domain that is the complementary heavy or light chain constant domain to that comprised in the first disulfide-linked polypeptide chain.

In one embodiment of all aspects the virus-derived polypeptide comprises the amino acid sequence of SEQ ID NO: 01, the first linker peptide comprises the amino acid sequence of SEQ ID NO: 21, the second linker peptide comprises the amino acid sequence of SEQ ID NO: 22, the third linker peptide comprises the amino acid sequence of SEQ ID NO: 12, the human IgG1 CH2 domain comprises the amino acid sequence of SEQ ID NO: 32 or 33, and the human IgG1 CH3 domain of one disulfide-linked polypeptide comprises the amino acid sequence of SEQ ID NO: 35 and the human IgG1 CH3 domain of the other disulfide-linked polypeptide comprises the amino acid sequence of SEQ ID NO: 36.

In one embodiment of all aspects
- the first linker peptide comprises the amino acid sequence of SEQ ID NO: 21, and/or
- the second linker peptide comprises the amino acid sequence of SEQ ID NO: 22, and/or
- the third linker peptide comprises the amino acid sequence of SEQ ID NO: 12, and/or
- the human IgG1 CH2 domain comprises the amino acid sequence of SEQ ID NO: 32 or 33, and/or
- the human IgG1 CH3 domain of one disulfide-linked polypeptide comprises the amino acid sequence of SEQ ID NO: 35 and the human IgG1 CH3 domain of the other disulfide-linked polypeptide comprises the amino acid sequence of SEQ ID NO: 36.

In one embodiment of all aspects the virus-derived peptide is selected from the group comprising SEQ ID NO: 01 to SEQ ID NO: 09, SEQ ID NO: 52 to SEQ ID NO: 112, or is a variant thereof comprising of from 1 to 3 amino acid exchanges, additions, and/or deletions.

One aspect as reported herein is a nucleic acid encoding the complex as reported herein.

In one embodiment the nucleic acid comprises two to four expression cassettes comprising structural genes encoding polypeptides with different amino acid sequence.

One aspect as reported herein is a host cell comprising the nucleic acid as reported herein.

One aspect as reported herein is a method of producing a complex as reported herein comprising culturing the host cell as reported herein so that the complex is produced.

In one embodiment the complex is recovered from the cells or the cultivation medium and thereby the complex is produced.

One aspect as reported herein is an immunoconjugate comprising the complex as reported herein and a cytotoxic agent.
One aspect as reported herein is a pharmaceutical formulation comprising the complex as reported herein and optionally a pharmaceutically acceptable carrier.
In one embodiment the pharmaceutical formulation further comprises an additional therapeutic agent.
One aspect as reported herein is the complex as reported herein for use as a medicament.

One aspect as reported herein is the complex as reported herein for use in the treatment of cancer or of a chronic viral infection.

One aspect as reported herein is the complex as reported herein for use in therapy by virus-specific cytotoxic T-cells of an individual to a target.

One aspect as reported herein is the complex as reported herein for use in therapy by removal cancer cells or of virus infected cells.

### Detailed Description of the Invention

### Short description of the figures

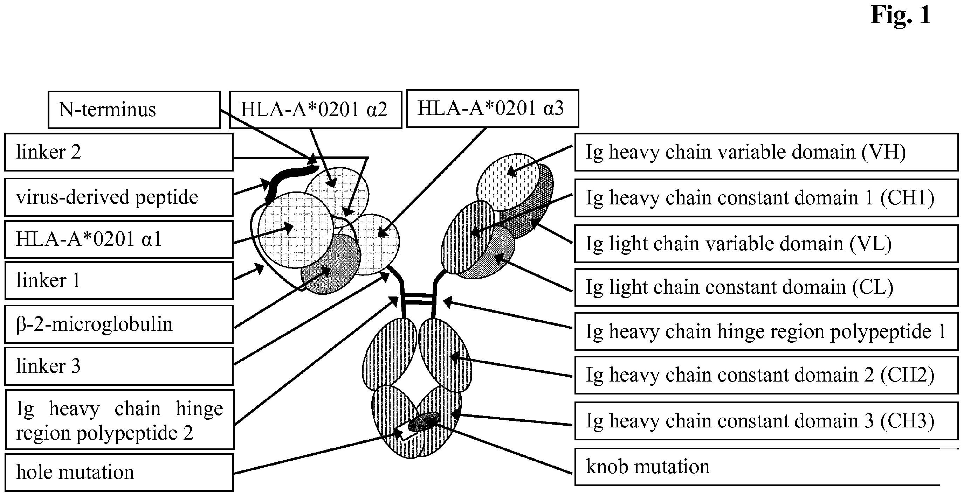

- **Figure 1**: Annotated scheme of the complexes as reported herein.
- **Figure 2**: Exemplary polypeptides comprised in the complex as reported herein: fusion polypeptides were N-terminally fused to either an antibody light chain or to an antibody heavy chain hinge region comprising polypeptide.

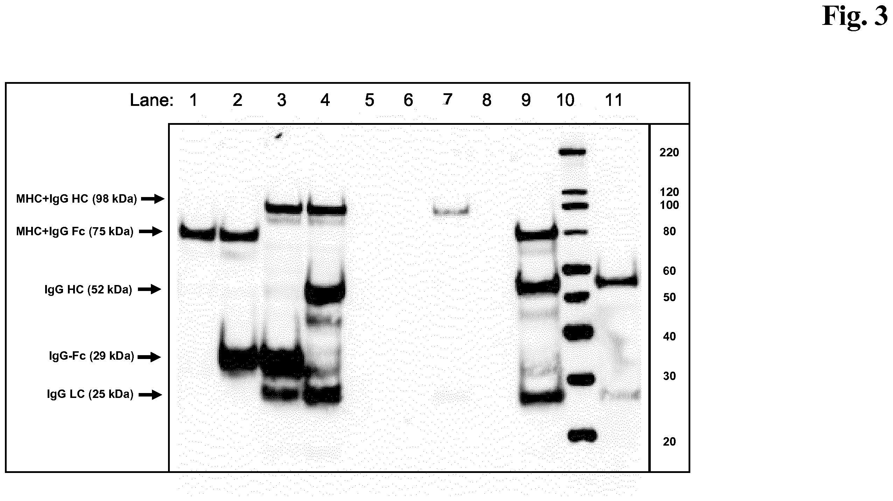

- **Figure 3**: Western blot of a SDS polyacrylamide gel of cell culture supernatant of HEK 293 cells transfected with the corresponding expression plasmids. Staining was performed with peroxidase conjugated polyclonal rabbit anti-human κ-light chain antibody and polyclonal rabbit anti-human IgG antibody conjugated to horseradish peroxidase.
Lanes: 1: two-armed peptide-β2-microglobulin-HLA-A0201-IgG-Fc; 2: one-armed peptide-β2-microglobulin-HLA-A0201-IgG-Fc + IgG-Fc; 3: one-armed peptide-β2-microglobulin-HLA-A0201-IgG-heavy chain + IgG-light chain + IgG-Fc; 4: one-armed peptide-β2-microglobulin-HLA-A0201-IgG-heavy chain + IgG-heavy chain + IgG-light chain; 5: two-armed β2-microglobulin-HLA-A0201-IgG-light chain + IgG-heavy chain; 6: two-armed peptide-β2-microglobulin-HLA-A0201-IgG-light chain + IgG-heavy chain; 7: two-armed peptide-β2-microglobulin-HLA-A0201-IgG-heavy chain + IgG-light chain; 8: two-armed peptide-β2-microglobulin-HLA-A0201-IgG-Fc-scFv; 9: one-armed peptide-β2-microglobulin-HLA-A0201-IgG-Fc + one-armed IgG (heavy and light chain); 10: molecular weight marker; 11: reference standard IgG1 antibody.

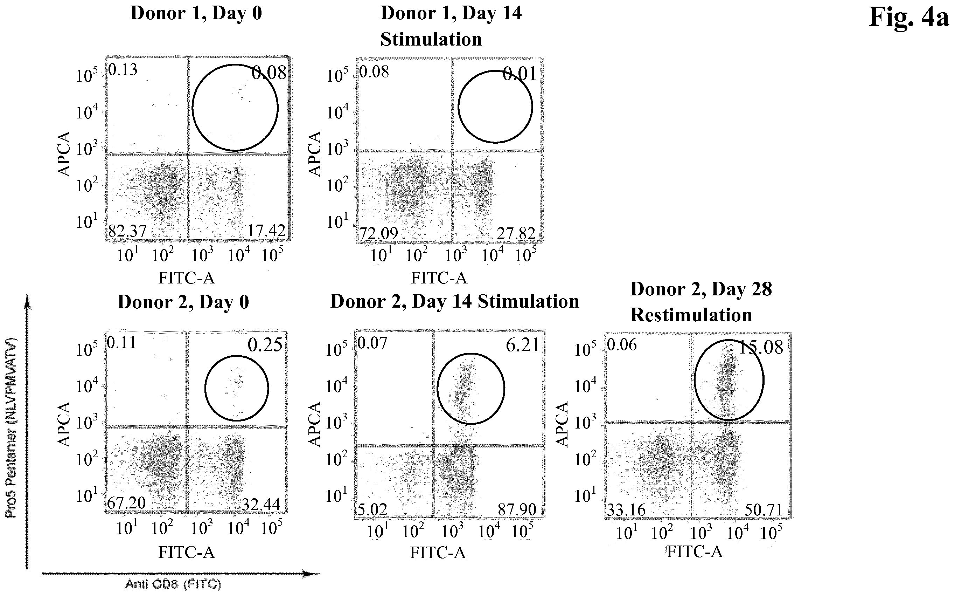

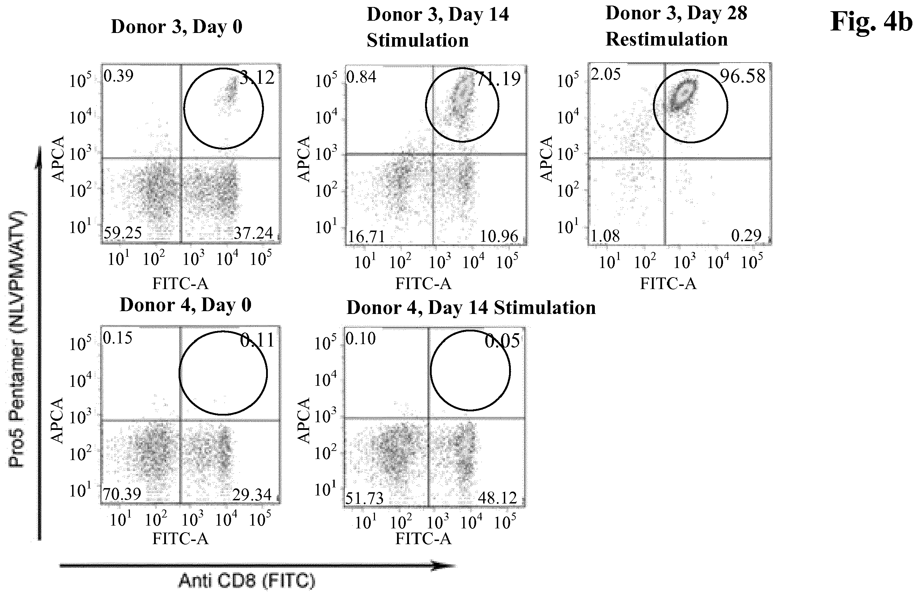

- **Figure 4**: Flow cytometric analysis to determine the number of CMV-specific cytolytic T-cells from different donors before and after in vitro stimulation with specific peptide: Analysis of 4 human donor derived peripheral blood lymphocytes (PBLs); anti-CD8 antibody conjugated to FITC label staining (BD, Cat. No. 345772) combined with Pro5 pentamer APC (ProImmune, Cat. No. F008-4A-E) stained TCR recognizing MHC-class I (HLA-A*0201) loaded with CMV-derived peptide (NLVPMVATV, SEQ ID NO: 01); circle: CMV-specific CD8⁺-T-cells.

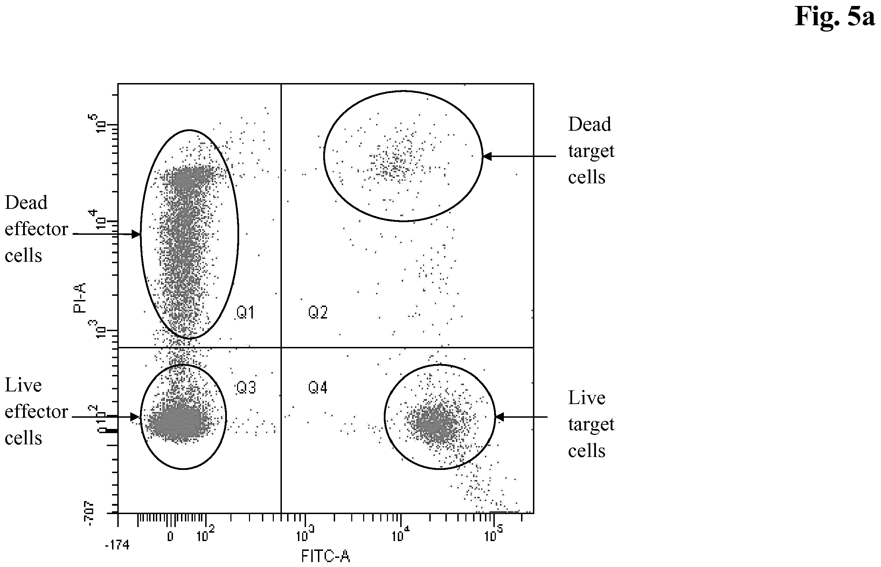

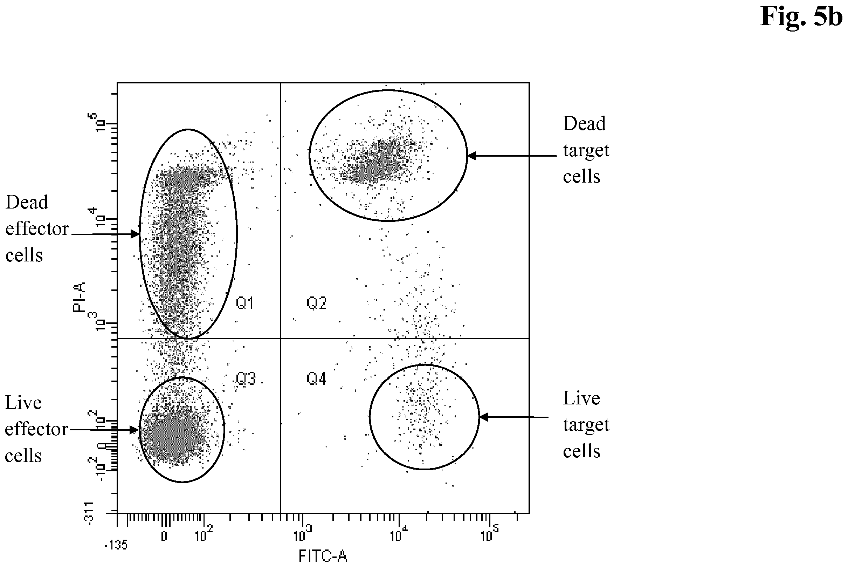

- **Figure 5**: Flow Cytometric Analysis to analyze the cytolytic capability of stimulated CTLs through lysis of MN60 tumor cells loaded with CMV peptide.

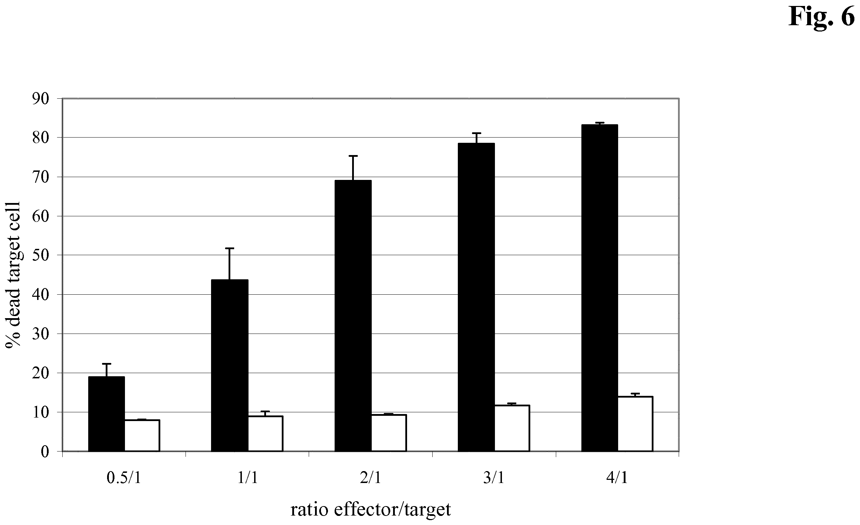

- **Figure 6**: T-cell specific removal of CMV pulsed T-cells: Flow Cytometric Analysis to analyze the cytolytic capability of stimulated CTLs through lysis of MN60 tumor cells loaded with CMV peptide depending on the effector to target cell ratio; black: MN60 cells loaded with CMV-peptide, white: MN60 cells not loaded with CMV-peptide.

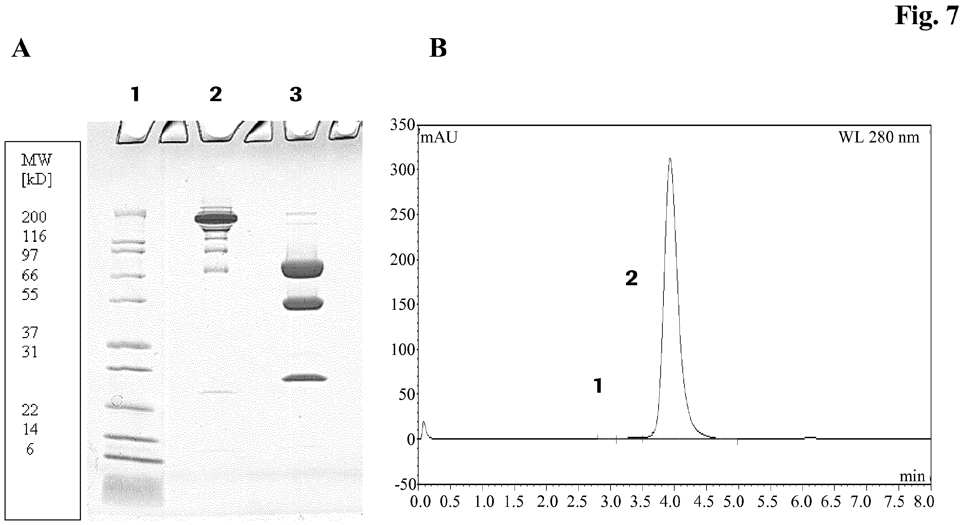

- **Figure 7**: A: SDS-PAGE gel with Coomassie staining: lane 1: molecular weight standard, lane 2: one-armed peptide-β2-microglobulin-HLA-A0201-IgG-Fc + one-armed IgG complex (heavy and light chain), non-reducing conditions; lane 3: one-armed peptide-β2-microglobulin-HLA-A0201-IgG-Fc + one-armed IgG complex (heavy and light chain), reducing conditions. B: Size exclusion chromatography chromatogram; 1: high molecular weight forms (0.7 area %); 2: monomeric complex (99.3 area %).

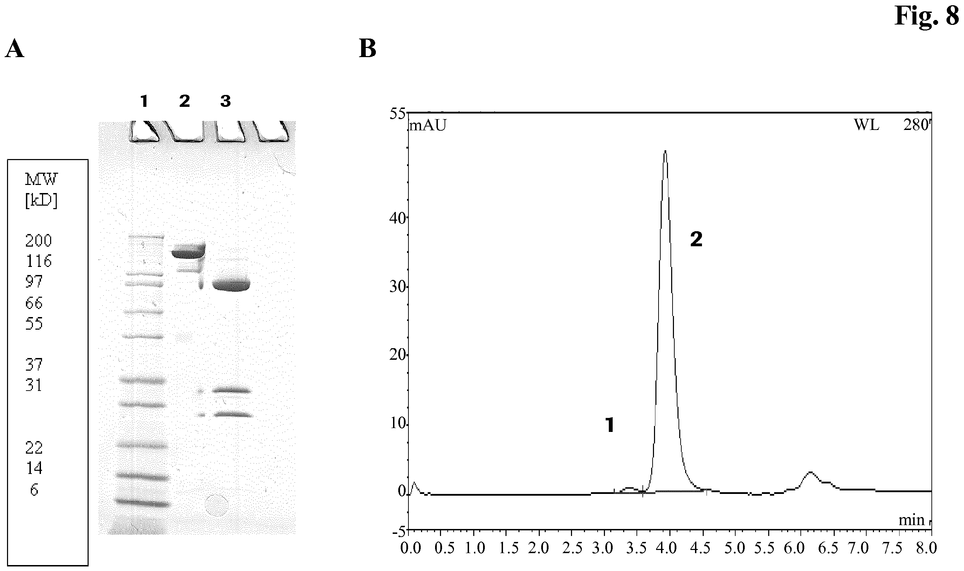

- **Figure 8**: A: SDS-PAGE gel with Coomassie staining: lane 1: molecular weight standard, lane 2: one-armed peptide-β2-microglobulin-HLA-A0201-IgG-heavy chain + IgG-light chain + IgG-Fc complex, non-reducing conditions; lane 3: one-armed peptide-β2-microglobulin-HLA-A0201-IgG-heavy chain + IgG-light chain + IgG-Fc complex, reducing conditions. B: Size exclusion chromatography chromatogram; 1: high molecular weight forms (1.8 area %); 2: monomeric complex (98.2 area %).

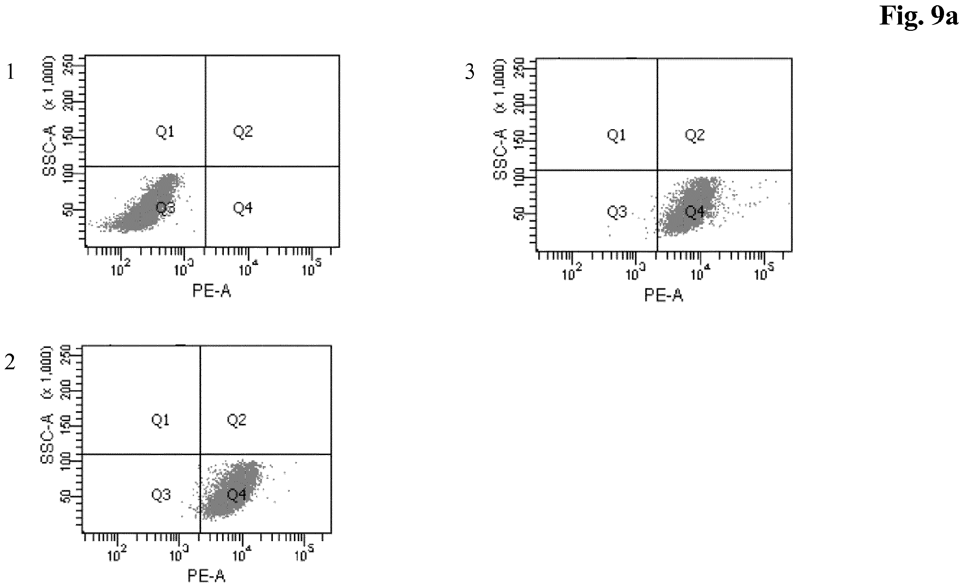

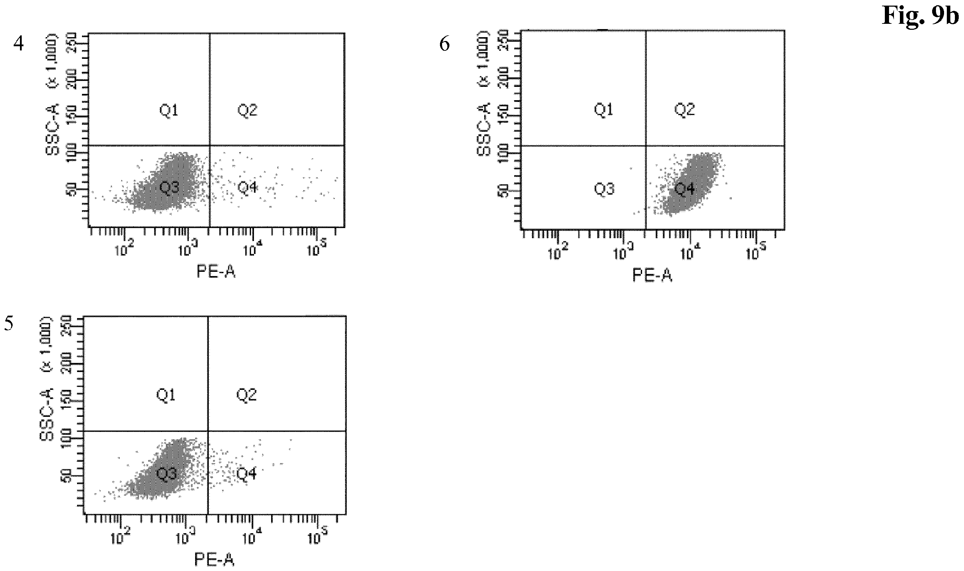

- **Figure 9**: Analysis of binding of a complex as reported herein to human IGF-1R positive Cell Line using FACS.;
1: H460M2 cells incubated with one-armed peptide-β2-microglobulin-HLA-A0201-IgG-heavy chain + IgG-light chain + IgG-Fc complex, not stained;
2: H460M2 cells incubated with one-armed peptide-β2-microglobulin-HLA-A0201-IgG-heavy chain + IgG-light chain + IgG-Fc complex at 4°C, stained with fluorescently labeled anti <human IgG> antibody;
3: H460M2 cells incubated with one-armed peptide-β2-microglobulin-HLA-A0201-IgG-heavy chain + IgG-light chain + IgG-Fc complex at 37°C, stained with fluorescently labeled anti <human IgG> antibody;
4: H460M2 cells incubated with one-armed peptide-β2-microglobulin-HLA-A0201-IgG-heavy chain + IgG-light chain + IgG-Fc complex, stained with fluorescently labeled control antibody (anti-DIG antibody);
5: H460M2 cells stained with fluorescently labeled anti-human IgG antibody;
6: H460M2 cells stained with fluorescently labeled anti-human IGF-1R antibody.

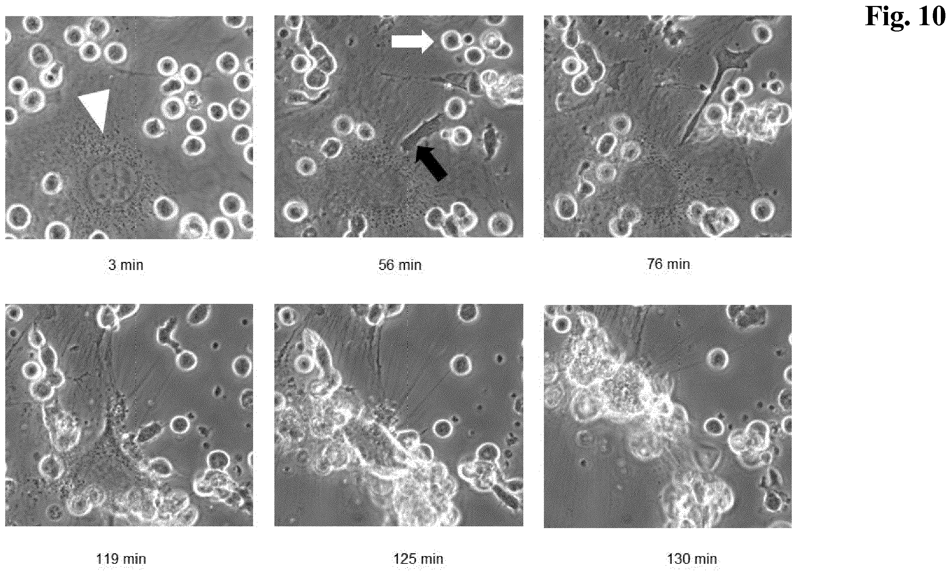

- **Figure 10**: Microscope imaging of antigen binding complex as reported herein mediated lysis of human IGF-1R expressing I24 3T3 cells (large adherently growing cells, white arrowhead). Lysis is mediated by human CMV-specific T-cells (small cells either round shaped, white arrow, or amoeboid migrating cells, black arrow).

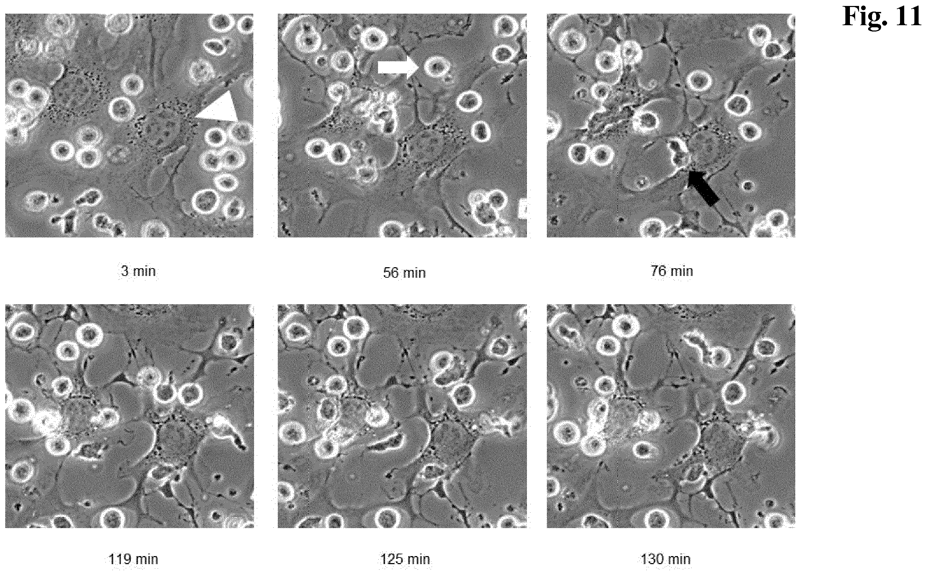

- **Figure 11**: Microscope imaging of I24 3T3 cells (large adherently growing cells, white arrowhead) incubated with human CMV-specific T-cells (small cells either round shaped, white arrow or amoeboid migrating cells, black arrow) in the absence of a complex as reported herein.

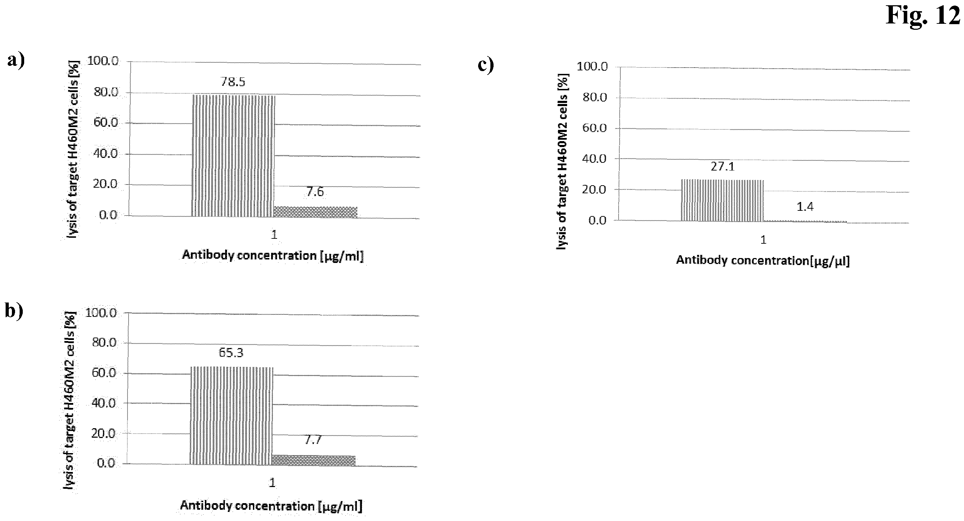

- **Figure 12**: Cytotoxicity assay: antigen binding complex as reported herein triggers lysis of H460M2 tumor cells through human CMV-specific T-cells. a) (6h) target Cells: CMV-specific effector T-cells 1:1.5; b) (6h) target Cells:CMV-specific effector T-cells 1:0.75; c) (6h) target Cells:CMV-specific effector T-cells 1:0.5; left bar: complex as reported herein; right bar MAB IGF-1R-afucosylated.

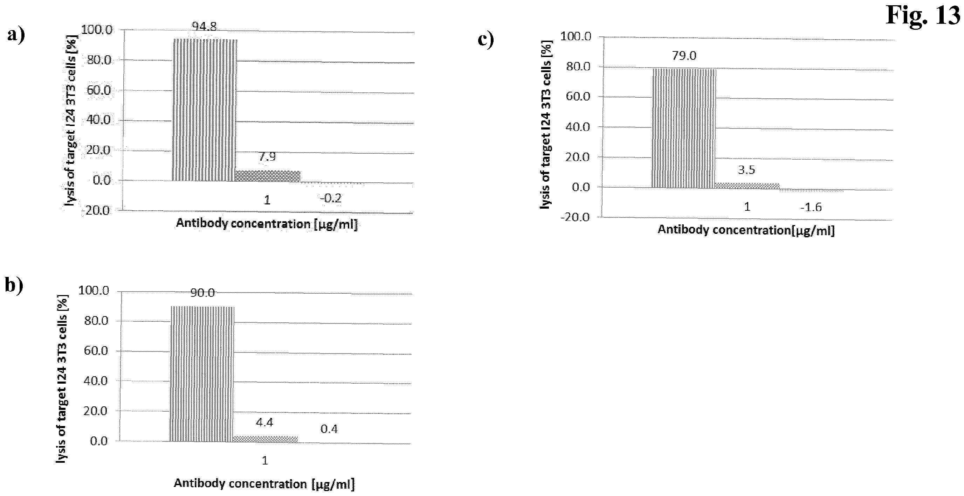

- **Figure 13**: Cytotoxicity assay: antigen binding complex as reported herein triggers lysis of I24 3T3 tumor cells through human CMV-specific T-cells; a) (9h) Target Cells : CMV-specific Effector T-cells 1:1.5; b) (9h) Target Cells : CMV-specific Effector T-cells 1:0.75; c) 9h) Target Cells : CMV-specific Effector T-cells 1:0.5; left bar: complex as reported herein; middle bar: MAB IGF-1R-afu; right bar: MAB ed; right bar: anti-digoxygenin antibody.

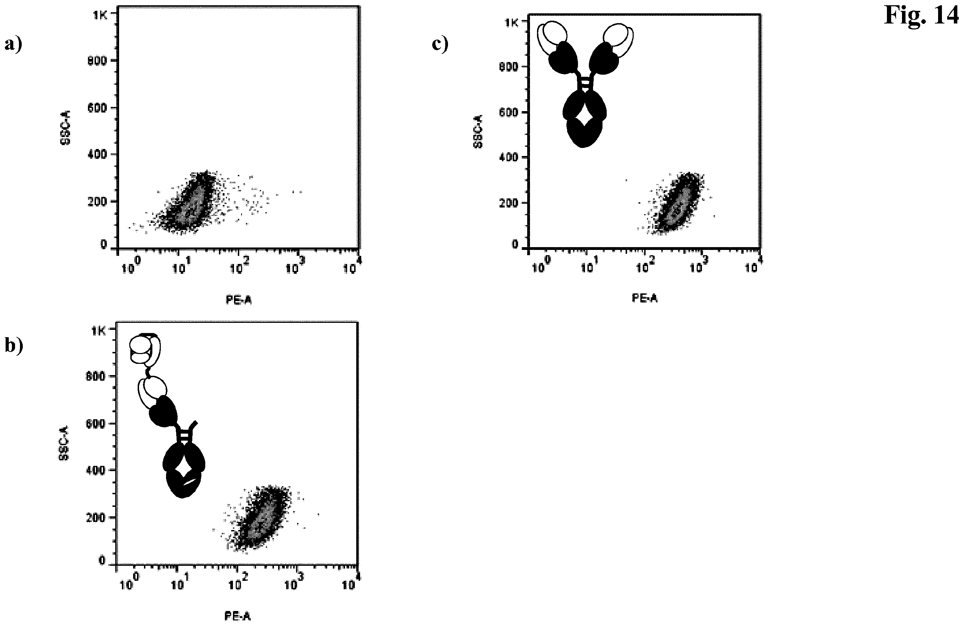

- **Figure 14**: FACS analysis of binding of anti-IGF-1R antibody and complexes as reported herein to lung adenocarcinoma cell line H460M2; a) secondary antibody only (goat anti-human IgG(H+L) (Jackson Laboratories, Cat# 109-116-088)); b) complex as reported herein wherein the fusion polypeptide is fused to the N-terminus of the heavy chain of an anti-IGF-1R antibody comprising only one pair of variable domains; c) anti-IGF-1R antibody.

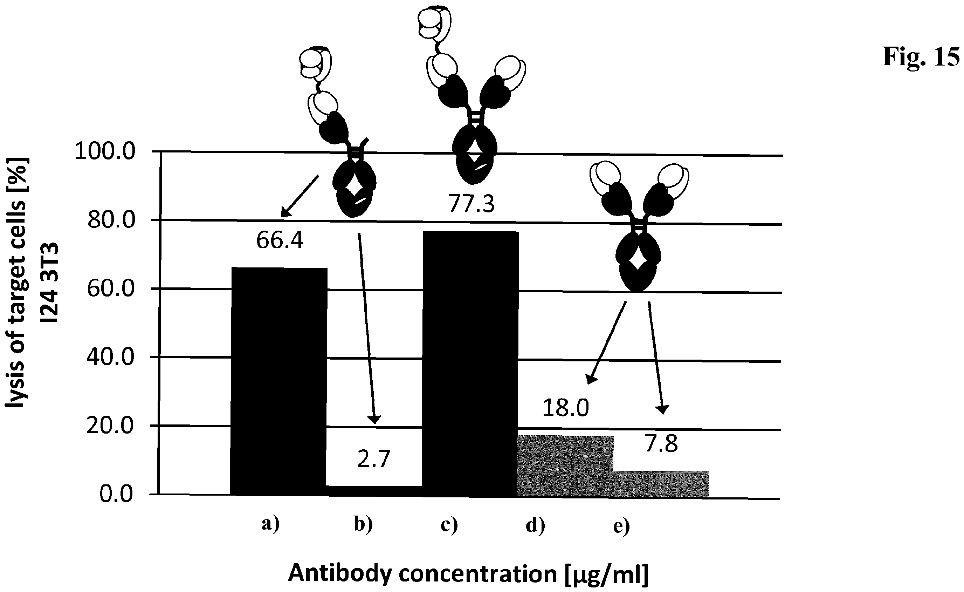

- **Figure 15**: In vitro efficacy and specificity (cytotoxicity assay) of different complexes as reported herein; a) complex comprising a monovalent anti-IGF1R antibody and a CMV-derived peptide ; b) complex comprising a monovalent anti-IGF1R antibody and an EBV-derived peptide (control); c) complex comprising a bivalent anti-IGF1R antibody and a CMV-derived peptide; d) anti-IGF-1R antibody (control); e)anti-digoxigenin antibody (control).

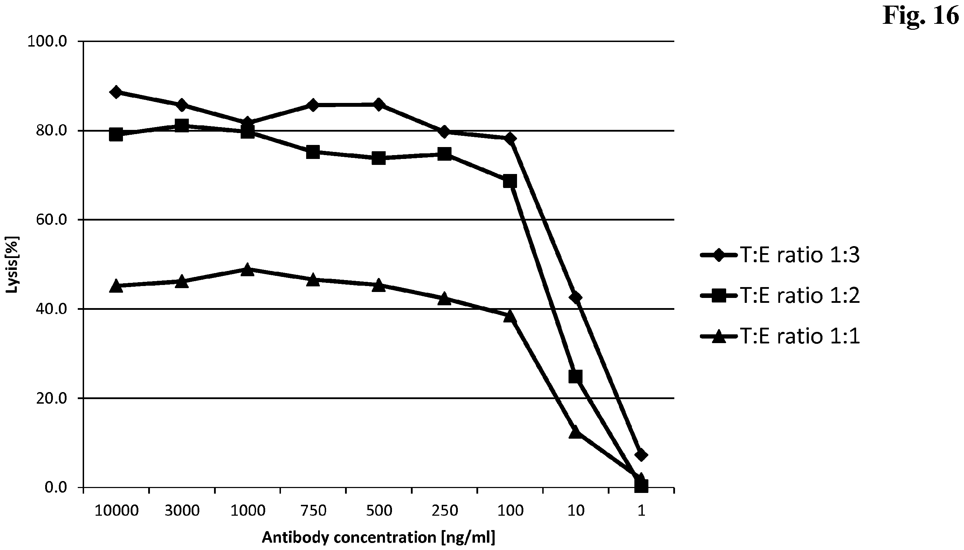

- Figure 16: In vitro efficacy and specificity (EC50 value) of a complex as reported herein wherein the fusion polypeptide is fused to the N-terminus of the heavy chain of a complete anti-IGF-1R antibody determined at different target (T) to effector (E) cell ratios.

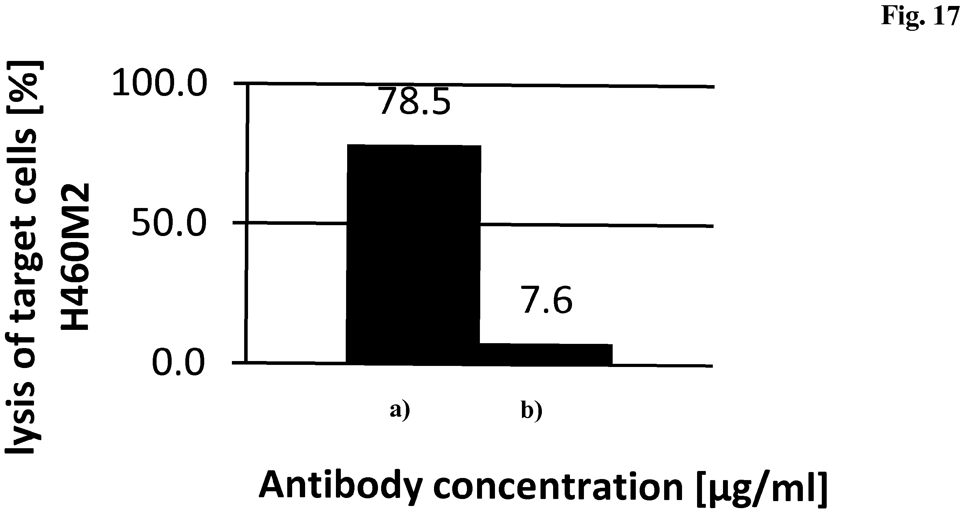

- **Figure 17**: Lysis of target cells after 6 hours incubation with a) a complex comprising a monovalent anti-IGF1R antibody and a fusion polypeptide comprising a CMV-derived peptide and b) an anti-IGF-1R antibody at a ratio of target to effector cells of 1:1.5.

**Short description of the sequences**

| | |
|---|---|
| **SEQ ID NO: 01** | Human cytomegalovirus-derived peptide. |
| **SEQ ID NO: 02** | Human immunodeficiency virus-derived peptide. |
| **SEQ ID NO: 03** | Human herpesvirus 4 derived peptide. |
| **SEQ ID NO: 04** | Influenza A virus-derived peptide. |
| **SEQ ID NO: 05** | Hepatitis-B-virus-derived peptide. |
| **SEQ ID NO: 06** | Human T-cell lymphotropic virus type 1 derived peptide. |
| **SEQ ID NO: 07** | V-jun Sarcoma Virus 17 Oncogene Homolog (JUN) derived peptide. |
| **SEQ ID NO: 08** | Human adenovirus type 3-derived peptide. |
| **SEQ ID NO: 09** | Hepatitis-C-virus-derived peptide. |
| **SEQ ID NO: 10** | Human β2-microglobulin amino acid sequence. |
| **SEQ ID NO: 11** | Human HLA-A*0201 α1 - α3 chain amino acid sequence. |
| **SEQ ID NO: 12-23** | Linker peptide amino acid sequences. |
| **SEQ ID NO: 24** | Human IgG1 heavy chain hinge polypeptide amino acid sequence. |
| **SEQ ID NO: 25** | Human IgG1 heavy chain hinge variant polypeptide amino acid sequence. |
| **SEQ ID NO: 26** | Human IgG2 heavy chain hinge polypeptide amino acid sequence. |
| **SEQ ID NO: 27** | Human IgG2 heavy chain hinge variant polypeptide amino acid sequence. |
| **SEQ ID NO: 28** | Human IgG2 heavy chain hinge variant polypeptide amino acid sequence. |
| **SEQ ID NO: 29** | Human IgG3 heavy chain hinge polypeptide amino acid sequence. |
| **SEQ ID NO: 30** | Human IgG4 heavy chain hinge polypeptide amino acid sequence. |
| **SEQ ID NO: 31** | Human IgG1 CH2 domain amino acid sequence. |
| **SEQ ID NO: 32** | Human IgG1 CH2 domain L234A, L235A mutant amino acid sequence. |
| **SEQ ID NO: 33** | Human IgG1 CH2 domain L234A, L235A, P329G amino acid sequence. |
| **SEQ ID NO: 34** | Human IgG1 CH3 domain amino acid sequence. |
| **SEQ ID NO: 35** | Human IgG1 CH3 domain knob variant amino acid sequence. |
| **SEQ ID NO: 36** | Human IgG1 CH3 domain hole variant amino acid sequence. |
| **SEQ ID NO: 37** | Human IgG1 Fc-region amino acid sequence. |
| **SEQ ID NO: 38** | Human IgG1 Fc-region L234A, L235A mutant amino acid sequence. |
| **SEQ ID NO: 39** | Human IgG1 Fc-region L234A, L235A mutant and hole variant amino acid sequence. |
| **SEQ ID NO: 40** | Human IgG1 Fc-region L234A, L235A mutant and knob variant amino acid sequence. |
| **SEQ ID NO: 41** | Humanized anti-IGF-1R monoclonal light chain antibody amino acid sequence (kappa). |
| **SEQ ID NO: 42** | Humanized anti-IGF-1R monoclonal heavy chain antibody amino acid sequence (IgG1 L234A, L235A mutant). |
| **SEQ ID NO: 43** | Humanized anti-IGF-1R monoclonal heavy chain antibody amino acid sequence (IgG1 L234A, L235A mutant and knob variant). |
| **SEQ ID NO: 44** | Humanized anti-IGF-1R monoclonal heavy chain antibody amino acid sequence (IgG1 L234A, L235A mutant and hole variant). |
| **SEQ ID NO: 45** | Human IgG1 Fc-region mutant hinge region and L234A, L235A mutant and knob variant. |
| **SEQ ID NO: 46** | Disulfide-stabilized single chain Fv of humanized anti-IGF-1R monoclonal antibody. |
| **SEQ ID NO: 47-51** | Shortened human antibody heavy chain hinge polypeptide amino acid sequences. |
| **SEQ ID NO: 52-66** | Dengue virus-derived peptides. |
| **SEQ ID NO: 67-112** | Human cytomegalovirus-derived peptides. |

### I. DEFINITIONS

"Affinity" refers to the strength of the sum total of non-covalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

The term "amino acid" as used within this application denotes the group of carboxy α-amino acids, which directly or in form of a precursor can be encoded by a nucleic acid. The individual amino acids are encoded by nucleic acids consisting of three nucleotides, so called codons or base-triplets. Each amino acid is encoded by at least one codon. This is known as "degeneration of the genetic code". The term "amino acid" as used within this application denotes the naturally occurring carboxy α-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

The terms "anti-target antibody" and "an antibody that binds to a target" refer to an antibody that is capable of binding a target with sufficient affinity such that the antibody is useful as a diagnostic and/or therapeutic agent in targeting the target. In certain embodiments, an antibody that binds to the target has a dissociation constant (Kd) of ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M).

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g. scFv); single domain antibodies; and multispecific antibodies formed from antibody fragments.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called α, δ, ε, γ, and µ, respectively.

The term "class I MHC molecule with a relative frequency of" denotes that the respective class I MHC molecule has a frequency of occurrence in a specific population of humans or within all humans of the given relative frequency. That is a class I MHC molecule with a relative frequency of 10 % or more can be found in 10 % or more of all humans of a specific population, such as e.g. in 27.2 % of all humans of European origin.

The "conjugation" of a complex to its conjugation partner can be performed by different methods, such as chemical binding, or binding via a specific binding pair. The term "conjugation partner" denotes e.g. polypeptides, detectable labels, members of specific binding pairs. In one embodiment the conjugation of complex to its conjugation partner is performed by chemically binding via N-terminal and/or ε-amino groups (lysine), ε-amino groups of different lysins, carboxy-, sulfhydryl-, hydroxyl-, and/or phenolic functional groups of the amino acid sequence of the parts of the complex, and/or sugar alcohol groups of the carbohydrate structure of the complex. In one embodiment the complex is conjugated to its conjugation partner via a specific binding pair.

The term "cytotoxic agent" as used herein refers to a substance that inhibits or prevents a cellular function and/or causes cell death or destruction. Cytotoxic agents include but are not limited to radioactive isotopes (e.g., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents.

Chromogens (fluorescent or luminescent groups and dyes), enzymes, NMR-active groups or metal particles, haptens, e.g. digoxigenin, are examples of "detectable labels". The detectable label can also be a photoactivatable crosslinking group, e.g. an azido or an azirine group. Metal chelates which can be detected by electrochemiluminescense are also suitable signal-emitting groups, with particular interest being given to ruthenium chelates, e.g. a ruthenium (bispyridyl)₃²⁺ chelate. Suitable ruthenium labeling groups are described, for example, in EP 0 580 979, WO 90/05301, WO 90/11511, and WO 92/14138. For direct detection the labeling group can be selected from any known detectable marker groups, such as dyes, luminescent labeling groups such as chemiluminescent groups, e.g. acridinium esters or dioxetanes, or fluorescent dyes, e.g. fluorescein, coumarin, rhodamine, oxazine, resorufin, cyanine and derivatives thereof. Other examples of labeling groups are luminescent metal complexes, such as ruthenium or europium complexes, enzymes, e.g. as used for ELISA or for CEDIA (Cloned Enzyme Donor Immunoassay, e.g. EP-A-0 061 888), and radioisotopes.

"Effector functions" refer to those biological activities attributable to the Fc-region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

An "effective amount" of an agent, e.g., a pharmaceutical formulation, refers to an amount effective, at dosages and for periods of time necessary, to achieve the desired therapeutic or prophylactic result.

The term "expression" as used herein refers to transcription and/or translation and secretion processes occurring within a cell. The level of transcription of a nucleic acid sequence of interest in a cell can be determined on the basis of the amount of corresponding mRNA that is present in the cell. For example, mRNA transcribed from a sequence of interest can be quantitated by RT-PCR or by Northern hybridization (see Sambrook, et al., Molecular Cloning: A Laboratory Manual, Second Edition, Cold Spring Harbor Laboratory Press, Cold Spring Harbor, N.Y. (1989)). Polypeptides encoded by a nucleic acid can be quantitated by various methods, e.g. by ELISA, by assaying for the biological activity of the polypeptide, or by employing assays that are independent of such activity, such as Western blotting or radioimmunoassay, using immunoglobulins that recognize and bind to the polypeptide (see Sambrook, et al., (1989), supra).

An "expression cassette" denotes a construct that contains the necessary regulatory elements, such as promoter and polyadenylation site, for expression of at least the contained nucleic acid in a cell.

The term "expression machinery" denotes the sum of the enzymes, cofactors, etc. of a cell that is involved in the steps beginning with the transcription step of a nucleic acid or gene (i.e. also called "gene expression machinery") to the post-translational modification of the polypeptide encoded by the nucleic acid. The expression machinery e.g. comprises the steps of transcription of DNA into pre-mRNA, pre-mRNA splicing to mature mRNA, translation into a polypeptide of the mRNA, and post translational modification of the polypeptide.

An "expression plasmid" is a nucleic acid providing all required elements for the expression of the comprised structural gene(s) in a host cell. Typically, an expression plasmid comprises a prokaryotic plasmid propagation unit, e.g. for E. coli, comprising an origin of replication, and a selectable marker, an eukaryotic selection marker, and one or more expression cassettes for the expression of the structural gene(s) of interest each comprising a promoter, a structural gene, and a transcription terminator including a polyadenylation signal. Gene expression is usually placed under the control of a promoter, and such a structural gene is said to be "operably linked to" the promoter. Similarly, a regulatory element and a core promoter are operably linked if the regulatory element modulates the activity of the core promoter.

The term "Fc-region" herein is used to define a C-terminal region of an immunoglobulin heavy chain that contains at least a portion of the constant region. The term includes native sequence Fc-regions and variant Fc-regions. In one embodiment, a human IgG heavy chain Fc-region extends from Cys226, or from Pro230, to the carboxyl-terminus of the heavy chain. However, the C-terminal lysine (Lys447) of the Fc-region may or may not be present. Unless otherwise specified herein, numbering of amino acid residues in the Fc-region or constant region is according to the EU numbering system, also called the EU index, as described in Kabat, E.A., et al., Sequences of Proteins of Immunological Interest, 5th ed., Public Health Service, National Institutes of Health, Bethesda, MD (1991), NIH Publication 91-3242.

An "Fc-region" is a term well known and defined on basis of the papain cleavage of an antibody heavy chain. The complexes as reported herein may comprise in one embodiment as antibody heavy chain hinge region polypeptide a human Fc-region or an Fc-region derived from human origin. In a further embodiment the Fc-region is either an Fc-region of a human antibody of the subclass IgG4 or an Fc-region of a human antibody of the subclass IgG1, IgG2, or IgG3, which is modified in such a way that no Fcγ receptor (e.g. FcγRIIIa) binding and/or no C1q binding can be detected. In one embodiment the Fc-region is a human Fc-region and especially either from human IgG4 subclass or a mutated Fc-region from human IgG1 subclass. In one embodiment the Fc-region is from human IgG1 subclass with mutations L234A and L235A. While IgG4 shows reduced Fcγ receptor (FcγRIIIa) binding, antibodies of other IgG subclasses show strong binding. However Pro238, Asp265, Asp270, Asn297 (loss of Fc carbohydrate), Pro329, Leu234, Leu235, Gly236, Gly237, Ile253, Ser254, Lys288, Thr307, Gln311, Asn434, or/and His435 are residues which, if altered, provide also reduced Fcγ receptor binding (Shields, R.L., et al., J. Biol. Chem. 276 (2001) 6591-6604; Lund, J., et al., FASEB J. 9 (1995) 115-119; Morgan, A., et al., Immunology 86 (1995) 319-324; EP 0 307 434). In one embodiment a complex as reported herein is in regard to Fcγ receptor binding of IgG4 subclass or of IgG1 or IgG2 subclass, with a mutation in L234, L235, and/or D265, and/or contains the PVA236 mutation. In one embodiment the mutations are S228P, L234A, L235A, L235E, and/or PVA236 (PVA236 denotes that the amino acid sequence ELLG (given in one letter amino acid code) from amino acid position 233 to 236 of IgG1 or EFLG of IgG4 is replaced by PVA). In one embodiment the mutations are S228P of IgG4, and L234A and L235A of IgG1. The Fc-region of an antibody is directly involved in ADCC (antibody-dependent cell-mediated cytotoxicity) and CDC (complement-dependent cytotoxicity). A complex which does not bind Fcγ receptor and/or complement factor C1q does not elicit antibody-dependent cellular cytotoxicity (ADCC) and/or complement dependent cytotoxicity (CDC).

The terms "host cell", "host cell line", and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

The term "cell" includes both prokaryotic cells, which are used for propagation of plasmids, and eukaryotic cells, which are used for the expression of a nucleic acid. In one embodiment the eukaryotic cell is a mammalian cell. In one embodiment the mammalian cell is selected from the group of mammalian cells comprising CHO cells (e.g. CHO K1, CHO DG44), BHK cells, NS0 cells, Sp2/0 cells, HEK 293 cells, HEK 293 EBNA cells, PER.C6® cells, and COS cells.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

An "immunoconjugate" denotes a complex as reported herein conjugated to one or more heterologous molecule(s), including but not limited to a cytotoxic agent.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

An "isolated" complex is one which has been separated from a component of its natural environment. In some embodiments, a complex is purified to greater than 95 % or 99 % purity as determined by, for example, electrophoretic (e.g., SDS-PAGE, isoelectric focusing (IEF), capillary electrophoresis) or chromatographic (e.g., ion exchange or reverse phase HPLC).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "one fusion polypeptide" denotes exactly one fusion polypeptide as defined and excludes the presence of a further fusion polypeptide as defined. The term "one" denotes "exactly one" or "a single".

"Operably linked" refers to a juxtaposition of two or more components, wherein the components so described are in a relationship permitting them to function in their intended manner. For example, a promoter and/or enhancer are operably linked to a coding sequence, if it acts in cis to control or modulate the transcription of the linked sequence. Generally, but not necessarily, the DNA sequences that are "operably linked" are contiguous and, where necessary to join two protein encoding regions such as a secretory leader and a polypeptide, contiguous and in (reading) frame. However, although an operably linked promoter is generally located upstream of the coding sequence, it is not necessarily contiguous with it. Enhancers do not have to be contiguous. An enhancer is operably linked to a coding sequence if the enhancer increases transcription of the coding sequence. Operably linked enhancers can be located upstream, within or downstream of coding sequences and at considerable distance from the promoter. A polyadenylation site is operably linked to a coding sequence if it is located at the downstream end of the coding sequence such that transcription proceeds through the coding sequence into the polyadenylation sequence. A translation stop codon is operably linked to an exonic nucleic acid sequence if it is located at the downstream end (3' end) of the coding sequence such that translation proceeds through the coding sequence to the stop codon and is terminated there. Linking is accomplished by recombinant methods known in the art, e.g., using PCR methodology and/or by ligation at convenient restriction sites. If convenient restriction sites do not exist, then synthetic oligonucleotide adaptors or linkers are used in accord with conventional practice.

The term "package insert" is used to refer to instructions customarily included in commercial packages of therapeutic products, that contain information about the indications, usage, dosage, administration, combination therapy, contraindications and/or warnings concerning the use of such therapeutic products.

The term "peptide linker" denotes amino acid sequences of natural and/or synthetic origin. They consist of a linear amino acid chain wherein the 20 naturally occurring amino acids are the monomeric building blocks. The peptide linker has a length of from 1 to 50 amino acids, in one embodiment between 1 and 28 amino acids, in a further embodiment between 2 and 25 amino acids. The peptide linker may contain repetitive amino acid sequences or sequences of naturally occurring polypeptides. The linker has the function to ensure that polypeptides conjugated to each other can perform their biological activity by allowing the polypeptides to fold correctly and to be presented properly. In one embodiment the peptide linker is rich in glycine, glutamine, and/or serine residues. These residues are arranged e.g. in small repetitive units of up to five amino acids, such as GS (SEQ ID NO: 12), GGS (SEQ ID NO: 13), GGGS (SEQ ID NO: 14), and GGGGS (SEQ ID NO: 19). This small repetitive unit may be repeated for one to five times. At the amino- and/or carboxy-terminal ends of the multimeric unit up to six additional arbitrary, naturally occurring amino acids may be added. Other synthetic peptidic linkers are composed of a single amino acid, which is repeated between 10 to 20 times and may comprise at the amino- and/or carboxy-terminal end up to six additional arbitrary, naturally occurring amino acids. All peptidic linkers can be encoded by a nucleic acid molecule and therefore can be recombinantly expressed. As the linkers are themselves peptides, the polypeptide connected by the linker are connected to the linker via a peptide bond that is formed between two amino acids.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

A "polypeptide" is a polymer consisting of amino acids joined by peptide bonds, whether produced naturally or synthetically. Polypeptides of less than about 25 amino acid residues may be referred to as "peptides", whereas molecules consisting of two or more polypeptides or comprising one polypeptide of more than 100 amino acid residues may be referred to as "proteins". A polypeptide may also comprise non-amino acid components, such as carbohydrate groups, metal ions, or carboxylic acid esters. The non-amino acid components may be added by the cell, in which the polypeptide is expressed, and may vary with the type of cell. Polypeptides are defined herein in terms of their amino acid backbone structure or the nucleic acid encoding the same. Additions such as carbohydrate groups are generally not specified, but may be present nonetheless.

A "structural gene" denotes the region of a gene without a signal sequence, i.e. the coding region.

The term "T-cell response eliciting peptide" denotes a peptide that is presented in the peptide-binding grove of a class I MHC complex and which is recognized by circulating memory or effector T-cells. Recognition of the peptide results in an immune response effecting the removal of the cell presenting such a peptide-class I MHC complex.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt, T.J., et al., Kuby Immunology, 6th ed., W.H. Freeman and Co., N.Y. (2007), page 91) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano, S., et al., J. Immunol. 150 (1993) 880-887; Clackson, T., et al., Nature 352 (1991) 624-628).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors".

### II. COMPOSITIONS AND METHODS

### A. Exemplary complexes

Herein is reported an antigen binding complex comprising as first part an antibody derived part that specifically binds to a target antigen, and as second part a virus-derived peptide linked to a MHC class I protein complex.

With the complex as reported herein existing virus-specific circulating cytotoxic T-cells (T-memory-cells and/or T-effector-cells) of an individual can be directed to cells expressing the target antigen, to which the antibody derived part of the complex specifically binds to, by dressing these cells with MHC class I complexes mimicking an acute viral infection.

In one aspect, the invention is based, in part, on the finding that a complex as reported herein, which comprises as first part a virus-derived peptide linked to a MHC class I protein and as second part an antibody derived disulfide-linked molecule, can be used to direct the existing virus-specific cytotoxic T-cells of an individual to cells expressing a target antigen mimicking an acute viral infection and thereby removal of the cells expressing the target antigen can be initiated.

In certain embodiments a complex comprising a fusion polypeptide comprising (i) a virus-derived peptide, (ii) the soluble HLA-A allele A*0201, and (iii) beta-2-microglobulin, is provided.

Complexes of the invention are useful, e.g., for the diagnosis or treatment of various diseases like cancer or viral infections.

Disclosed herein are fusion polypeptides that bind (i) to a cell surface antigen and (ii) to cytotoxic T-cells. In certain embodiments, the fusion polypeptide binds to the cell surface antigen with an affinity of ≤ 10 nM.

The complexes as reported herein exploit a naturally occurring, highly effective anti-viral immune response to remove/disintegrate target cells, e.g. tumor cells or virus infected cells. The cell removal is achieved by using an individual's own very powerful circulating T-cells that do not need any co-stimulation for their activation. Additionally a small number of therapeutic molecules is needed on the cell surface for mechanism of action (Mottez et al., 1995).

During the treatment the fusion polypeptide can trigger the anti-viral immune response of the individual similar to an immunization and thereby enhancing the efficacy with multiple treatments/applications.

Although only a limited patient population is of a specific allotype (HLA-A allotype: 30 % to 50 % of population with allele A*0201) and the prevalence for specific virus infections is also limited (CMV infection 60 % to 90 % mainly depending on age) an immunization as pretreatment can be used in order to enhance efficacy.

Alternatively an allotype can be used whose frequency within the population is very low, in one embodiment below 1 %. The use of such an allotype may make the use of an immunization step obsolete as the allotype will be recognized by the individual's immune system as foreign and an immune response will be initiated. The targeting antibody needs to be highly cell or antigen specific to limit toxicity and side effects.

Thus by using a complex as reported herein
(i) only a highly specific T-cell population is activated (CD8 positive effector/memory cells specific for a single MHC-peptide complex), all other CD3⁺-cells are not affected (CD4⁺-T-cells: TH1, TH2, TH17, regulatory T-cells);
(ii) the natural response of the individual's immune system is mimicked (normal removal of virus-infected cells); and
(iii) the response to the application will be low at the beginning but can boost during treatment (specific T-cells will be activated and expand in number), therewith initially infusion reactions and initial cytokine release can be reduced.

In one embodiment the method comprises the step of stimulating CD8-positive cytotoxic T-cell by application of a selected virus-derived peptide, e.g. a human cytomegalovirus (HCMV) derived peptide.

It has been shown that the activated CMV-peptide specific T-cells could mediate effective tumor cell removal in vitro (tumor cells loaded with the CMV-derived peptide in vitro).

Virus-infected cells present virus-derived peptides with MHC class I proteins on their cell surface. These are recognized by specific CD8⁺ T-cells which remove/deplete the presenting cells. Cytolytic (cytotoxic) CD8⁺-T-cells (CTL) recognize peptides in MHC class I proteins by their specific T-cell-receptor. The CTLs trigger removal of virus infected cells without the requirement of a co-stimulating signal.

Effector cells, e.g. peripheral blood mononuclear cells (PBMC) or FACS-sorted CD8⁺-T-cells, which can be pre-stimulated with the CMV-derived peptide as comprised in the fusion polypeptide as reported herein can be used.

The HLA-allotype of an individual to be treated has to be recognized.

According to NCBI the HLA-allotypes with a frequency of 10 % or more are distributed as it is shown in the following table.

**Table.**

| **HLA-allotype** | **Australian frequency** | **European frequency** | **North American frequency** | **South-East Asian frequency** |
|---|---|---|---|---|
| **HLA-** | **[%]** | **[%]** | **[%]** | **[%]** |
| A*01:01 | | 16.4 | | |
| A*02:01 | 12.7 | 27.2 | 19.7 | |
| A*02:04 | | | | |
| A*03:01 | | 14.1 | | |
| A*11:01 | 13.5 | | | 20.4 |
| A*24:02 | 25.9 | | 37.7 | 29.9 |
| A*31:01:02 | | | | |
| A*34:01:01 | 40.1 | | | |
| B*07:02 | | 13.9 | | |
| B*08:01 | | 11.8 | | |
| B*13:01 | 23.8 | | | |
| B*15:04 | | | | 11.7 |
| B*15:21 | 10.6 | | | |
| B*44:02 | | 10.6 | | |
| B*56:01 | 16.1 | | | |
| B*56:02 | 10.3 | | | |
| C*01:02 | 24.6 | | | 13.3 |
| C*02:02 | | | 12.7 | |
| C*03:03 | | | | |
| C*03:04 | | | 20.4 | 17.3 |
| C*04:01 | 26.0 | 10.1 | 15.0 | |
| C*04:03 | 13.9 | | | |
| C*05:01 | | 10.6 | | |
| C*06:02 | | | | |
| C*07:01 | | 17.0 | | |
| C*07:02 | | 15.9 | 10.2 | 18.9 |
| C*08:01 | | | | 12.8 |
| C*15:02 | 16.5 | | | |

Thus, one aspect as reported herein is an antigen binding complex, characterized in that it comprises
- one single fusion polypeptide that comprises in N- to C-terminal direction either
   (i) a β2-microglobulin, and
   (ii) the extracellular domains α1, α2, and α3 of a class I MHC molecule with a relative frequency of less than 10 %,
   or
   (i) a T-cell-response eliciting peptide,
   (ii) a β2-microglobulin, and
   (iii) the extracellular domains α1, α2, and α3 of a class I MHC molecule with a relative frequency of 10 % or more,
   and
- two polypeptide chains, which are linked by one or more disulfide bonds, wherein the first disulfide-linked polypeptide chain comprises in N- to C-terminal direction
   (i) an immunoglobulin light or heavy chain variable domain,
   (ii) an immunoglobulin light or heavy chain constant domain, and
   (iii) an antibody heavy chain hinge region polypeptide,
   wherein the light or heavy chain variable domain either alone or in combination with the respective complementary heavy or light chain variable domain specifically binds to an antigen,
   and the second disulfide-linked polypeptide chain comprises an antibody heavy chain hinge region polypeptide,
wherein the fusion polypeptide is
- covalently bound either to the C-terminus or the N-terminus of one of the disulfide-linked polypeptide chains, or
- covalently bound to the N-terminus of an antibody variable domain that is the complementary heavy or light chain variable domain to that comprised in the first disulfide-linked polypeptide chain, or
- covalently bound to the C-terminus of an antibody constant domain that is the complementary heavy or light chain constant domain to that comprised in the first disulfide-linked polypeptide chain.

In one embodiment the fusion polypeptide that comprises in N- to C-terminal direction a β2-microglobulin and the extracellular domains α1, α2, and α3 of a class I MHC molecule with a relative frequency of less than 1 % further comprises at its N-terminus a peptide binding to the MHC-peptide binding grove. In one embodiment the peptide is a T-cell-response eliciting peptide.

In one embodiment the complex is characterized in that it comprises
- one fusion polypeptide that comprises in N- to C-terminal direction either
   (i) a β2-microglobulin, and
   (ii) the extracellular domains α1, α2 and α3 of a class I MHC molecule with a relative frequency of less than 10 %,
   or
   (i) a T-cell-response eliciting peptide,
   (ii) a β2-microglobulin, and
   (iii) the extracellular domains α1, α2, and α3 of a class I MHC molecule with a relative frequency of 10 % or more,
   and
- two polypeptide chains, which are linked by one or more disulfide bonds, wherein the first disulfide-linked polypeptide chain comprises in N- to C-terminal direction
   (i) an immunoglobulin light or heavy chain variable domain,
   (ii) an immunoglobulin light or heavy chain constant domain, and
   (iii) an antibody heavy chain hinge region polypeptide,
   wherein the light or heavy chain variable domain either alone or in combination with the respective complementary heavy or light chain variable domain specifically binds to an antigen,
   and the second disulfide-linked polypeptide chain comprises an antibody heavy chain hinge region polypeptide,
wherein the fusion polypeptide is
- covalently bound either to the C-terminus or the N-terminus of one of the disulfide-linked polypeptide chains, or
- covalently bound to the N-terminus of an antibody variable domain that is the complementary heavy or light chain variable domain to that comprised in the first disulfide-linked polypeptide chain, or
- covalently bound to the C-terminus of an antibody constant domain that is the complementary heavy or light chain constant domain to that comprised in the first disulfide-linked polypeptide chain.

In one embodiment the complex is characterized in that it comprises
- one fusion polypeptide that comprises in N- to C-terminal direction
   (i) a T-cell-response eliciting peptide,
   (ii) a β2-microglobulin, and
   (iii) the extracellular domains α1, α2, and α3 of a class I MHC molecule with a relative frequency of 10 % or more,
   and
- two polypeptide chains, which are linked by one or more disulfide bonds, wherein the first disulfide-linked polypeptide chain comprises in N- to C-terminal direction
   (i) an immunoglobulin light chain variable domain (VL) and a constant domain selected from immunoglobulin light chain constant domain (CL) and immunoglobulin heavy chain first constant domain (CH1), or
      an immunoglobulin heavy chain variable domain (VH) and a constant domain selected from immunoglobulin heavy chain first constant domain (CH1) and immunoglobulin light chain constant domain (CL), and
   (ii) an antibody heavy chain hinge region polypeptide,
      wherein the light or heavy chain variable domain either alone or in combination with the respective complementary heavy or light chain variable domain specifically binds to an antigen,
   and the second disulfide-linked polypeptide chain comprises an antibody heavy chain hinge region polypeptide,
wherein the fusion polypeptide is
- covalently bound either to the C-terminus or the N-terminus of one of the disulfide-linked polypeptide chains, or
- covalently bound to the N-terminus of an antibody variable domain that is the complementary heavy or light chain variable domain to that comprised in the first disulfide-linked polypeptide chain, or
- covalently bound to the C-terminus of an antibody constant domain that is the complementary heavy or light chain constant domain to that comprised in the first disulfide-linked polypeptide chain.

In one embodiment the complex is characterized in that it comprises
- one fusion polypeptide that comprises in N- to C-terminal direction
   (i) a T-cell-response eliciting peptide,
   (ii) a β2-microglobulin, and
   (iii) the extracellular domains α1, α2, and α3 of a class I MHC molecule with a relative frequency of 10 % or more,
   and
- two polypeptide chains, which are linked by one or more disulfide bonds, wherein the first disulfide-linked polypeptide chain comprises in N- to C-terminal direction
   (i) an immunoglobulin light chain variable domain (VL) and a constant domain selected from immunoglobulin light chain constant domain (CL) and immunoglobulin heavy chain first constant domain (CH1), or
      an immunoglobulin heavy chain variable domain (VH) and a constant domain selected from immunoglobulin heavy chain first constant domain (CH1) and immunoglobulin light chain constant domain (CL), and
   (ii) an antibody heavy chain hinge region polypeptide, and
   (iii) an immunoglobulin heavy chain second (CH2) and third (CH3) constant domain,
   wherein the light or heavy chain variable domain either alone or in combination with the respective complementary heavy or light chain variable domain specifically binds to an antigen,
   and the second disulfide-linked polypeptide chain comprises an antibody heavy chain hinge region polypeptide,
wherein the fusion polypeptide is
- covalently bound either to the C-terminus or the N-terminus of one of the disulfide-linked polypeptide chains, or
- covalently bound to the N-terminus of an antibody variable domain that is the complementary heavy or light chain variable domain to that comprised in the first disulfide-linked polypeptide chain, or
- covalently bound to the C-terminus of an antibody constant domain that is the complementary heavy or light chain constant domain to that comprised in the first disulfide-linked polypeptide chain.

In one embodiment the complex is characterized in that it comprises
- one fusion polypeptide that comprises in N- to C-terminal direction
   (i) a T-cell-response eliciting peptide,
   (ii) a β2-microglobulin, and
   (iii) the extracellular domains α1, α2, and α3 of a class I MHC molecule with a relative frequency of 10 % or more,
   and
- two polypeptide chains, which are linked by one or more disulfide bonds, wherein the first disulfide-linked polypeptide chain comprises in N- to C-terminal direction
   (i) an immunoglobulin light chain variable domain (VL) and a constant domain selected from immunoglobulin light chain constant domain (CL) and immunoglobulin heavy chain first constant domain (CH1), or
      an immunoglobulin heavy chain variable domain (VH) and a constant domain selected from immunoglobulin heavy chain first constant domain (CH1) and immunoglobulin light chain constant domain (CL), and
   (ii) an antibody heavy chain hinge region polypeptide, and
   (iii) an immunoglobulin heavy chain second (CH2) and third (CH3) constant domain,
   wherein the light or heavy chain variable domain either alone or in combination with the respective complementary heavy or light chain variable domain specifically binds to an antigen,
   and the second disulfide-linked polypeptide chain comprises in N- to C-terminal direction an antibody heavy chain hinge region polypeptide, an immunoglobulin heavy chain second constant region (CH2), and an immunoglobulin heavy chain third constant region (CH3),
wherein the fusion polypeptide is
- covalently bound either to the C-terminus or the N-terminus of one of the disulfide-linked polypeptide chains, or
- covalently bound to the N-terminus of an antibody variable domain that is the complementary heavy or light chain variable domain to that comprised in the first disulfide-linked polypeptide chain, or
- covalently bound to the C-terminus of an antibody constant domain that is the complementary heavy or light chain constant domain to that comprised in the first disulfide-linked polypeptide chain.

In one embodiment the complex is characterized in that it comprises
- one fusion polypeptide that comprises in N- to C-terminal direction
   (i) a T-cell-response eliciting peptide,
   (ii) a β2-microglobulin, and
   (iii) the extracellular domains α1, α2, and α3 of a class I MHC molecule with a relative frequency of 10 % or more,
   and
- two polypeptide chains, which are linked by one or more disulfide bonds, wherein the first disulfide-linked polypeptide chain comprises in N- to C-terminal direction
   (i) an immunoglobulin light chain variable domain (VL) and a constant domain selected from immunoglobulin light chain constant domain (CL) and immunoglobulin heavy chain first constant domain (CH1), or
      an immunoglobulin heavy chain variable domain (VH) and a constant domain selected from immunoglobulin heavy chain first constant domain (CH1) and immunoglobulin light chain constant domain (CL), and
   (ii) an antibody heavy chain hinge region polypeptide, and
   (iii) an immunoglobulin heavy chain second (CH2) and third (CH3) constant domain,
   wherein the light or heavy chain variable domain either alone or in combination with the respective complementary heavy or light chain variable domain specifically binds to an antigen,
   and the second disulfide-linked polypeptide chain comprises in N- to C-terminal direction
   (i) an immunoglobulin light chain variable domain (VL) and a constant domain selected from immunoglobulin light chain constant domain (CL) and immunoglobulin heavy chain first constant domain (CH1), or
      an immunoglobulin heavy chain variable domain (VH) and a constant domain selected from immunoglobulin heavy chain first constant domain (CH1) and immunoglobulin light chain constant domain (CL),
   (ii) an antibody heavy chain hinge region polypeptide, and
   (iii) an immunoglobulin heavy chain second constant region (CH2), and an immunoglobulin heavy chain third constant region (CH3),
wherein the fusion polypeptide is
- covalently bound either to the C-terminus or the N-terminus of one of the disulfide-linked polypeptide chains, or
- covalently bound to the N-terminus of an antibody variable domain that is the complementary heavy or light chain variable domain to that comprised in the first disulfide-linked polypeptide chain, or
- covalently bound to the C-terminus of an antibody constant domain that is the complementary heavy or light chain constant domain to that comprised in the first disulfide-linked polypeptide chain.

In one embodiment the complex is characterized in that it comprises
- one fusion polypeptide that comprises in N- to C-terminal direction
   (i) a T-cell-response eliciting peptide,
   (ii) a β2-microglobulin, and
   (iii) the extracellular domains α1, α2, and α3 of a class I MHC molecule with a relative frequency of 10 % or more,
- two polypeptide chains, which are linked by one or more disulfide bonds, wherein the first disulfide-linked polypeptide chain comprises in N- to C-terminal direction
   (i) an immunoglobulin light chain variable domain (VL) and a constant domain selected from immunoglobulin light chain constant domain (CL) and immunoglobulin heavy chain first constant domain (CH1), or
      an immunoglobulin heavy chain variable domain (VH) and a constant domain selected from immunoglobulin heavy chain first constant domain (CH1) and immunoglobulin light chain constant domain (CL), and
   (ii) an antibody heavy chain hinge region polypeptide, and
   (iii) an immunoglobulin heavy chain second (CH2) and third (CH3) constant domain,
   wherein the light or heavy chain variable domain either alone or in combination with the respective complementary heavy or light chain variable domain specifically binds to an antigen,
   and the second disulfide-linked polypeptide chain comprises in N- to C-terminal direction
   (i) an immunoglobulin light chain variable domain (VL) and a constant domain selected from immunoglobulin light chain constant domain (CL) and immunoglobulin heavy chain first constant domain (CH1), or
      an immunoglobulin heavy chain variable domain (VH) and a constant domain selected from immunoglobulin heavy chain first constant domain (CH1) and immunoglobulin light chain constant domain (CL),
   (ii) an antibody heavy chain hinge region polypeptide, and
   (iii) an immunoglobulin heavy chain second constant region (CH2), and an immunoglobulin heavy chain third constant region (CH3),
- one polypeptide chain that comprises in N- to C-terminal direction an immunoglobulin variable domain complementary to the variable domain in the first disulfide-linked polypeptide chain and a constant domain complementary to the constant domain following the variable domain in the first disulfide-linked polypeptide chain,
wherein the fusion polypeptide is
- covalently bound either to the C-terminus or the N-terminus of one of the disulfide-linked polypeptide chains, or
- covalently bound to the N-terminus of an antibody variable domain that is the complementary heavy or light chain variable domain to that comprised in the first disulfide-linked polypeptide chain, or
- covalently bound to the C-terminus of an antibody constant domain that is the complementary heavy or light chain constant domain to that comprised in the first disulfide-linked polypeptide chain.

In one embodiment the complex is characterized in that it comprises
- one fusion polypeptide that comprises in N- to C-terminal direction
   (i) a T-cell-response eliciting peptide,
   (ii) a β2-microglobulin,
   (iii) the extracellular domains α1, α2, and α3 of a class I MHC molecule with a relative frequency of 10 % or more,
   (iv) an antibody heavy chain hinge region polypeptide,
   (v) an immunoglobulin heavy chain second (CH2) and third (CH3) constant domain,
   and
- one first polypeptide chain that comprises in N- to C-terminal direction
   (i) an immunoglobulin light chain variable domain (VL) and a constant domain selected from immunoglobulin light chain constant domain (CL) and immunoglobulin heavy chain first constant domain (CH1), or
      an immunoglobulin heavy chain variable domain (VH) and a constant domain selected from immunoglobulin heavy chain first constant domain (CH1) and immunoglobulin light chain constant domain (CL), and
   (ii) an antibody heavy chain hinge region polypeptide, and
   (iii) an immunoglobulin heavy chain second (CH2) and third (CH3) constant domain,
   and
- one second polypeptide chain that comprises in N- to C-terminal direction an immunoglobulin variable domain complementary to the variable domain in the first polypeptide chain and a constant domain complementary to the constant domain in the first polypeptide chain,
wherein the light or heavy chain variable domain either alone or in combination with the respective complementary heavy or light chain variable domain specifically binds to an antigen, and
wherein the fusion polypeptide and the first polypeptide chain are disulfide-linked.

In one embodiment the complex is characterized in that it comprises
- one fusion polypeptide that comprises in N- to C-terminal direction
   (i) a T-cell-response eliciting peptide,
   (ii) a β2-microglobulin,
   (iii) the extracellular domains α1, α2, and α3 of a class I MHC molecule with a relative frequency of 10 % or more,
   (iv) an immunoglobulin light chain variable domain (VL) and a constant domain selected from immunoglobulin light chain constant domain (CL) and immunoglobulin heavy chain first constant domain (CH1), or
      an immunoglobulin heavy chain variable domain (VH) and a constant domain selected from immunoglobulin heavy chain first constant domain (CH1) and immunoglobulin light chain constant domain (CL),
   (v) an antibody heavy chain hinge region polypeptide,
   (vi) an immunoglobulin heavy chain second (CH2) and third (CH3) constant domain,
   and
- one first polypeptide chain that comprises in N- to C-terminal direction
   (i) an antibody heavy chain hinge region polypeptide, and
   (ii) an immunoglobulin heavy chain second (CH2) and third (CH3) constant domain,
   and
- one second polypeptide chain that comprises in N- to C-terminal direction an immunoglobulin variable domain complementary to the variable domain in the first polypeptide chain and a constant domain complementary to the constant domain in the first polypeptide chain,
wherein the light or heavy chain variable domain either alone or in combination with the respective complementary heavy or light chain variable domain specifically binds to an antigen, and
wherein the fusion polypeptide and the first polypeptide chain are disulfide-linked.

In one embodiment the complex is characterized in that it comprises
- one fusion polypeptide that comprises in N- to C-terminal direction
   (i) a T-cell-response eliciting peptide,
   (ii) a β2-microglobulin,
   (iii) the extracellular domains α1, α2, and α3 of a class I MHC molecule with a relative frequency of 10 % or more, and
   (iv) an immunoglobulin light chain variable domain (VL) and a constant domain selected from immunoglobulin light chain constant domain (CL) and immunoglobulin heavy chain first constant domain (CH1), or
      an immunoglobulin heavy chain variable domain (VH) and a constant domain selected from immunoglobulin heavy chain first constant domain (CH1) and immunoglobulin light chain constant domain (CL),
   and
- one first polypeptide chain that comprises in N- to C-terminal direction
   (i) an immunoglobulin variable domain complementary to the variable domain in the fusion polypeptide and a constant domain complementary to the constant domain in the fusion polypeptide,
   (ii) an antibody heavy chain hinge region polypeptide, and
   (iii) an immunoglobulin heavy chain second (CH2) and third (CH3) constant domain,
wherein the light or heavy chain variable domain either alone or in combination with the respective complementary heavy or light chain variable domain specifically binds to an antigen, and
wherein the fusion polypeptide and the first polypeptide chain are disulfide-linked.

In one embodiment the complex is characterized in that it comprises
- one fusion polypeptide that comprises in N- to C-terminal direction
   (i) a T-cell-response eliciting peptide,
   (ii) a β2-microglobulin,
   (iii) the extracellular domains α1, α2, and α3 of a class I MHC molecule with a relative frequency of 10 % or more,
   (iv) an immunoglobulin light chain variable domain (VL) and a constant domain selected from immunoglobulin light chain constant domain (CL) and immunoglobulin heavy chain first constant domain (CH1), or
      an immunoglobulin heavy chain variable domain (VH) and a constant domain selected from immunoglobulin heavy chain first constant domain (CH1) and immunoglobulin light chain constant domain (CL),
   (v) an antibody heavy chain hinge region polypeptide,
   (vi) an immunoglobulin heavy chain second (CH2) and third (CH3) constant domain,
   and
- one first polypeptide chain that comprises in N- to C-terminal direction
   (i) an immunoglobulin light chain variable domain (VL) and a constant domain selected from immunoglobulin light chain constant domain (CL) and immunoglobulin heavy chain first constant domain (CH1), or
      an immunoglobulin heavy chain variable domain (VH) and a constant domain selected from immunoglobulin heavy chain first constant domain (CH1) and immunoglobulin light chain constant domain (CL),
   (ii) an antibody heavy chain hinge region polypeptide, and
   (iii) an immunoglobulin heavy chain second (CH2) and third (CH3) constant domain,
   and
- one second polypeptide chain that comprises in N- to C-terminal direction an immunoglobulin variable domain complementary to the variable domain in the fusion polypeptide chain and a constant domain complementary to the constant domain in the fusion polypeptide chain,
   and
- one third polypeptide chain that comprises in N- to C-terminal direction an immunoglobulin variable domain complementary to the variable domain in the first polypeptide chain and a constant domain complementary to the constant domain in the first polypeptide chain,
wherein the light or heavy chain variable domain of the fusion polypeptide and/or the first polypeptide chain either alone or in combination with the respective complementary heavy or light chain variable domain specifically binds to an antigen, and
wherein the fusion polypeptide and the first polypeptide chain are disulfide-linked.

In one embodiment the complex is characterized in that it comprises
- one fusion polypeptide that comprises in N- to C-terminal direction
   (i) a T-cell-response eliciting peptide,
   (ii) a β2-microglobulin,
   (iii) the extracellular domains α1, α2, and α3 of a class I MHC molecule with a relative frequency of 10 % or more, and
   (iv) an immunoglobulin light chain variable domain (VL) and a constant domain selected from immunoglobulin light chain constant domain (CL) and immunoglobulin heavy chain first constant domain (CH1), or
      an immunoglobulin heavy chain variable domain (VH) and a constant domain selected from immunoglobulin heavy chain first constant domain (CH1) and immunoglobulin light chain constant domain (CL),
   and
- one first polypeptide chain that comprises in N- to C-terminal direction
   (i) an immunoglobulin variable domain complementary to the variable domain in the fusion polypeptide and a constant domain complementary to the constant domain in the fusion polypeptide,
   (ii) an antibody heavy chain hinge region polypeptide, and
   (iii) an immunoglobulin heavy chain second (CH2) and third (CH3) constant domain,
   and
- one second polypeptide chain that comprises in N- to C-terminal direction
   (i) an immunoglobulin light chain variable domain (VL) and a constant domain selected from immunoglobulin light chain constant domain (CL) and immunoglobulin heavy chain first constant domain (CH1), or
      an immunoglobulin heavy chain variable domain (VH) and a constant domain selected from immunoglobulin heavy chain first constant domain (CH1) and immunoglobulin light chain constant domain (CL),
   (ii) an antibody heavy chain hinge region polypeptide, and
   (iii) an immunoglobulin heavy chain second (CH2) and third (CH3) constant domain,
   and
- one third polypeptide chain that comprises in N- to C-terminal direction an immunoglobulin variable domain complementary to the variable domain in the second polypeptide chain and a constant domain complementary to the constant domain in the second polypeptide chain,
wherein the light or heavy chain variable domain of the fusion polypeptide and/or of the second polypeptide chain either alone or in combination with the respective complementary heavy or light chain variable domain specifically binds to an antigen, and
wherein the first polypeptide chain and the second polypeptide chain are disulfide-linked.

In one embodiment the T-cell-response eliciting peptide is a virus-derived peptide. In one embodiment the virus is selected from adenovirus, human herpesvirus 1, human herpesvirus 2, human herpesvirus 4 (Epstein-Barr virus), hepatitis-B-virus, hepatitis-C-virus, human cytomegalovirus, human immunodeficiency virus, human papillomavirus type 16, human papillomavirus type 18, human papillomavirus type 31, human papillomavirus type 33, human papillomavirus type 35, human papillomavirus type 39, human papillomavirus type 45, human papillomavirus type 51, human papillomavirus type 52, human papillomavirus type 56, human papillomavirus type 58, human papillomavirus type 59, human papillomavirus type 68, human papillomavirus type 73, human papillomavirus type 82, human T-cell lymphotropic virus type I, human influenza A virus, human influenza B virus, or vaccinia virus.

In one embodiment the virus-derived peptide is selected from NLVPMVATV (SEQ ID NO: 01), SLYNTVATL (SEQ ID NO: 02), GLCTLVAML (SEQ ID NO: 03), GILGFVFTL (SEQ ID NO: 04), STNRQSGRQ (SEQ ID NO: 5), LLFGYPVYV (SEQ ID NO: 06), FAEGFVRAL (SEQ ID NO: 07), LIVIGILIL (SEQ ID NO: 08), or ILHTPGCV (SEQ ID NO: 09).

In one embodiment the β2-microglobulin is human β2-microglobulin. In one embodiment the β2-microglobulin is consisting of the amino acid sequence of SEQ ID NO: 10.

In one embodiment the class I MHC molecule with a relative frequency of 10 % or more is human HLA-A*0201. In one embodiment the extracellular domains α1, α2, and α3 of a class I MHC molecule is consisting of the amino acid sequence of (SEQ ID NO: 11.

In one embodiment the virus-derived peptide is fused to the β2-microglobulin via a first linker peptide.

In one embodiment the β2-microglobulin is fused to the extracellular domain α1 of a class I MHC molecule via a second linker peptide.

In one embodiment the extracellular domain α3 of a class I MHC molecule is fused to the polypeptide (either disulfide-linked or not disulfide-linked) via a third linker peptide.

In one embodiment the first, second, and third linker peptide is the same or different.

In one embodiment the first linker peptide, the second linker peptide, and the third linker peptide are selected independently from each other from the amino acid sequences GS (SEQ ID NO: 12), GGS (SEQ ID NO: 13), GGGS (SEQ ID NO: 14), GGGSGGGS (SEQ ID NO: 15), GGGSGGGSGGGS (SEQ ID NO: 16), GGGSGGGSGGGSGGGS (SEQ ID NO: 17), GGGSGGGSGGGSGGGSGGGS (SEQ ID NO: 18), GGGGS (SEQ ID NO: 19), GGGGSGGGGS (SEQ ID NO: 20), GGGGSGGGGSGGGGS (SEQ ID NO: 21), GGGGSGGGGSGGGGSGGGGS (SEQ ID NO: 22), and GGGGSGGGGSGGGGSGGGGSGGGGS (SEQ ID NO: 23).

In one embodiment the first linker peptide comprises the amino acid sequence of SEQ ID NO: 21.

In one embodiment the second linker peptide comprises the amino acid sequence of SEQ ID NO: 22.

In one embodiment the third linker peptide comprises the amino acid sequence of SEQ ID NO: 12.

In one embodiment the antibody heavy chain hinge region polypeptide is selected from an antibody heavy chain hinge region polypeptide of a human antibody of the class IgG or the class IgE.

In one embodiment the antibody heavy chain hinge region polypeptide is selected from an antibody heavy chain hinge region polypeptide of a human antibody of the subclass IgG1, or IgG2, or IgG3, or IgG4.

In one embodiment the antibody heavy chain hinge region polypeptide comprises or is consisting of the amino acid sequence of EPKSCDKTHTCPPCP (SEQ ID NO: 24), EPKSADKTHTCPPCP (SEQ ID NO: 25), ERKCCVECPPCP (SEQ ID NO: 26), ERKCAVECPPCP (SEQ ID NO: 27), ERKACVECPPCP (SEQ ID NO: 28), ELKTPLGDTTHTCPRCP (EPKSCDTPPPCPRCP)₃ (SEQ ID NO: 29), or ESKYGPPCPSCP (SEQ ID NO: 30).

In one embodiment the first disulfide-linked polypeptide and/or the second disulfide-linked polypeptide comprises a CH2 domain and/or a CH3 domain of human origin. In one embodiment the CH2 domain and the CH3 of human origin is of a human antibody of the class IgG or IgE. In one embodiment the CH2 domain and the CH3 domain is of a human antibody of the subclass IgG1, or IgG2, or IgG3, or IgG4. In one embodiment the CH2 domain comprises the amino acid sequence of SEQ ID NO: 31. In one embodiment the CH2 domain is of a human antibody of the subclass IgG1 or IgG2 and comprises at least one mutation of E233, L234, L235, G236, D265, D270, N297, E318, K320, K322, A327, A330, P331 and/or P329 (numbering according to the EU index of Kabat). In one embodiment the CH2 domain is of a human antibody of the subclass IgG1 or the human subclass IgG2 with the mutations L234A and L235A, and/or the mutations D265A and N297A, and/or contains the PVA236 mutation, and/or contains the mutation P329G. In one embodiment the CH2 domain is of a human antibody of the subclass IgG1 with the mutations L234A and L235A, and/or P329G. In one embodiment the CH2 domain is of a human antibody of the subclass IgG4 with the mutations S228P and/or L235E. In one embodiment the CH2 domain comprises the amino acid sequence of SEQ ID NO: 32 or SEQ ID NO: 33. In one embodiment the CH3 domain comprises the amino acid sequence of SEQ ID NO: 34.

In one embodiment the first disulfide-linked polypeptide comprises the amino acid sequence of SEQ ID NO: 35 and the second disulfide-linked polypeptide comprises the amino acid sequence of SEQ ID NO: 36.

In one embodiment the first and the second disulfide-linked polypeptide comprise the amino acid sequence of SEQ ID NO: 37 or SEQ ID NO: 38.

In one embodiment the first disulfide-linked polypeptide or the second disulfide-linked polypeptide is consisting of the amino acid sequence of SEQ ID NO: 37 or SEQ ID NO: 38.

In one embodiment the first disulfide-linked polypeptide comprises the amino acid sequence of SEQ ID NO: 39 and the second disulfide-linked polypeptide comprises the amino acid sequence of SEQ ID NO: 40.

In one embodiment the polypeptide chains, which are linked by one or more disulfide bonds, are linked by two, or three, or four disulfide bonds.

In one embodiment the complex is characterized in that it comprises
- one fusion polypeptide that comprises in N- to C-terminal direction
   (i) a virus-derived peptide that has an amino acid sequence of SEQ ID NO: 01,
   (ii) a first linker peptide that has an amino acid sequence of SEQ ID NO: 21.
   (iii) a β2-microglobulin that has an amino acid sequence of SEQ ID NO: 10,
   (iv) a second linker peptide that has an amino acid sequence of SEQ ID NO: 22,
   (v) the extracellular domains α1, α2, and α3 of a class I MHC molecule that has an amino acid sequence of SEQ ID NO: 11, and
   (vi) a third linker peptide that has an amino acid sequence of SEQ ID NO: 12,
   and
- two polypeptide chains, which are linked by one or more disulfide bonds,
   wherein the first disulfide-linked polypeptide chain comprises in N- to C-terminal direction
   (i) an immunoglobulin light or heavy chain variable domain,
   (ii) an immunoglobulin light or heavy chain constant domain,
   (iii) an antibody heavy chain hinge region polypeptide,
   (iv) a human IgG1 CH2 domain comprising an amino acid sequence selected from SEQ ID NO: 32, and 33, and
   (v) a human IgG1 CH3 domain comprising an amino acid sequence of SEQ ID NO: 35 or 36,
   and the second disulfide-linked polypeptide chain comprises in N- to C-terminal direction
   (i) an antibody heavy chain hinge region polypeptide,
   (ii) a human IgG1 CH2 domain comprising an amino acid sequence selected from SEQ ID NO: 32, and 33, and
   (iii) a human IgG1 CH3 domain comprising an amino acid sequence of SEQ ID NO: 36 or 35,
wherein the fusion polypeptide is
- covalently bound either to the C-terminus or the N-terminus of the second disulfide-linked polypeptide chain.

In one embodiment the complex is characterized in that it comprises
- one fusion polypeptide that comprises in N- to C-terminal direction
   (i) a virus-derived peptide that has an amino acid sequence of SEQ ID NO: 01,
   (ii) a first linker peptide that has an amino acid sequence of SEQ ID NO: 21.
   (iii) a β2-microglobulin that has an amino acid sequence of SEQ ID NO: 10,
   (iv) a second linker peptide that has an amino acid sequence of SEQ ID NO: 22.
   (v) the extracellular domains α1, α2, and α3 of a class I MHC molecule that has an amino acid sequence of SEQ ID NO: 11, and
   (vi) a third linker peptide that has an amino acid sequence of SEQ ID NO: 12,
   and
- two polypeptide chains, which are linked by one or more disulfide bonds, wherein the first disulfide-linked polypeptide chain comprises in N- to C-terminal direction
   (i) an immunoglobulin light or heavy chain variable domain,
   (ii) an immunoglobulin light or heavy chain constant domain,
   (iii) an antibody heavy chain hinge region polypeptide,
   (iv) a human IgG1 CH2 domain comprising an amino acid sequence selected from SEQ ID NO: 32, and 33, and
   (v) a human IgG1 CH3 domain comprising an amino acid sequence selected from SEQ ID NO: 35 or 36,
   and the second disulfide-linked polypeptide chain comprises an antibody heavy chain hinge region polypeptide,
wherein the fusion polypeptide is
- covalently bound either to the C-terminus or the N-terminus of the first disulfide-linked polypeptide chain, or
- covalently bound to the N-terminus of an antibody variable domain that is the complementary heavy or light chain variable domain to that comprised in the first disulfide-linked polypeptide chain, or
- covalently bound to the C-terminus of an antibody constant domain that is the complementary heavy or light chain constant domain to that comprised in the first disulfide-linked polypeptide chain.

In one embodiment the complex is characterized in that it comprises
- one fusion polypeptide that comprises in N- to C-terminal direction
   (i) a virus-derived peptide that has an amino acid sequence of SEQ ID NO: 01,
   (ii) a first linker peptide that has an amino acid sequence of SEQ ID NO: 21.
   (iii) a β2-microglobulin that has an amino acid sequence of SEQ ID NO: 10,
   (iv) a second linker peptide that has an amino acid sequence of SEQ ID NO: 22.
   (v) the extracellular domains α1, α2, and α3 of a class I MHC molecule that has an amino acid sequence of SEQ ID NO: 11, and
   (vi) a third linker peptide that has an amino acid sequence of SEQ ID NO: 12,
   and
- two polypeptide chains, which are linked by one or more disulfide bonds, wherein the first and the second disulfide-linked polypeptide chain each comprise in N- to C-terminal direction
   (i) an immunoglobulin light or heavy chain variable domain,
   (ii) an immunoglobulin light or heavy chain constant domain,
   (iii) an antibody heavy chain hinge region polypeptide,
   (iv) a human IgG1 CH2 domain comprising an amino acid sequence selected from SEQ ID NO: 32, and 33, and
   (v) a human IgG1 CH3 domain comprising an amino acid sequence selected from SEQ ID NO: 35, and 36,
wherein the fusion polypeptide is
- covalently bound either to the C-terminus or the N-terminus of the second disulfide-linked polypeptide chain, or
- covalently bound to the N-terminus of an antibody variable domain that is the complementary heavy or light chain variable domain to that comprised in the first disulfide-linked polypeptide chain, or
- covalently bound to the C-terminus of an antibody constant domain that is the complementary heavy or light chain constant domain to that comprised in the first disulfide-linked polypeptide chain.

From Figure 14 it can be seen that the complex as reported herein maintains the binding properties of the antibody to which it is fused (Figure 14 b) and c)).

In Figures 15 and 17 the in vitro efficacy and specificity of a complex as reported herein is shown.

The cytotoxicity assay was performed in the presence of CMV-specific CD8⁺ T-cells. It can be seen that complexes comprising a CMV-derived virus peptide induce the lysis/removal/disintegration of the target cells (see Figure 15a) for monovalent antibody, Figure 15b) for bivalent antibody). It can further be seen that the lysis of the target cells is highly specific as the incubation with complexes comprising an EBV-derived viral peptide (Figure 15b)) and control antibodies (Figure 15d) and e)) do not result in extensive cell lysis (the spontaneous lysis is about 3.5 %).

In Figure 17 the lysis of IGF-1R positive lung adenocarcinoma cell line H460M2 is shown.

The EC₅₀ value for a complex comprising a CMV-derived peptide and a bivalent antibody is about 10 ng/ml corresponding to about 50 pM. The determined EC₅₀ value is independent from the target cell to effector cell ratio (see Figure 16; target cell to effector cell ratio from 1:3 to 1:1 corresponding to an effective ratio of 1:0.44 to 1:0.14 (76 % of effector cells are CD8 positive and 19 % are CMV specific)).

Thus, in one embodiment the complex as reported herein has an EC₅₀ value of about 50 pM.

### 1. Affinity

In certain embodiments, a complex as provided herein comprises an antigen binding pair of antibody variable domains. In certain embodiments the variable domain has a dissociation constant (Kd) of ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M) with respect to its antigen.

In one embodiment, Kd is measured using surface plasmon resonance assays.

For example this can be done by using a BIACORE®-2000 or a BIACORE®-3000 instrument (BIAcore, Inc., Piscataway, NJ) at 25 °C with immobilized antigen CM5 chips at ∼10 response units (RU). Briefly, carboxymethylated dextran biosensor chips (CM5, BIAcore, Inc.) are activated with N-ethyl-N'-(3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (∼0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05 % polysorbate 20 (TWEEN-20™) surfactant (PBST) at 25 °C at a flow rate of approximately 25 µl/min. Association rates (kon) and dissociation rates (koff) are calculated using a simple one-to-one Langmuir binding model (BIACORE® Evaluation Software version 3.2) by simultaneously fitting the association and dissociation sensorgrams. The equilibrium dissociation constant (Kd) is calculated as the ratio koff/kon. See, e.g., Chen, Y., et al., J. Mol. Biol. 293 (1999) 865-881.

### 2. Expression

Expression of specific formats of the complex as reported herein (different linker, different combinations of HLA and β2-microglobulin) in HEK 293 and CHO led to an accumulation of the complex, if detectable at all, within the endoplasmatic reticulum, i.e. isolation and secretion of the complex was strongly impaired.

No secretion of a complex to the cultivation medium could be detected when the complex was intended to comprise one of the polypeptides as outlined in the following tables.

**Table.**

| | | | | | | | |
|---|---|---|---|---|---|---|---|
| signal peptide | CMV-derived peptide | G₄S(G₃S)₂-linker | β2-microglobuli n | (G₄S)₃-linker | α1-α2-α3-chains | (G₄S)₂-linker | antibody light chain |
| signal peptide | β2-microglobulin | (G₄S)₃-linker | α1-α2-α3-chains | (G₄S)₂-linker | antibody light chain | | |
| signal peptide | CMV-derived peptide | (G₃S)₂GG-linker | α1-α2-α3-chains | (G₄S)₃-linker | β2-microglobulin | (G₄S)₂-linker | antibody light chain |
| signal peptide | CMV-derived peptide | GGPGGGSG GG-linker | α1-α2-α3-chains | (G₄S)₃-linker | β2-microglobulin | (G₄S)₂-linker | antibody light chain |
| signal peptide | α1-α2-α3-chains | (G₄S)₃-linker | β2-microglobulin | (G₄S)₂-linker | antibody light chain | | |
| signal peptide | CMV-derived peptide | (G₃S)₂GG-linker | α1-α2-α3-chains | (G₄S)₃-linker | antibody light chain | | |
| signal peptide signal peptide | CMV-derived peptide | GGPGGGSG GG-linker | α1-α2-α3-chains | (G₄S)₃-linker (G₄S)₃-linker | antibody light chain | | |
| signal peptide | CMV-derived peptide | (G₃S)₂GG-linker | α1-α2-α3-chains | (G₄S)₃-linker | β2-microglobulin | (G₄S)₂-linker | antibody heavy chain |

**Table.**

| | | | | | |
|---|---|---|---|---|---|
| signal peptide | CMV-derived peptide | β2-microglobulin | α1-α2-α3-chains | IgG-Fc-region | scFv |
| signal peptide | CMV-derived peptide | β2-microglobulin | α1-α2-α3-chains | antibody heavy chain | |

It has been found that the expression, and especially the secretion, of complexes comprising two parts of a virus-derived peptide linked to a MHC class I protein complex, and at least one variable domain and one constant domain of an antibody is not possible in eukaryotic cells.

Further it has been found that the expression, and especially the secretion, of complexes comprising two fusion polypeptide comprising a virus-derived peptide linked to a MHC class I protein complex, at least one variable domain, and a pair of hinge region derived disulfide-linked polypeptides is not possible in eukaryotic cells.

Thus, in a complex as reported herein a fusion polypeptide comprising a virus-derived peptide linked to a MHC class I protein cannot be present more than once and at least one antibody variable domain and one antibody constant domain has to be present in order to allow for the production and the secretion of the complex using eukaryotic cells.

Thus, a complex comprising exactly one fusion polypeptide of a virus-derived peptide linked to a MHC class I protein, an antibody heavy chain hinge region, and at least one antibody variable domain and one antibody constant domain can be recombinantly produced in and secreted from eukaryotic cells. In one embodiment the at least one constant domain is either an antibody heavy chain constant domain 1 (CH1) or an antibody light chain constant domain (CL).

Thus, a complex comprising an antibody heavy chain hinge region, at least one pair of antibody variable domains, optionally an antibody constant domain, and exactly one fusion polypeptide of a virus-derived peptide linked to a MHC class I protein can be recombinantly produced in and secreted from eukaryotic cells. In one embodiment the at least one constant domain is either an antibody heavy chain constant domain 1 (CH1) or an antibody light chain constant domain (CL).

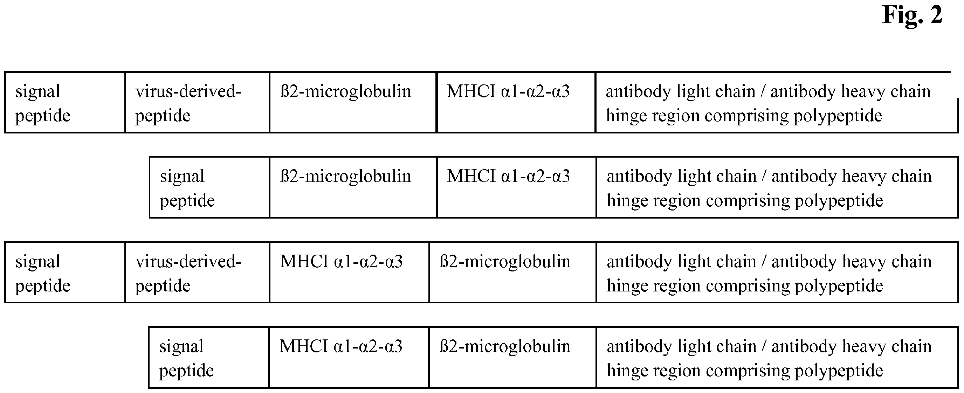

Various combinations of different polypeptides were tested. Secreted expression can be accomplished by e.g. N-terminal fusion of an immunoglobulin-derived signal peptide in complexes wherein the virus-derived peptide is fused N-terminally to the class I MHC molecule. Class I MHC molecule heavy chain (α1-α2-α3- lacking the transmembrane and the cytoplasmatic domain) and light chain (β2-microglobulin) can be changed in order. The different fusion polypeptides were N-terminally fused to either an antibody light chain or an antibody heavy chain hinge region comprising polypeptide. Exemplary combinations are shown in Figure 2.

As can be seen from the following table complexes comprising fusion polypeptides can only be expressed with variable antibody domain and antibody heavy chain hinge region derived polypeptides when a single viral-derived-peptide-microglobulin-HLA-fusion polypeptide is present.

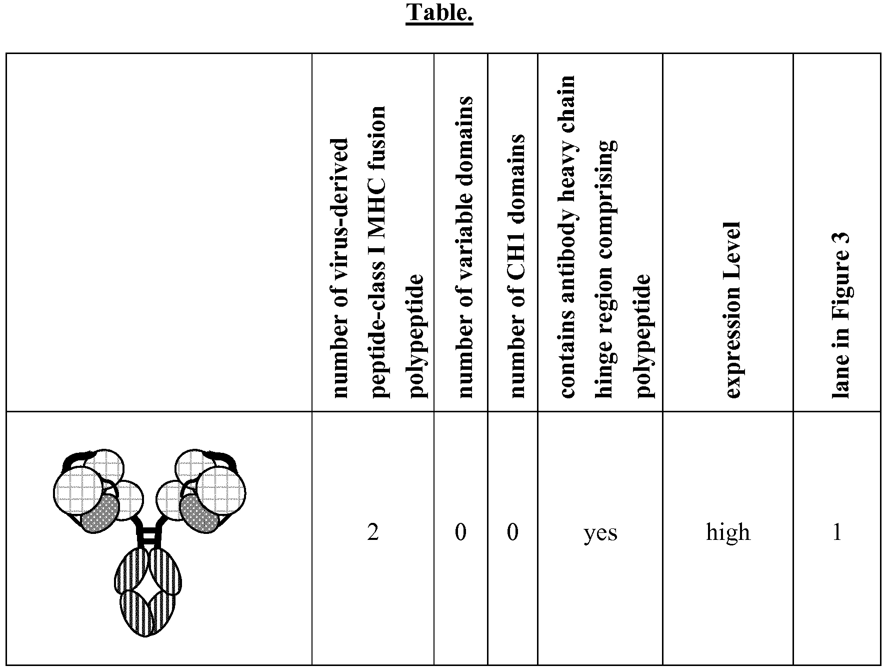

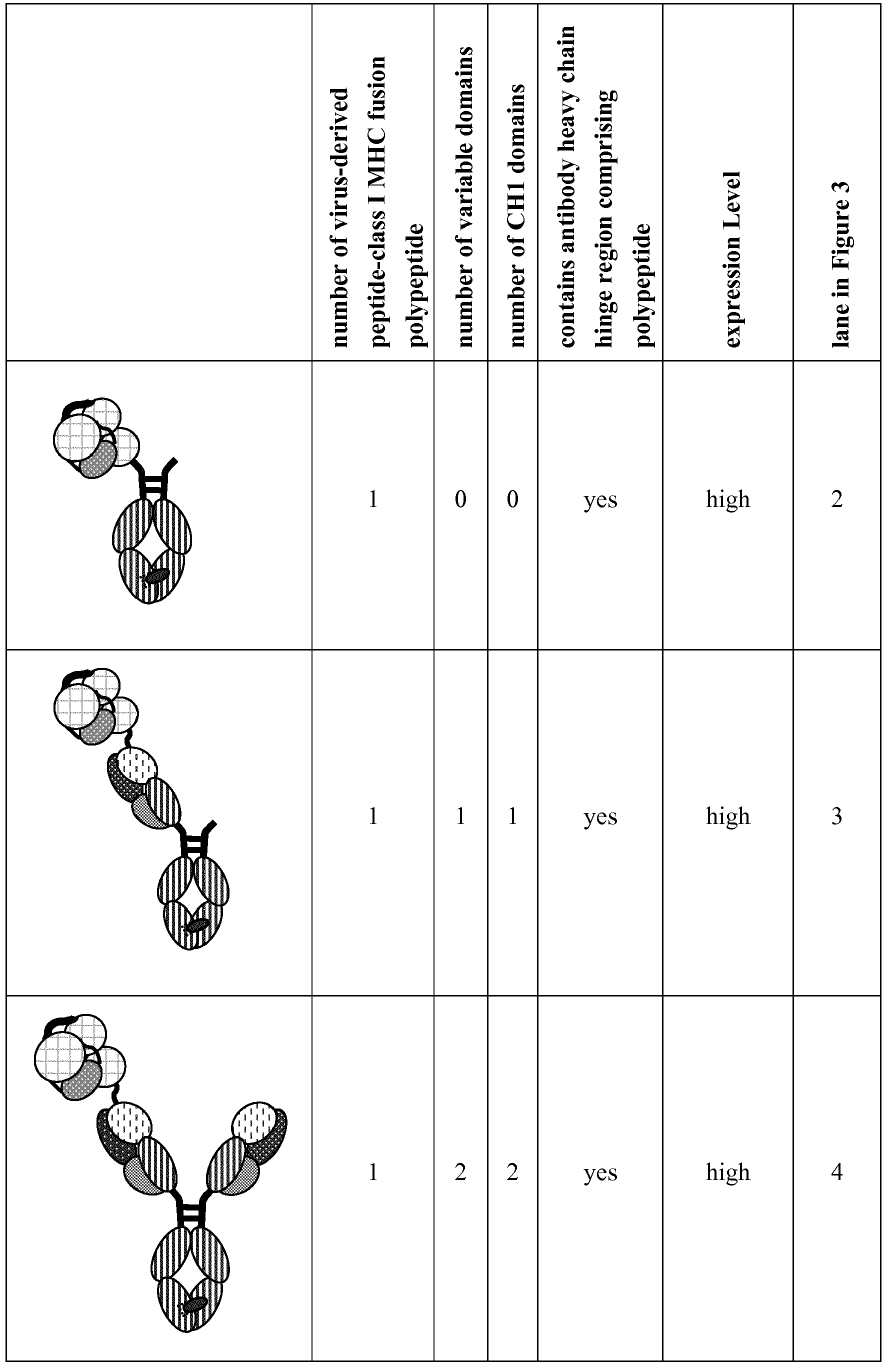

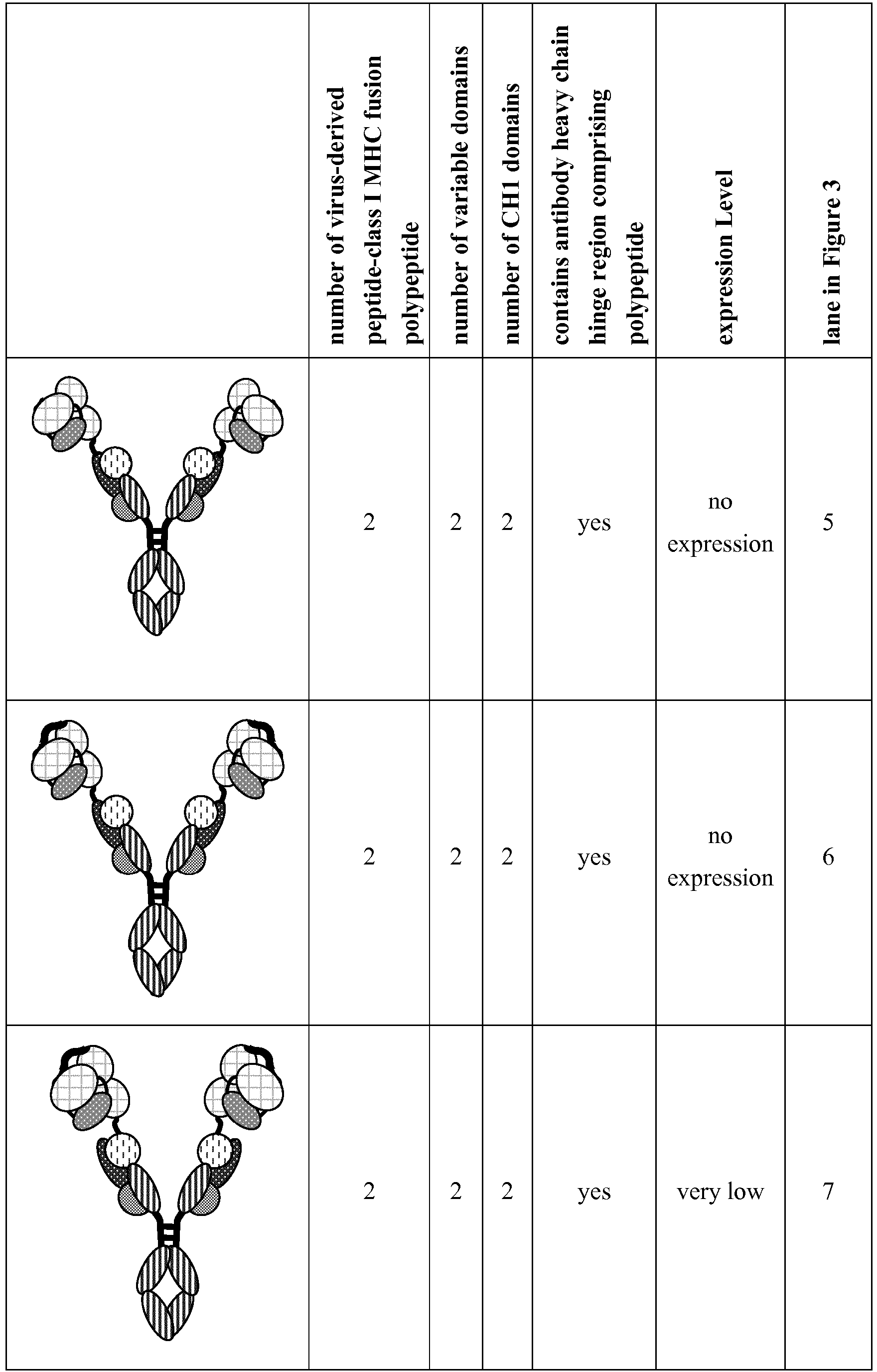

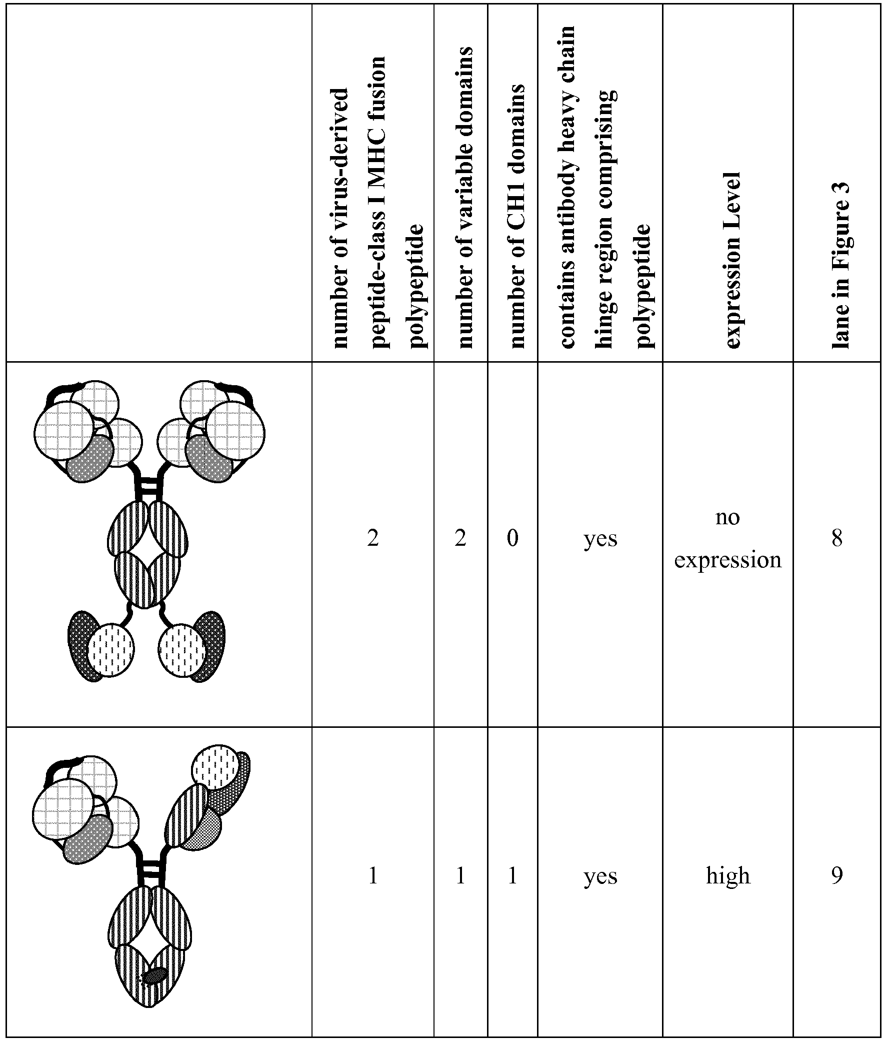

In some embodiments the complex as reported herein comprises different pairs of polypeptides. In order to allow proper pairing of the polypeptides the knobs-into-holes technology or the cross-mAb technology can be used in order to reduce the amount of not correctly associated complex.

The knob modification denotes the mutation T366W in the CH3 domain of an antibody (numbering according to EU index of Kabat).

The hole-modification denotes the mutations T366S, L368A and Y407V in the CH3 domain of an antibody (numbering according to EU index of Kabat).

In addition to the knob and hole modification the mutation S354C in the one CH3 domain and the mutation Y349C in the other CH3 domain can be present.

The cross-mAb technology is reported e.g. in WO 2009/080251, WO 2009/080252, WO 2009/080254, WO 2009/080253, WO 2010/112193, WO 2010/115589, WO 2010/136172, WO 2010/145792, and WO 2010/145793.

### 3. Variants

In certain embodiments, amino acid sequence variants of the complex provided herein are contemplated. For example, it may be desirable to improve the binding affinity and/or other biological properties of the complex. Amino acid sequence variants of the complex may be prepared by introducing appropriate modifications into the nucleotide sequence encoding the polypeptide chains of the complex, or by peptide synthesis. Such modifications include, for example, deletions from, and/or insertions into and/or substitutions of residues within the amino acid sequences of the polypeptides of the complex. Any combination of deletion, insertion, and substitution can be made to arrive at the final construct, provided that the final construct possesses the desired characteristics, e.g., antigen-binding.

### a) Substitution, Insertion, and Deletion Variants

In certain embodiments, complex variants having one or more amino acid substitutions in the polypeptide chains are provided. Conservative substitutions are shown in the following Table under the heading of "preferred substitutions". More substantial changes are provided in the following table under the heading of "exemplary substitutions", and as further described below in reference to amino acid side chain classes. Amino acid substitutions may be introduced into a complex of interest and the products screened for a desired activity, e.g., retained/improved antigen binding, decreased immunogenicity, or improved ADCC or CDC.

**Table.**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include a complex comprising a polypeptide with an N-terminal methionyl residue. Other insertional variants include the fusion to the N- or C-terminus of the polypeptide chains of the complex to an enzyme.

### b) Glycosylation variants

In certain embodiments, a polypeptide of the complex provided herein is altered to increase or decrease the extent to which the polypeptide is glycosylated. Addition or deletion of glycosylation sites to a polypeptide may be conveniently accomplished by altering the amino acid sequence such that one or more glycosylation sites is created or removed.

Where the complex comprises an antibody Fc-region, the carbohydrate attached thereto may be altered. Native Fc-regions produced by mammalian cells typically comprise a branched, biantennary oligosaccharide that is generally attached by an N-linkage to Asn297 of the CH2 domain of the Fc-region. See, e.g., Wright, A. and Morrison, S.L., TIBTECH 15 (1997) 26-32. The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, complex comprising polypeptide variants are provided having a carbohydrate structure that lacks fucose attached (directly or indirectly) to an Fc-region. For example, the amount of fucose in such Fc-region may be from 1 % to 80 %, from 1 % to 65 %, from 5 % to 65 % or from 20 % to 40 %. The amount of fucose is determined by calculating the average amount of fucose within the sugar chain at Asn297, relative to the sum of all glycostructures attached to Asn 297 (e.g. complex, hybrid and high mannose structures) as measured by MALDI-TOF mass spectrometry, as described in WO 2008/077546, for example. Asn297 refers to the asparagine residue located at about position 297 in the Fc-region (EU numbering of Fc-region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US 2003/0157108; US 2004/0093621. Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO 2005/053742; WO 2002/031140; Okazaki, A., et al., J. Mol. Biol. 336 (2004) 1239-1249; Yamane-Ohnuki, N., et al., Biotech. Bioeng. 87 (2004) 614-622. Examples of cell lines capable of producing defucosylated antibodies include Lec13 CHO cells deficient in protein fucosylation (Ripka, J., et al., Arch. Biochem. Biophys. 249 (1986) 533-545; US 2003/0157108; and WO 2004/056312, especially at Example 11), and knockout cell lines, such as alpha-1,6-fucosyltransferase gene, FUT8, knockout CHO cells (see, e.g., Yamane-Ohnuki, N., et al., Biotech. Bioeng. 87 (2004) 614-622; Kanda, Y., et al., Biotechnol. Bioeng. 94 (2006) 680-688; and WO 2003/085107).

Complexes comprising Fc-region variants are further provided with bisected oligosaccharides, e.g., in which a biantennary oligosaccharide attached to the Fc-region is bisected by GlcNAc. Such variants may have reduced fucosylation and/or improved ADCC function. Examples of such antibody variants are described, e.g., in WO 2003/011878; US 6,602,684; and US 2005/0123546. Fc-region variants with at least one galactose residue in the oligosaccharide attached to the Fc-region are also provided. Such Fc-region variants may have improved CDC function. Corresponding antibody variants are described, e.g., in WO 97/30087; WO 98/58964; and WO 99/22764.

### c) Fc-region variants

In certain embodiments, one or more amino acid modifications may be introduced into the Fc-region of a polypeptide comprised in the complex provided herein, thereby generating an Fc-region variant. The Fc-region variant may comprise a human Fc-region sequence (e.g., a human IgG1, IgG2, IgG3 or IgG4 Fc-region) comprising an amino acid modification (e.g. a substitution) at one or more amino acid positions.

In certain embodiments, the invention contemplates an Fc-region variant that possesses some but not all effector functions, which make it a desirable candidate for applications in which the half-life of the complex in vivo is important yet certain effector functions (such as complement and ADCC) are unnecessary or deleterious. In vitro and/or in vivo cytotoxicity assays can be conducted to confirm the reduction/depletion of CDC and/or ADCC activities. For example, Fc receptor (FcR) binding assays can be conducted to ensure that the complex lacks FcγR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch, J.V. and Kinet, J.P., Annu. Rev. Immunol. 9 (1991) 457-492. Non-limiting examples of in vitro assays to assess ADCC activity of a molecule of interest is described in U.S. Patent No. 5,500,362 (see, e.g. Hellstrom, I., et al., Proc. Natl. Acad. Sci. USA 83 (1986) 7059-7063; and Hellstrom, I., et al., Proc. Natl. Acad. Sci. USA 82 (1985) 1499-1502); U.S. Patent No. 5,821,337 (see Bruggemann, M., et al., J. Exp. Med. 166 (1987) 1351-1361). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed in vivo, e.g., in an animal model such as that disclosed in Clynes, R., et al., Proc. Natl. Acad. Sci. USA 95 (1998) 652-656. C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro, H., et al., J. Immunol. Methods 202 (1996) 163-171; Cragg, M.S., et al., Blood 101 (2003) 1045-1052; and Cragg, M.S. and Glennie, M.J., Blood 103 (2004) 2738-2743). FcRn binding and in vivo clearance/half-life determinations can also be performed using methods known in the art (see, e.g., Petkova, S.B., et al., Int. Immunol. 18 (2006) 1759-1769).

Fc-regions with reduced effector function include those with substitution of one or more of Fc-region residues 234, 235, 238, 265, 269, 270, 297, 327 and 329 (see e.g. US 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US 7,332,581).

Certain Fc-region variants with improved or diminished binding to FcRs are described. (See, e.g., US 6,737,056; WO 2004/056312, and Shields, R.L., et al., J. Biol. Chem. 276 (2001) 6591-6604).

In certain embodiments, a polypeptide variant comprises an Fc-region with one or more amino acid substitutions which improve ADCC, e.g., substitutions at positions 298, 333, and/or 334 of the Fc-region (EU numbering of residues).

In some embodiments, alterations are made in the Fc-region that result in altered (i.e., either improved or diminished) C1q binding and/or Complement Dependent Cytotoxicity (CDC), e.g., as described in US 6,194,551, WO 99/51642, and Idusogie, E.E., et al., J. Immunol. 164 (2000) 4178-4184.

Antibodies with increased half-lives and improved binding to the neonatal Fc receptor (FcRn), which is responsible for the transfer of maternal IgGs to the fetus (Guyer, R.L., et al., J. Immunol. 117 (1976) 587-593, and Kim, J.K., et al., J. Immunol. 24 (1994) 2429-2434), are described in US 2005/0014934. Those antibodies comprise an Fc-region with one or more substitutions therein which improve binding of the Fc-region to FcRn. Such Fc-region variants include those with substitutions at one or more of Fc-region residues: 238, 256, 265, 272, 286, 303, 305, 307, 311, 312, 317, 340, 356, 360, 362, 376, 378, 380, 382, 413, 424 or 434, e.g., substitution of Fc-region residue 434 (US 7,371,826).

See also Duncan, A.R. and Winter, G., Nature 322 (1988) 738-740; US 5,648,260; US 5,624,821; and WO 94/29351 concerning other examples of Fc-region variants.

### d) Derivatives

In certain embodiments, a complex provided herein may be further modified to contain additional non-proteinaceous moieties that are known in the art and readily available. The moieties suitable for derivatization of the complex include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propylene glycol homopolymers, poly propylene oxide/ethylene oxide co-polymers, polyoxyethylated polyols (e.g., glycerol), polyvinyl alcohol, and mixtures thereof.

Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of a complex and non-proteinaceous moiety that may be selectively heated by exposure to radiation are provided. In one embodiment, the non-proteinaceous moiety is a carbon nanotube (Kam, N.W., et al., Proc. Natl. Acad. Sci. USA 102 (2005) 11600-11605). The radiation may be of any wavelength, and includes, but is not limited to, wavelengths that do not harm ordinary cells, but which heat the non-proteinaceous moiety to a temperature at which cells proximal to the antibody-non-proteinaceous moiety are killed.

### B. Recombinant Methods and Compositions

Complexes may be produced using recombinant methods and compositions, e.g., as described in US 4,816,567. In one embodiment, isolated nucleic acids encoding the polypeptides of the complex described herein are provided. In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp2/0 cell). In one embodiment, a method of making a complex as reported herein is provided, wherein the method comprises culturing a host cell comprising a nucleic acid encoding the polypeptides of the complex, as provided above, under conditions suitable for expression of the polypeptides and formation of the complex, and optionally recovering the complex from the host cell (or host cell culture medium).

For recombinant production of a complex, nucleic acid encoding the polypeptides of the complex, e.g., as described above, are isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression vectors include prokaryotic or eukaryotic cells described herein. For example, complexes may be produced in bacteria, in particular when glycosylation and Fc effector function are not needed. For expression of antibody fragments and polypeptides in bacteria, see, e.g., US 5,648,237, US 5,789,199, and US 5,840,523. (See also Charlton, K.A., In: Methods in Molecular Biology, Vol. 248, Lo, B.K.C. (ed.), Humana Press, Totowa, NJ (2003), pp. 245-254, describing expression of antibody fragments in *E. coli*.) After expression, the complex may be isolated from the bacterial cell paste in a soluble fraction and can be further purified.

In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of an antibody with a partially or fully human glycosylation pattern. See Gerngross, T.U., Nat. Biotech. 22 (2004) 1409-1414; and Li, H., et al., Nat. Biotech. 24 (2006) 210-215.

Suitable host cells for the expression of glycosylated complexes are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells.

Plant cell cultures can also be utilized as hosts. See, e.g., US 5,959,177, US 6,040,498, US 6,420,548, US 7,125,978, and US 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (HEK 293 or 293 cells as described, e.g., in Graham, F.L., et al., J. Gen Virol. 36 (1977) 59-74); baby hamster kidney cells (BHK); mouse sertoli cells (TM4 cells as described, e.g., in Mather, J.P., Biol. Reprod. 23 (1980) 243-252); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather, J.P., et al., Annals N.Y. Acad. Sci. 383 (1982) 44-68; MRC 5 cells; and FS4 cells. Other useful mammalian host cell lines include Chinese hamster ovary (CHO) cells, including DHFR⁻ CHO cells (Urlaub, G., et al., Proc. Natl. Acad. Sci. USA 77 (1980) 4216-4220); and myeloma cell lines such as Y0, NS0 and Sp2/0. For a review of certain mammalian host cell lines suitable for antibody production, see, e.g., Yazaki, P. and Wu, A.M., Methods in Molecular Biology, Vol. 248, Lo, B.K.C. (ed.), Humana Press, Totowa, NJ (2004), pp. 255-268.

### C. Pharmaceutical Formulations

Pharmaceutical formulations of a complex as described herein are prepared by mixing such complex having the desired degree of purity with one or more optional pharmaceutically acceptable carriers (Remington's Pharmaceutical Sciences, 16th edition, Osol, A. (ed.) (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyl dimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as poly(vinylpyrrolidone); amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rhuPH20 (HYLENEX®, Baxter International, Inc.). Certain exemplary sHASEGPs and methods of use, including rhuPH20, are described in US 2005/0260186 and US 2006/0104968. In one aspect, a sHASEGP is combined with one or more additional glycosaminoglycanases such as chondroitinases. Exemplary lyophilized antibody formulations are described in US 6,267,958. Aqueous antibody formulations include those described in US 6,171,586 and WO 2006/044908, the latter formulations including a histidine-acetate buffer.

The formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methyl methacrylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. (ed.) (1980).

Sustained-release preparations may be prepared. Suitable examples of sustained-release preparations include semi-permeable matrices of solid hydrophobic polymers containing the antibody, which matrices are in the form of shaped articles, e.g. films, or microcapsules.

The formulations to be used for in vivo administration are generally sterile. Sterility may be readily accomplished, e.g., by filtration through sterile filtration membranes.

### D. Therapeutic use and Compositions

Any of the complexes provided herein may be used in therapeutic methods.
In one aspect, a complex as reported herein for use as a medicament is provided.
In further aspects, a complex as reported herein for use in treating cancer or a viral infection is provided.

In certain embodiments, a complex as reported herein for use in a method of treatment is provided.

Disclosed herein is a complex as reported herein for use in a method of treating an individual having cancer or a viral infection comprising administering to the individual an effective amount of the complex as reported herein. In one such embodiment, the method further comprises administering to the individual an effective amount of at least one additional therapeutic agent, e.g., as described below.

Also disclosed herein is a complex as reported herein for use in removal of cancer cells or for removal of virus infected cells. In certain embodiments, the invention provides a complex as reported herein for use in a method of removal of cancer cells or removal of virus infected cells in an individual comprising administering to the individual an effective of the complex as reported herein to remove cancer cells or to remove virus infected cells. An "individual" according to any of the above embodiments may be a human.

Also disclosed herein is the use of a complex as reported herein in the manufacture or preparation of a medicament. In one embodiment, the medicament is for treatment of cancer or viral infections. In a further embodiment, the medicament is for use in a method of treating cancer or viral infections comprising administering to an individual having cancer or a chronic viral infection an effective amount of the medicament. In one such embodiment, the method further comprises administering to the individual an effective amount of at least one additional therapeutic agent. In a further embodiment, the medicament is for the removal of cancer cells or for the removal of virus infected cells. In a further embodiment, the medicament is for use in a method of removal of cancer cells or removal of virus infected cells in an individual comprising administering to the individual an amount effective of the medicament to remove cancer cells or to remove virus infected cells. An "individual" according to any of the above embodiments may be a human.

Disclosed herein is a method for treating cancer or a viral infection. In one embodiment, the method comprises administering to an individual having such cancer or viral infection an effective amount of a complex as reported herein. In one such embodiment, the method further comprises administering to the individual an effective amount of at least one additional therapeutic agent. An "individual" according to any of the above embodiments may be a human.

Disclosed herein is a method for removal of cancer cells or virus infected cells in an individual. In one embodiment, the method comprises administering to the individual an effective amount of a complex as reported herein to remove cancer cells or virus infected cells. In one embodiment, an "individual" is a human.

In a further aspect, the invention provides pharmaceutical formulations comprising any of the complexes as reported herein, e.g., for use in any of the above therapeutic methods. In one embodiment, a pharmaceutical formulation comprises any of the complexes as reported herein and a pharmaceutically acceptable carrier. In another embodiment, a pharmaceutical formulation comprises any of the complexes as reported herein and at least one additional therapeutic agent.

Complexes of the invention can be used either alone or in combination with other agents in a therapy. For instance, a complex of the invention may be co-administered with at least one additional therapeutic agent.

Such combination therapies noted above encompass combined administration (where two or more therapeutic agents are included in the same or separate formulations), and separate administration, in which case, administration of the complex of the invention can occur prior to, simultaneously, and/or following, administration of the additional therapeutic agent and/or adjuvant. Complexes of the invention can also be used in combination with radiation therapy.

A complex of the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Complexes of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The complex need not be, but is optionally formulated with one or more agents currently used to prevent or treat the disorder in question. The effective amount of such other agents depends on the amount of complex present in the formulation, the type of disorder or treatment, and other factors discussed above. These are generally used in the same dosages and with administration routes as described herein, or about from 1 to 99 % of the dosages described herein, or in any dosage and by any route that is empirically/clinically determined to be appropriate.

For the prevention or treatment of disease, the appropriate dosage of a complex of the invention (when used alone or in combination with one or more other additional therapeutic agents) will depend on the type of disease to be treated, the type of complex, the severity and course of the disease, whether the complex is administered for preventive or therapeutic purposes, previous therapy, the patient's clinical history and response to the complex, and the discretion of the attending physician. The complex is suitably administered to the patient at one time or over a series of treatments. Depending on the type and severity of the disease, about 1 µg/kg to 15 mg/kg (e.g. 0.5 mg/kg - 10 mg/kg) of complex can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (*e.g*. such that the patient receives from about two to about twenty, or e.g. about six doses of the antibody). An initial higher loading dose, followed by one or more lower doses may be administered. However, other dosage regimens may be useful. The progress of this therapy is easily monitored by conventional techniques and assays.

It is understood that any of the above formulations or therapeutic methods may be carried out using an immunoconjugate of the invention in place of or in addition to a complex as reported herein.

One aspect as reported herein is the complex as reported herein for use in a method of treating a cancer or a viral infection in a patient, wherein the complex is to be administered before, simultaneously or after the immunization of the patient with the virus-derived peptide comprised in the complex.

Disclosed herein is the use of a complex as reported herein for the manufacture of a medicament for the treatment of cancer or a viral infection in combination with immunization against the virus-derived peptide comprised in the complex.

In the first step the virus-derived peptide as contained in the complex is administered first to the individual to be treated. At a certain time span later, i.e. between 4 days and 28 days, the complex as reported herein is administered to the individual.

By this successive and separated application of the virus-derived polypeptide, in the first step alone and in the second step in the complex as reported herein, it is possible to increase the number of virus-derived peptide specific T-cell and, thus, to increase the efficacy of the treatment.

### III. Articles of Manufacture

In another aspect of the disclosure, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a complex of the invention. The label or package insert indicates that the composition is used for treating the condition of choice. Moreover, the article of manufacture may comprise (a) a first container with a composition contained therein, wherein the composition comprises a complex of the invention; and (b) a second container with a composition contained therein, wherein the composition comprises a further cytotoxic or otherwise therapeutic agent. The article of manufacture in this embodiment of the disclosure may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

It is understood that any of the above articles of manufacture may include an immunoconjugate of the invention in place of or in addition to a complex as reported herein.

### IV. EXAMPLES

The following are examples of methods and compositions of the invention. It is understood that various other embodiments may be practiced, given the general description provided above.

### Example 1

### Procedure for isolation and stimulation of CMV-specific CD8 positive T-cells from human donors

### Isolation of PBLs

PBL were isolated by Ficoll gradient centrifugation from human donor blood (Greiner bio-one, Cat. No. 227290). PBLs were cultured in RPMI supplemented with 5 % human serum (Sigma Cat. No. H2520), 2 mM L-glutamine (PAN Biotech, Cat. No. P04-80100), 100 µg/ml Penicillin/Streptomycin (Roche, Cat. No. 14001100).

### Stimulation of PBLs

Cells (2 x 10⁷ cells/ml) were cultured in cell culture medium supplemented with 50 µg/ml CMV pp65-derived peptide (SEQ ID NO: 01) for two hours under cell culture conditions (37 °C, 5 % CO₂, 80 % humidity). Thereafter the cell suspension was 20-fold diluted with culture medium and further cultured in flat-bottom 96-well plates at a seeding density of 2 x 10⁵ cells per 96 well. After 4 to 5 days, 20 U/ml IL-2 (Roche, Cat. No. 11011456001), 25 ng/ml IL-7 (Peprotech, Cat. No. 200-01) and 25 ng/ml IL-15 (Peprotech, Cat. No. 200-15) were added and the cells were cultured for another 7 to 8 days. Stimulation of T-cells is visible under the microscope as cell clusters.

### Re-Stimulation of PBLs

T-cells were co-cultured with stimulator cells, which are peptide-pulsed autologous primary PBLs of the same donor (either freshly prepared or derived from frozen stocks). The stimulator cells were pulsed with the peptide as described above. After the two hours of peptide incubation the PBLs were irradiated (4000 rad; STS GmbH OB29 Nr.9510-5) and washed twice in culture medium without peptide. The re-stimulation was carried out in 96 well plates round bottom plates. 8 x 10⁴ to 1 x 10⁵ stimulator cells were used per 96 well. Cells from the primary culture were washed twice with culture medium, resuspended in 200 µl culture medium and 80 µl were transferred to each well of the stimulator cells. After 3 days 20 U/ml IL-2, 25 ng/ml IL-7 and 25 ng/ml IL-15 were added. Cells did proliferate and were expanded every 2 to 3 days in new wells with fresh medium.

### Analysis of T-cells

Cells were stained for CD8 expression (BD, Cat. No. 345772) and CMV-specific T-cell receptors (ProImmune, Cat. No. F008-4A-E) and analyzed in FACS.

### Cell culture medium

RPMI1640 (PAN Biotech, Cat. No. P04-17500), 5 % Human Serum (HS; Sigma Cat. No. H2520), 2 mM L-glutamine (PAN Biotech, Cat. No. P04-80100), 100 µg/ml Penicillin/Streptomycin (Roche, Cat. No. 14001100).

### Results

FACS analysis of four human donor derived peripheral blood lymphocytes (PBLs) was performed. The cells were labeled with a FITC-conjugated anti-CD8 antibody (BD, Cat. No. 345772) combined with APC-conjugated Pro5 pentamer (ProImmune, Cat. No. F008-4A-E) to stain T-cells which carry a T-cell receptor (TCR) recognizing MHC-class I (HLA-A*0201) loaded with CMV-derived peptide (NLVPMVATV (SEQ ID NO: 01)). For results see Figure 4. At day 0 donor 1 and 4 carry low numbers of CMV-specific CD8 T-cells (0.08 % and 0.1 %, respectively). Donor 2 and 3 carry a higher number of CMV-specific CD8 T cells in their peripheral blood (0.25 % and 3.12 %, respectively). Fourteen days later after stimulation with CMV-derived peptide pulsed autologous cells only donors 2 and 3 show a significant increase in CMV-specific CD8 T cells (6.2 % and 71.2 %, respectively) whereas donors 1 and 4 do not show increased numbers of CMV-specific CD8 T cells (0.01 % and 0.05 %, respectively). Another 14 days later after a second stimulation with CMV-derived peptide pulsed autologous cells donors 2 and 3 show a further increase in CMV-specific CD8 T cells (15.1 % and 96.6 %, respectively).

### Example 2

### Cytotoxicity Assay

Acute lymphoblastic leukemia cells MN60 carry the A*0201 HLA-A allele. MN60 cells (1 x 10⁶ cells/ml) were incubated with 50 µg/ml CMV pp65 peptide (SEQ ID NO: 01) for 45 minutes under cell culture conditions (37 °C, 5 % CO₂, 80 % humidity). The incubation results in a peptide exchange in the HLA-A*0201 peptide binding groove. The peptide exchanged MN60 cells were centrifuged and diluted to a density of 1 x 10⁶ cells/ml with PBS (PanBiotech, Cat. No. P04-36500) and stained with 1 µM of the cell tracer carboxyfluorescein succinimidyl ester (CFSE, Invitrogen, Cat. No. 34554) 15 minutes at room temperature (RT). Cells were washed thereafter once with PBS and diluted to 1 x 10⁵ cells/ml with AIM-V media (Gibco, Cat. No. 0870112DK). For the assay MN60 cells (target cells) were co-cultured in 96-well round bottom plates with CMV-specific human donor 3 derived CD8⁺ T-cells (effector cells, see example 1) for four hours under cell culture conditions. The effector to target cell ratio of was 4:1. Dead cells are stained with 1 µl/100 µl propidium iodide (PI, Sigma, Cat. No. P-4864) and were FACS analyzed.

### Results

Flow Cytometric Analysis was performed to analyze the cytolytic capability of stimulated CTLs through lysis of MN60 tumor cells loaded with CMV peptide:

In Figure 5a the FACS analysis of a co-culture of MN60 cells not loaded with the CMV-derived peptide is shown. MN60 cells are FITC-positive. Effector cells are FITC-negative. Dead cells are PI positive, alive cells are PI-negative. It can be seen that more than 85 % of the MN60 cells are alive when they are not loaded with the CMV-derived peptide (Q2 and Q4).

In Figure 5b the FACS analysis of MN60 cells loaded with CMV-derived peptide is shown. It can be seen that more than 80 % of the MN60 cells are dead (Q2 and Q4) whereas the ratio of alive and dead effector cells is not remarkably altered between the FACS analysis shown in Figure 5a and Figure 5b (compare Q1 and Q3 in Figure 5a and Figure 5b) indicating a specific lysis of CMV-peptide-loaded target cells.

Flow Cytometric Analysis to analyze the cytolytic capability of stimulated CTLs through lysis of MN60 tumor cells loaded with CMV peptide depending on the effector to target cell ratio:

The cytotoxic assay was performed as described above. Different effector cell to target cell ratios were applied ranging from 0.5 effector cells per target cell to four effector cells per target cell (see Figure 6). Incubation time was four hours. MN60 cells which were not loaded with the CMV-derived peptide do not show an increased number of dead cells with an increased effector to target ratio, i.e. ranging from 8 % to 13 % with ratio 0.5:1 to 4:1.

Almost 20 % of the MN60 cells loaded with CMV-derived peptide are already killed with a low effector to target ratio of 0.5:1 within four hours. The number of dead cells increases steeply with an increase in effector to target ratio reaching up to 83 % at a ratio of 4:1 effector cells per target cell.

### Example 3

### DNA preparation, transfection, expression, purification and analysis

### DNA preparation

250 ml of overnight bacterial LB culture were harvested and plasmid DNA was extracted according to the manufacturer's protocol (High speed Maxi kit, Qiagen, Cat. No. 12663). The resulting plasmid DNA was eluted in 1 ml TE buffer and DNA concentration was determined by spectrophotometric measurement (Epoch, BioTek).

The final expression vector comprised the following elements:
- the endonucleolytic restriction sites HindIII, NheI,
- a CMV-promoter,
- an 5'UTR 1 (derived from the human CMV),
- Intron A,
- a 5'UTR 2,
- an ampicillin-resistance gene,
- a BGH poly A site (bovine growth hormone polyadenylation signal),
- pUC Ori.

Amino acid sequences of the elements of the complex

| | |
|---|---|
| CMV pp65 Peptide: | SEQ ID NO: 01 |
| | NLVPMVATV |
| | |
| Linker 1: | SEQ ID NO: 21 |
| | GGGGSGGGGSGGGGS |
| | |
| β2-microglobulin: | SEQ ID NO: 10 |
| | |
| | |
| Linker 2: | SEQ ID NO: 22 |
| | GGGGSGGGGSGGGGSGGGGS |
| | |
| HLA-A*0201 α1 - α3: | SEQ ID NO: 11 |
| | |
| | |
| Linker 3: | SEQ ID NO: 12 |
| | GS |
| | |
| Linker 4: | SEQ ID NO: 22 |
| | GGGGSGGGGSGGGGSGGGGS |
| | |
| Ig light chain: | SEQ ID NO: 41 |
| | |
| Ig heavy chain (IgG1-L234A, L235A mutant): SEQ ID NO: 42 | |
| | |
| | |
| Ig heavy chain (IgG1-L234A, L235A mutant with knob variation): SEQ ID NO: 43 | |
| | |
| | |
| Ig heavy chain (IgG1-L234A, L235A mutant with hole variation): SEQ ID NO: 44 | |
| | |
| | |
| | |
| Ig heavy chain Fc-region (IgG1-L234A, L235A mutant Fc-region knob variant): | |
| | SEQ ID NO: 45 |
| | |
| | |
| scFv: | SEQ ID NO: 46 |
| | |

### Transfection

HEK 293 cells were diluted to 8 x 10⁵ cells/ml the day before transfection. About 1 to 1.6 x 10⁶ cells/ml were transfected according to the manufacturer's protocol. For a final transfection volume of 30 ml, 30 µg DNA were diluted to a final volume of 1 ml with Opti-MEM® I Reduced Serum Medium (Gibco, Cat. No. 31985070). 2 µl of 293fectin™Reagent (Invitrogen, Cat. No. 12347019) per 1 µg DNA were equally diluted to a final volume of 1 ml with Opti-MEM® medium and incubated for 5 minutes. After incubation the diluted DNA was added to the diluted 293fectin™Reagent, gently mixed, incubated for another 20-30 minutes and afterwards drop wise pipetted to 28 ml of the HEK 293 cells to obtain a final volume of 30 ml. The cells were incubated under cell culture condition (37 °C, 8 % CO₂, 80 % humidity) on an orbital shaker rotating at 125 rpm and harvested after 72 hours. The harvest was centrifuged for 10 minutes at 1000 rpm, for 10 minutes at 3000 rpm and filtered through a 22 µm sterile filter (Millipore, Cat. No. SCGPU05RE).

### Western Blotting

500 µl of cell culture supernatant was concentrated with Pall Nanosep Omega-Membran 30KD Centrifugal Devices (Pall, Cat. No. OD030C33) to a volume of 50 µl. 17.5 µl of each concentrate was diluted to a final volume of 25 µl with 4x XT Sample Buffer (Bio Rad, Cat. No. 161-0791) and 20x XT Reducing Agent (BioRad, Cat. No. 161-0792), heated for 8 minutes at 96 °C and applied on a 4-12 % Criterion XT Precast Gel (Cat. No. 345-0124). Blotting was performed with Trans-Blot SD semi-dry Transfer Cell (BioRad) at 20 V for 30 minutes on a Trans-blot Pure Nitrocellulose membrane (0.45 µm) (BioRad, Cat. No. 162-0117). Blocking of the membrane was performed with 1x Western Blocking Reagent (Roche, Cat. No. 11921681001) for one hour at room temperature. Staining was performed with peroxidase conjugated polyclonal rabbit anti-human κ-light chain (DAKO, Cat. No. P0129, diluted 1:3000) and polyclonal rabbit anti-human IgG antibody HRP conjugate (DAKO, Cat. No. P0214, diluted 1:5000) for one hour at room temperature. Luminescence detection was carried out with LUMI-Imager F1 (Roche).

### Purification

Cells were removed from culture medium by centrifugation. Complexes were purified from supernatants by Protein A affinity chromatography (MabSelect-Sepharose on an ÄKTA-Avant). Eluted complexes were concentrated with Amicon centrifugation tubes to a protein concentration of 3 mg/ml. An aliquot was analyzed on a size exclusion chromatography (HPLC TSKgel GFC300 Sys89). Preparative SEC on a Superdex 200 was performed to remove aggregates and buffer the fusion proteins in 20 mM histidine, 140 mM NaCl, pH 6.0. Eluted complexes were concentrated with Amicon centrifugation tube to a protein concentration of 1 mg/ml and sterile filtered (0.2 µm pore size).

### Analytics

Complex samples were analyzed by OD280 using a UV spectrophotometer to determine the protein concentration in solution. The extinction coefficient required for this was calculated from the amino acid sequence according to Pace (Pace, et al., Protein Science 4 (1995) 2411-2423). Size-exclusion chromatography (SE-HPLC) was performed on TSK-Gel300SWXL or Superdex 200 columns with a 0.2 M potassium phosphate buffer, comprising 0.25 M KCl, pH 7.0 as mobile phase in order to determine the content of monomeric, aggregated and degraded species in the samples. Sodium dodecyl sulfate (SDS) polyacrylamide gel electrophoresis (reducing and non-reducing) was performed to analyze the purity of the complex preparations with regard to product-related degradation products and unrelated impurities. Electrospray ionization mass spectrometry (ESI-MS) was performed with reduced (TCEP) and deglycosylated (N-glycosidase F) samples to confirm the correct mass/identity of each chain and detect chemical modifications. ESI-MS of the deglycosylated samples was carried out to analyze the nature and quality of the fully assembled protein and detect potential product-related side products.

**Method SDS-PAGE and Coomassie Staining**

| | |
|---|---|
| Device: | Invitrogen XCell Sure Lock Mini-Cell |
| Gel: | 4-20% Tris-Glycine Gel, Invitrogen EC6025BOX |
| Buffer: | Tris-Glycine SDS Running Buffer (10x), Invitrogen LC2675-5 |
| Sample buffer: | Tris-Glycine SDS Sample Buffer (2x), Invitrogen LC2676 |
| Reducing buffer: | NuPAGE Sample Reducing Agent (10x), Invitrogen NP0004 |
| Molecular Weight Marker: | Mark 12, MW Standard, Invitrogen LC5677 |

### Protein Sample preparation

The sample was adjusted to a protein concentration of 1 mg/ml with buffer. For sample reduction the following procedure was carried out:
- Reduction buffer: 4 ml Sample buffer (2x) and 1 ml reducing buffer (10x)
- dilute sample 1:1 with reduction buffer
- incubate for 5 minutes at 70 °C

The gel electrophoresis was carried out at 125 V for 90 minutes. The gels were stained with Simply Blue Safe Stain (Invitrogen, Cat. No. LC6065).

### Results

**Table.**

| **No.** | **polypeptides comprised in the complex** | **scheme** | **yield** |
|---|---|---|---|
| 1 | 1. [CMV-pp65-peptide]-[linker 1]-[β2-microglobulin]-[linker 2]-[HLA-A-α1-α2-α3]-[linker 3]-[IgG1-L234A, L235A mutant with hole variation] | 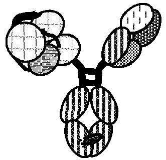 | 5 mg/l |
| | 2. Ig heavy chain (IgG1-L234A, L235A mutant with knob variation) | | |
| | 3. Ig light chain | | |
| 2 | A: | 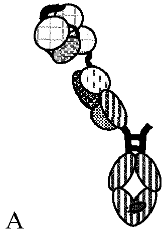 | A: 5 -18 mg/l |
| | 1. [CMV-pp65-peptide]-[linker 1]-[β2-microglobulin]-[linker 2]-[HLA-A-α1-α2-α3]-[linker 3]-[IgG1-L234A, L235A mutant with hole variation] | | |
| | 2. IgG1-L234A, L235A mutant Fc-region knob variant | 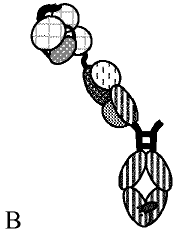 | |
| | 3. Ig light chain | | |
| | B: Fusion to the Ig light chain | | |
| 3 | A: | 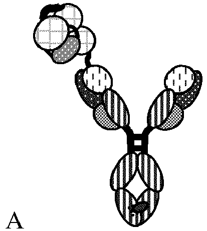 | A: 4 - 23 mg/l |
| | 1. [CMV-pp65-peptide]-[linker 1]-[β2-microglobulin]-[linker 2]-[HLA-A-α1-α2-α3]-[linker 3]- [IgG1-L234A, L235A mutant with hole variation] | | |
| | 2. IgG1-L234A, L235A mutant with knob variation | 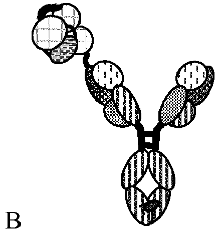 | |
| | 3. Ig light chain | | |
| | B: Fusion to the Ig light chain | | |
| 4 | 1. [CMV-pp65-Peptide]-[Linker 1]-[β2-microglobulin]-[Linker 2]-[HLA-A-α1-α2-α3]-[Linker 3]-[IgG1-L234A, L235A mutant with hole variation] | 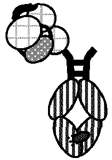 | 4 mg/l |
| | 2. IgG1-L234A, L235A mutant with knob variation | | |
| 5 | 1. [CMV-pp65-peptide]-[linker 1]-[β2-microglobulin]-[linker 2]-[HLA-A-α1-α2-α3]-[linker 3]-[IgG1-L234A, L235 A-Fc-region] | 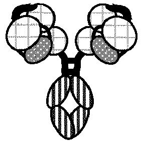 | 4 mg/l |
| 6 | 1. [CMV-pp65-peptide]-[linker 1]-[β2-microglobulin]-[linker 2]-[HLA-A-α1-α2-α3]-[linker 3]-[IgG1-L234A, L235A mutant Fc-region]-[linker 4]-[scFv] | 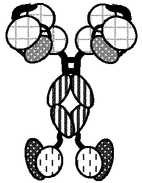 | < 1 µg/l |
| 7 | A: 1. [CMV-pp65-peptide]-[linker 1]-[β2-microglobulin]-[linker 2]-[HLA-A-α1-α2-α3]-[linker 3]-[IgG1-L234A, L235A mutant] | 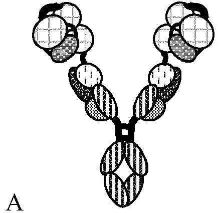 | < 1 µg/l |
| | 2. Ig light chain | 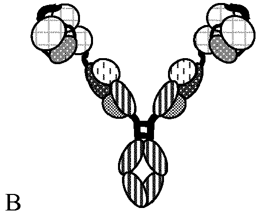 | |
| | B: Fusion to the Ig light chain | | |

The SDS gel with Coomassie staining and the corresponding SEC chromatograms of selected complexes number 1 and 2a according to the previous table are shown in Figure 7 and 8. It can be seen that defined complexes can be obtained.

### Example 4

### Binding of MHC-Antibody-Fusion to human IGF-1R positive Cell Line

H460M2 cells were diluted to 8 x 10⁵ cells/ml in AIM-V medium (Gibco, Cat. No. 0870112DK). 500 µl of the cell suspension was stained with 10 µg of a complex as reported herein either at 4 °C or 37 °C for one hour. Thereafter cells were washed once with ice-cold AIM-V medium and stained with a second antibody, which was a goat F(ab')₂ anti-human IgG (H+L) antibody conjugated to R-PE (Dianova, Cat. No. 109-116-088, dilution 1:50) for 30 minutes at 4 °C. Thereafter cells were washed once with ice-cold AIM-V medium and fluorescence was measured via FACS Canto II (BD Bioscience) with gating on living cells. A bispecific antibody served as Isotype control, an anti IGF-1R antibody (see e.g. WO 2004/087756 and WO 2007/115814) served as positive control.

### Results

The results are shown in Figure 9. Considering the shift in the PE-fluorescence measurement (X-axis), the complex as reported herein shows no visible difference in binding to H460M2 target cells (Figure 9-2) in comparison to the control Antibody (Figure 9-6). There is also no difference whether the incubation with the complex as reported herein is accomplished at 4 °C or 37 °C (see Figure 9-2 vs. Figure 9-3). Neither the incubation with the isotype control (Figure 9-4) nor with the fluorescence labeled secondary antibody alone (Figure 9-5) shows any shift in the PE fluorescence measurement. Despite the fusion of the class I MHC molecule the antibody variable domain of the complex as reported herein still binds to the H460M2 target cells.

Although the foregoing invention has been described in some detail by way of illustration and example for purposes of clarity of understanding, the descriptions and examples should not be construed as limiting the scope of the invention.

### Example 5

### In vitro removal of antigen expressing cells

I24 target cells (1x10⁵ cells/ml) were seeded in cell culture media (RPMI 1640 supplemented with 2 mM L-glutamine, 1 mM sodium pyruvate, 0.1 mM NEAA, and 10 % (v/w) FCS) on WillCo Glass Bottom Dishes (FA. WillCo Wells BV, REF GWST-3522) for 24 to 48 hours. WillCo Glass Bottom Dishes were precoated with 50 µg/ml poly-L-lysine hydrochloride (Sigma Aldrich, Cat # P2658) per dish for 30 min. After coating the dishes were thoroughly rinsed with sterile tissue culture grade water and dried for two hours.

After the cultivation cell culture media was removed and the antigen binding complex comprising one [CMV-pp65-peptide]-[linker 1]-[β2-microglobulin]-[linker 2]-[HLA-A-α1-α2-α3]-[linker 3]-[IgG1-L234A, L235A mutant with hole variation] fusion polypeptide, one IgG1-L234A, L235A mutant Fc-region knob variant disulfide-linked polypeptide and one Ig light chain, wherein the complex specifically binds to human IGF-1R as reported herein (see e.g. Example 3) was added in a final concentration of 5 µg/ml in 3 mM K⁺ Krebs Ringer HEPES Buffer pH 7.3 (supplemented with 0.5 mM DL-dithiothreitol, 1 mM ascorbic acid, and 4 mM glutathione).

T-cells were added in a target cell to effector cell ration of 1:10. Imaging was performed for 4 hours with a Zeiss Axiovert 135 microscope.

### Results

In Figure 10 microscope imaging of the incubation of the antigen binding complex comprising one [CMV-pp65-peptide]-[linker 1]-[β2-microglobulin]-[linker 2]-[HLA-A-α1-α2-α3]-[linker 3]-[IgG1-L234A, L235A mutant with hole variation] fusion polypeptide, one IgG1-L234A, L235A mutant Fc-region knob variant disulfide-linked polypeptide and one Ig light chain, wherein the complex specifically binds to human IGF-1R are shown. It can be seen that the complex mediated lysis of human IGF-1R expressing I24 3T3 cells (large adherently growing cells, white arrowhead). Lysis is mediated by human CMV-specific T-cells (small cells either round shaped, white arrow, or amoeboid migrating cells, black arrow). I24 cells are incubated with the complex at a concentration of 5 µg/ml and human CMV-specific T-cells (pre-activated with HLA-A0201⁺/CMV peptide pulsed APCs). Time lapse is indicated below the respective picture. Note the interaction of the I24 cells with the T-cells at 56 min and 76 min and subsequently the collapse of the I24 cell after 125 min.

In Figure 11 microscope imaging of a control showing the absence of lysis of I24 3T3 cells (large adherently growing cells, white arrowhead) through human CMV-specific T-cells (small cells either round shaped, white arrow or amoeboid migrating cells, black arrow) in the absence of an antigen binding complex as reported herein. I24 cells are incubated with specific cytotoxic T-cells (pre-activated with HLA-A0201⁺/CMV peptide pulsed APCs). Time lapse is indicated below the respective picture.

### Example 6

### Cytotoxicity assay

Cell culture medium (50 µl) was pipetted into each well of an Xcelligence 96well E-plate (Roche, Cat # 05232368001) to perform background measurement.

I24 cells were diluted to 1x10⁶ cells/ml in cell culture media (RPMI 1640, 2 mM L-glutamine, 1 mM Sodium pyruvate, 0.1 mM NEAA, 10 % (v/w) FCS) and 50 µl (2x10⁴ cells/well) were pipetted in each well of an Xcelligence 96well plate to a final volume of 100 µl and cultivated for 24 hours (37 °C, 8 % CO₂, 80 % humidity). After 24 hours the medium was removed and the cells were washed with 200 µl AIM-V (Serum Free Medium (Invitrogen) T-cell medium (Cat-No): 12055-083) medium. The antigen binding complex comprising one [CMV-pp65-peptide]-[linker 1]-[β2-microglobulin]-[linker 2]-[HLA-A-α1-α2-α3]-[linker 3]-[IgG1-L234A, L235A mutant with hole variation] fusion polypeptide, one IgG1-L234A, L235A mutant Fc-region knob variant disulfide-linked polypeptide and one Ig light chain, wherein the complex specifically binds to human IGF-1R, as reported herein was added to the washed target cells in a final concentration of 1 µg/ml in AIM-V medium. Effector cells in the respectable ratio were added in AIM-V media to a final volume of 150 µl. Afucosylated IgG1 monoclonal antibody directed against human IGF-1R (anti-IGF-1R antibody-afucosylated) and non-binding human anti-digoxigenin antibody (anti-digoxygenin antibody) served as Isotype control and specific antibody control, respectively. Measurement was performed for 6 to 9 hours respectively with the Xcelligence System (Roche).

### Results

The complex as reported herein triggers lysis of H460M2 tumor cells through human CMV-specific T-cells.

Tumor cells were incubated for 4 hours with 1 µg/ml of the complex comprising one [CMV-pp65-peptide]-[linker 1]-[β2-microglobulin]-[linker 2]-[HLA-A-α1-α2-α3]-[linker 3]-[IgG1-L234A, L235A mutant with hole variation] fusion polypeptide, one IgG1-L234A, L235A mutant Fc-region knob variant disulfide-linked polypeptide and one Ig light chain, wherein the complex specifically binds to human IGF-1R, and specific T-cells in the respective ratio (1:1.5 to 1:0.5) (see Figure 12). Percentage of lysis is denoted above the respective bars. Afucosylated IgG1 monoclonal antibody directed against human IGF-1R (MAB IGF-1R-afu) did not trigger a significant tumor cell lysis.

The complex as reported herein triggers lysis of I24 3T3 target cells through human CMV-specific T-cells.

Target cells were incubated for 4 hours with 1 µg/ml of an antigen binding complex comprising one [CMV-pp65-peptide]-[linker 1]-[β2-microglobulin]-[linker 2]-[HLA-A-α1-α2-α3]-[linker 3]-[IgG1-L234A, L235A mutant with hole variation] fusion polypeptide, one IgG1-L234A, L235A mutant Fc-region knob variant disulfide-linked polypeptide and one Ig light chain, wherein the complex specifically binds to human IGF-1R, and specific T-cells in the respective ratio (1:1.5 to 1:0.5) (see Figure 13). Percentage of lysis is denoted above the respective bars. Afucosylated IgG1 monoclonal antibody directed against human IGF-1R (anti-IGF-1R antibody-afucosylated) and non-binding human anti-Digoxigenin antibody (anti-digoxygenin antibody) did not trigger a significant target cell lysis.

### Example 7

### In vitro efficacy

IGF-1R positive lung adenocarcinoma cell line H460M2 was incubated with 1 µg/ml of a complex comprising an hCMV-derived peptide and an anti-IGF-1R antibody and human CMV-specific CD8-positive T-cells at a low effector cell to target cell ratio (1.5 to 0.5 specific T-cells per tumor cell). Control antibody was a glyco-engineered anti-IGF-1R antibody. The incubation time was 6 hours. The incubation with complex results in a potent removal of H460M2 tumor cells.

## Claims

1. A method for the recombinant production of a complex comprising
i) a fusion polypeptide comprising in N- to C-terminal direction a T-cell response eliciting peptide, β2-microglobulin and the extracellular domains α1, α2, and α3 of a class I MHC molecule with a relative frequency of 1 % or more,
ii) a pair of disulfide-linked polypeptide chains derived from an antibody hinge region, and wherein each disulfide-linked polypeptide chain comprises a CH2 domain and a CH3 domain of human origin, wherein one CH3 domain comprises the mutation T366W and the other CH3 domain comprises the mutations T366S, L368A and Y407V (numberings according to EU index of Kabat); and
iii) at least one pair of an antibody light chain variable domain and an antibody heavy chain variable domain in a eukaryotic cell,
comprising the following steps:
- cultivating a eukaryotic cell comprising one or more nucleic acids encoding the complex, and
- recovering the complex from the cell or the cultivation medium,
wherein the complex comprises exactly one fusion polypeptide of a T-cell response eliciting peptide, β2-microglobulin and the extracellular domains α1, α2, and α3 of a class I MHC molecule, and
wherein the fusion polypeptide is
- covalently bound either to the C-terminus or the N-terminus of one of the disulfide-linked polypeptide chains, or
- covalently bound to the N-terminus of an antibody variable domain that is the complementary heavy or light chain variable domain to that comprised in the first disulfide-linked polypeptide chain, or
- covalently bound to the C-terminus of an antibody constant domain that is the complementary heavy or light chain constant domain to that comprised in the first disulfide-linked polypeptide chain.

2. The method according to claim 1, **characterized in that** the complex is obtained with a concentration of 1 mg/ml or more in the cultivation medium.

3. The method according to any one of the preceding claims, **characterized in that** the eukaryotic cell is a mammalian cell.

4. The method according to claim 3, **characterized in that** the mammalian cell is a human embryonic kidney cell, or a chinese hamster ovary cell, or a baby hamster kidney cell, or a mouse myeloma cell.

5. The method according to any one of the preceding claims, **characterized in that** the disulfide-linked polypeptide chains derived from an antibody hinge region are i) linked by one or more disulfide bonds, ii) the first disulfide-linked polypeptide chain comprises in N- to C-terminal direction an immunoglobulin light or heavy chain variable domain, an immunoglobulin light or heavy chain constant domain, and an antibody heavy chain hinge region polypeptide, and the second disulfide-linked polypeptide chain comprises an antibody heavy chain hinge region polypeptide.

6. The method according to any one of the preceding claims, **characterized in that** the T-cell response eliciting peptide is a virus-derived peptide.

7. The method according to any one of the preceding claims, **characterized in that** the fusion polypeptide comprises in N- to C-terminal direction
(i) a virus-derived peptide that has an amino acid sequence selected from SEQ ID NO: 01 to SEQ ID NO: 09,
(ii) a first linker peptide that has an amino acid sequence selected from SEQ ID NO: 16, 17, 18, 21, 22, and 23,
(iii) a β2-microglobulin that has an amino acid sequence of SEQ ID NO: 10,
(iv) a second linker peptide that has an amino acid sequence selected from SEQ ID NO: 16, 17, 18, 21, 22, and 23,
(v) the extracellular domains α1, α2, and α3 of a class I MHC molecule that has an amino acid sequence of SEQ ID NO: 11, and
(vi) a third linker peptide that has an amino acid sequence selected from SEQ ID NO: 12, 16, 17, 18, 21, 22, and 23.

8. The method according to any one of the preceding claims, **characterized in that** the first disulfide-linked polypeptide chain and the second disulfide-linked polypeptide chain comprise i) a human IgG1 CH2 domain comprising an amino acid sequence selected from SEQ ID NO: 31, 32, and 33, and a human IgG1 CH3 domain comprising an amino acid sequence selected from SEQ ID NO: 34, 35, and 36.

9. The method according to any one of claims 7 to 8, **characterized in that** the complex comprises i) a first linker peptide that has the amino acid sequence of SEQ ID NO: 21, and/or ii) a second linker peptide that has the amino acid sequence of SEQ ID NO: 22, and/or iii) a third linker peptide that has the amino acid sequence of SEQ ID NO: 12, and/or iv) a human IgG1 CH2 domain that has the amino acid sequence of SEQ ID NO: 32 or 33, and/or v) in the first disulfide-linked polypeptide a human IgG1 CH3 domain that has the amino acid sequence of SEQ ID NO: 35 and in the second disulfide-linked polypeptide a human IgG1 CH3 domain that has the amino acid sequence of SEQ ID NO: 36.

10. A complex, **characterized in that** it comprises
- one fusion polypeptide that comprises in N- to C-terminal direction
(i) a T-cell response eliciting peptide,
(ii) a β2-microglobulin, and
(iii) the extracellular domains α1, α2, and α3 of a class I MHC molecule with a relative frequency of 1 % or more,
and
- two polypeptide chains, which are linked by one or more disulfide bonds,
wherein the first disulfide-linked polypeptide chain comprises in N- to C-terminal direction
(i) an immunoglobulin light or heavy chain variable domain,
(ii) an immunoglobulin light or heavy chain constant domain, and
(iii) an antibody heavy chain hinge region polypeptide,
and
wherein the second disulfide-linked polypeptide chain comprises an antibody heavy chain hinge region polypeptide, wherein the first disulfide-linked polypeptide chain and the second disulfide-linked polypeptide chain both comprise a CH2 domain and a CH3 domain of human origin, wherein one CH3 domain comprises the mutation T366W and the other CH3 domain comprises the mutations T366S, L368A and Y407V (numberings according to EU index of Kabat);
wherein the fusion polypeptide is
- covalently bound either to the C-terminus or the N-terminus of one of the disulfide-linked polypeptide chains, or
- covalently bound to the N-terminus of an antibody variable domain that is the complementary heavy or light chain variable domain to that comprised in the first disulfide-linked polypeptide chain, or
- covalently bound to the C-terminus of an antibody constant domain that is the complementary heavy or light chain constant domain to that comprised in the first disulfide-linked polypeptide chain
with the proviso that the complex comprises exactly one fusion polypeptide.

11. The complex of claim 10, **characterized in that** the T-cell response eliciting peptide is a virus-derived peptide.

12. The complex of any one of claims 10 or 11, **characterized in that** the fusion polypeptide comprises in N- to C-terminal direction
(i) a virus-derived peptide that has an amino acid sequence selected from SEQ ID NO: 01 to SEQ ID NO: 09,
(ii) a first linker peptide that has an amino acid sequence selected from SEQ ID NO: 16, 17, 18, 21, 22, and 23.
(iii) a β2-microglobulin that has an amino acid sequence of SEQ ID NO: 10,
(iv) a second linker peptide that has an amino acid sequence selected from SEQ ID NO: 16, 17, 18, 21, 22, and 23.
(v) the extracellular domains α1, α2, and α3 of a class I MHC molecule that has an amino acid sequence of SEQ ID NO: 11, and
(vi) a third linker peptide that has an amino acid sequence selected from SEQ ID NO: 12, 16, 17, 18, 21, 22, and 23.

13. The complex according to any one of claims 10 to 12, **characterized in that** the first disulfide-linked polypeptide and the second disulfide-linked polypeptide further comprise
- a human IgG1 CH2 domain comprising an amino acid sequence selected from SEQ ID NO: 31, 32, and 33, and
- a human IgG1 CH3 domain comprising an amino acid sequence selected from SEQ ID NO: 34, 35, and 36.

14. The complex according to any one of claims 10 to 12, **characterized in that**
- the first linker peptide has the amino acid sequence of SEQ ID NO: 21, and/or
- the second linker peptide has the amino acid sequence of SEQ ID NO: 22, and/or
- the third linker peptide has the amino acid sequence of SEQ ID NO: 12, and/or
- the human IgG1 CH2 domain has the amino acid sequence of SEQ ID NO: 32 or 33, and/or
- the human IgG1 CH3 domain of one disulfide-linked polypeptide has the amino acid sequence of SEQ ID NO: 35 and the human IgG1 CH3 domain of the other disulfide-linked polypeptide has the amino acid sequence of SEQ ID NO: 36.

15. A nucleic acid encoding the complex of any one of claims 10 to 14.

16. A host cell comprising the nucleic acid of claim 15

17. A pharmaceutical formulation comprising the complex according to any one of claims 10 to 14 and optionally a pharmaceutically acceptable carrier.

18. The complex according to any one of claims 10 to 14 for use as a medicament.

19. The complex according to any one of claims 10 to 14 for use in the treatment of cancer or of a chronic viral infection.

20. The complex according to any one of claims 10 to 14 for use in therapy by attracting virus-specific cytotoxic T-cells of an individual to a target.

21. The complex according to any one of claims 10 to 14 for use in therapy by removal of cancer cells or of virus infected cells.

## Patentansprüche

1. Verfahren zur rekombinanten Herstellung eines Komplexes, umfassend:
i) ein Fusionspolypeptid, umfassend in Richtung vom N- zum C-Terminus ein T-Zell-Antwort auslösendes Peptid, β2-Mikroglobulin und die extrazellulären Domänen α1, α2 und α3 eines Klasse-I-MHC-Moleküls mit einer relativen Häufigkeit von 1 % oder mehr,
ii) ein Paar Disulfid-verknüpfte Polypeptidketten, die von einer Antikörper-Gelenkregion abgeleitet sind, und wobei jede Disulfid-verknüpfte Polypeptidkette eine CH2-Domäne und eine CH3-Domäne menschlichen Ursprungs umfasst, wobei eine CH3-Domäne die Mutation T366W umfasst und die andere CH3-Domäne die Mutationen T366S, L368A und Y407V umfasst (Nummerierungen nach EU-Index von Kabat); und
iii) mindestens ein Paar einer variablen Domäne einer leichten Kette eines Antikörpers und einer variablen Domäne einer schweren Kette eines Antikörpers in einer eukaryotischen Zelle,
welches die folgenden Schritte umfasst:
- Kultivieren einer eukaryotischen Zelle, die eine oder mehrere Nukleinsäuren umfasst, die für den Komplex kodieren, und
- Gewinnen des Komplexes aus der Zelle oder dem Kultivierungsmedium,
wobei der Komplex genau ein Fusionspolypeptid eines T-Zell-Antwort auslösenden Peptids, β2-Mikroglobulin und der extrazellulären Domänen α1, α2 und α3 eines Klasse-I-MHC-Moleküls umfasst, und
wobei das Fusionspolypeptid
- kovalent entweder an den C-Terminus oder den N-Terminus einer der Disulfid-verknüpften Polypeptidketten gebunden ist, oder
- kovalent an den N-Terminus einer variablen Domäne eines Antikörpers gebunden ist, welche die komplementäre variable Domäne der schweren oder leichten Kette zu derjenigen ist, die in der ersten Disulfid-verknüpften Polypeptidkette enthalten ist, oder
- kovalent an den C-Terminus einer konstanten Domäne eines Antikörpers gebunden ist, welche die komplementäre konstante Domäne der schweren oder leichten Kette zu derjenigen ist, die in der ersten Disulfid-verknüpften Polypeptidkette enthalten ist.

2. Verfahren nach Anspruch 1, **dadurch gekennzeichnet, dass** der Komplex in dem Kultivierungsmedium mit einer Konzentration von 1 mg/ml oder mehr erhalten wird.

3. Verfahren nach einem der vorhergehenden Ansprüche, **dadurch gekennzeichnet, dass** die eukaryotische Zelle eine Säugetierzelle ist.

4. Verfahren nach Anspruch 3, **dadurch gekennzeichnet, dass** die Säugetierzelle eine menschliche embryonale Nierenzelle oder eine Eierstockzelle eines chinesischen Hamsters oder eine Nierenzelle eines Hamsterbabys oder eine Myelomzelle einer Maus ist.

5. Verfahren nach einem der vorhergehenden Ansprüche, **dadurch gekennzeichnet, dass** die Disulfid-verknüpften Polypeptidketten, die von einer Antikörper-Gelenkregion abgeleitet sind, i) durch eine oder mehrere Disulfidbindungen verknüpft sind, ii) die erste Disulfid-verknüpfte Polypeptidkette in Richtung vom N- zum C-Terminus eine variable Domäne der leichten oder schweren Immunglobulinkette, eine konstante Domäne der leichten oder schweren Immunglobulinkette und ein Polypeptid einer schweren Antikörperketten-Gelenkregion umfasst und die zweite Disulfid-verknüpfte Polypeptidkette ein Polypeptid einer schweren Antikörperketten-Gelenkregion umfasst.

6. Verfahren nach einem der vorhergehenden Ansprüche, **dadurch gekennzeichnet, dass** das T-Zell-Antwort auslösende Peptid ein Virus-abgeleitetes Peptid ist.

7. Verfahren nach einem der vorhergehenden Ansprüche, **dadurch gekennzeichnet, dass** das Fusionspolypeptid in Richtung vom N- zum C-Terminus umfasst
(i) ein Virus-abgeleitetes Peptid, das eine aus SEQ ID NO: 01 bis SEQ ID NO: 09 ausgewählte Aminosäuresequenz aufweist,
(ii) ein erstes Verbindungspeptid, das eine aus SEQ ID NO: 16, 17, 18, 21, 22 und 23 ausgewählte Aminosäuresequenz aufweist,
(iii) ein β2-Mikroglobulin, das eine Aminosäuresequenz der SEQ ID NO: 10 aufweist,
(iv) ein zweites Verbindungspeptid, das eine aus SEQ ID NO: 16, 17, 18, 21, 22 und 23 ausgewählte Aminosäuresequenz aufweist,
(v) die extrazellulären Domänen α1, α2 und α3 eines Klasse-I-MHC-Moleküls, das eine Aminosäuresequenz der SEQ ID NO: 11 aufweist, und
(vi) ein drittes Verbindungspeptid, das eine aus SEQ ID NO: 12, 16, 17, 18, 21, 22 und 23 ausgewählte Aminosäuresequenz aufweist.

8. Verfahren nach einem der vorhergehenden Ansprüche, **dadurch gekennzeichnet, dass** die erste Disulfid-verknüpfte Polypeptidkette und die zweite Disulfid-verknüpfte Polypeptidkette i) eine menschliche IgG1-CH2-Domäne, die eine Aminosäuresequenz umfasst, ausgewählt aus SEQ ID NO: 31, 32 und 33, und eine menschliche IgG1-CH3-Domäne umfassen, die eine Aminosäuresequenz umfasst, ausgewählt aus SEQ ID NO: 34, 35 und 36.

9. Verfahren nach einem der Ansprüche 7 bis 8, **dadurch gekennzeichnet, dass** der Komplex i) ein erstes Verbindungspeptid, das die Aminosäuresequenz der SEQ ID NO: 21 aufweist, und/oder ii) ein zweites Verbindungspeptid, das die Aminosäuresequenz der SEQ ID NO: 22 aufweist, und/oder iii) ein drittes Verbindungspeptid, das die Aminosäuresequenz der SEQ ID NO: 12 aufweist, und/oder iv) eine menschliche IgG1-CH2-Domäne, welche die Aminosäuresequenz der SEQ ID NO: 32 oder 33 aufweist, und/oder v) im ersten Disulfid-verknüpften Polypeptid eine menschliche IgG1-CH3-Domäne, welche die Aminosäuresequenz der SEQ ID NO: 35 aufweist, und im zweiten Disulfid-verknüpften Polypeptid eine menschliche IgG1-CH3-Domäne umfasst, welche die Aminosäuresequenz SEQ ID NO: 36 aufweist.

10. Komplex, **dadurch gekennzeichnet, dass** er umfasst:
- ein Fusionspolypeptid, das in Richtung vom N- zum C-Terminus umfasst
(i) eine T-Zell-Antwort auslösendes Peptid,
(ii) ein β2-Mikroglobulin und
(iii) die extrazellulären Domänen α1, α2 und α3 eines Klasse-I-MHC-Moleküls mit einer relativen Häufigkeit von 1 % oder mehr,
und
- zwei Polypeptidketten, die durch eine oder mehrere Disulfidbindungen verknüpft sind,
wobei die erste Disulfid-verknüpfte Polypeptidkette in Richtung vom N- zum C-Terminus umfasst
(i) eine variable Domäne einer leichten oder schweren Immunglobulinkette,
(ii) eine konstante Domäne einer leichten oder schweren Immunglobulinkette, und
(iii) ein Polypeptid einer Gelenkregion einer schweren Antikörperkette,
und
wobei die zweite Disulfid-verknüpfte Polypeptidkette ein Polypeptid einer Gelenkregion einer schweren Antikörperkette umfasst, wobei die erste Disulfid-verknüpfte Polypeptidkette und die zweite Disulfid-verknüpfte Polypeptidkette sowohl eine CH2-Domäne als auch eine CH3-Domäne menschlichen Ursprungs umfassen, wobei eine CH3-Domäne die Mutation T366W umfasst und die andere CH3-Domäne die Mutationen T366S, L368A und Y407V umfasst (Nummerierung gemäß EU-Index von Kabat);
wobei das Fusionspolypeptid
- kovalent entweder an den C-Terminus oder den N-Terminus einer der Disulfid-verknüpften Polypeptidketten gebunden ist, oder
- kovalent an den N-Terminus einer variablen Antikörperdomäne gebunden ist, welche die komplementäre variable Domäne der schweren oder leichten Kette zu derjenigen ist, die in der ersten Disulfid-verknüpften Polypeptidkette enthalten ist, oder
- kovalent an den C-Terminus einer konstanten Antikörperdomäne gebunden ist, welche die komplementäre konstante Domäne der schweren oder leichten Kette zu derjenigen ist, die in der ersten Disulfid-verknüpften Polypeptidkette enthalten ist
mit der Maßgabe, dass der Komplex genau ein Fusionspolypeptid umfasst.

11. Komplex nach Anspruch 10, **dadurch gekennzeichnet, dass** das T-Zell-Antwort auslösende Peptid ein Virus-abgeleitetes Peptid ist.

12. Komplex nach einem der Ansprüche 10 oder 11, **dadurch gekennzeichnet, dass** das Fusionspolypeptid in Richtung vom N- zum C-Terminus umfasst
(i) ein Virus-abgeleitetes Peptid, das eine aus SEQ ID NO: 01 bis SEQ ID NO: 09 ausgewählte Aminosäuresequenz aufweist,
(ii) ein erstes Verbindungspeptid, das eine aus SEQ ID NO: 16, 17, 18, 21, 22 und 23 ausgewählte Aminosäuresequenz aufweist,
(iii) ein β2-Mikroglobulin, das eine Aminosäuresequenz der SEQ ID NO: 10 aufweist,
(iv) ein zweites Verbindungspeptid, das eine aus SEQ ID NO: 16, 17, 18, 21, 22 und 23 ausgewählte Aminosäuresequenz aufweist,
(v) die extrazellulären Domänen α1, α2 und α3 eines Klasse-I-MHC-Moleküls, das eine Aminosäuresequenz der SEQ ID NO: 11 aufweist, und
(vi) ein drittes Verbindungspeptid, das eine aus SEQ ID NO: 12, 16, 17, 18, 21, 22 und 23 ausgewählte Aminosäuresequenz aufweist.

13. Komplex nach einem der Ansprüche 10 bis 12, **dadurch gekennzeichnet, dass** das erste Disulfid-verknüpfte Polypeptid und das zweite Disulfid-verknüpfte Polypeptid ferner umfassen:
- eine menschliche IgG1-CH2-Domäne, umfassend eine aus SEQ ID NO: 31, 32 und 33 ausgewählte Aminosäuresequenz, und
- eine menschliche IgG1-CH3-Domäne, umfassend eine aus SEQ ID NO: 34, 35 und 36 ausgewählte Aminosäuresequenz.

14. Komplex nach einem der Ansprüche 10 bis 12, **dadurch gekennzeichnet, dass**
- das erste Verbindungspeptid die Aminosäuresequenz der SEQ ID NO: 21 aufweist und/oder
- das zweite Verbindungspeptid die Aminosäuresequenz der SEQ ID NO: 22 aufweist und/oder
- das dritte Verbindungspeptid die Aminosäuresequenz der SEQ ID NO: 12 aufweist und/oder
- die menschliche IgG1-CH2-Domäne die Aminosäuresequenz der SEQ ID NO: 32 oder 33 aufweist und/oder
- die menschliche IgG1-CH3-Domäne eines Disulfid-verknüpften Polypeptids die Aminosäuresequenz der SEQ ID NO: 35 aufweist und die menschliche IgG1-CH3-Domäne des anderen Disulfid-verknüpften Polypeptids die Aminosäuresequenz der SEQ ID NO: 36 aufweist.

15. Nukleinsäure, die für den Komplex nach einem der Ansprüche 10 bis 14 kodiert.

16. Wirtszelle, die eine Nukleinsäure nach Anspruch 15 umfasst.

17. Pharmazeutische Formulierung, die den Komplex nach einem der Ansprüche 10 bis 14 und gegebenenfalls einen pharmazeutisch verträglichen Träger umfasst.

18. Komplex nach einem der Ansprüche 10 bis 14 zur Verwendung als Arzneimittel.

19. Komplex nach einem der Ansprüche 10 bis 14 zur Verwendung bei der Behandlung von Krebs oder einer chronischen Virusinfektion.

20. Komplex nach einem der Ansprüche 10 bis 14 zur Verwendung in der Therapie durch Anziehen von virusspezifischen zytotoxischen T-Zellen eines Individuums zu einem Target.

21. Komplex nach einem der Ansprüche 10 bis 14 zur Verwendung in der Therapie durch Töten von Krebszellen oder von virusinfizierten Zellen.

## Revendications

1. Procédé pour la production par recombinaison d'un complexe comprenant
i) un polypeptide de fusion comprenant dans le sens N- à C-terminal un peptide provoquant la réponse des lymphocytes T, une β2-microglobuline et les domaines extracellulaires α1, α2, et α3 d'une molécule CMH de classe I présente à une fréquence relative de 1 % ou plus,
ii) une paire de chaînes polypeptidiques à liaison disulfure dérivées d'une région charnière d'anticorps, et chaque chaîne polypeptidique à liaison disulfure comprenant un domaine CH2 et un domaine CH3 d'origine humaine, un domaine CH3 comprenant la mutation T366W et l'autre domaine CH3 comprenant les mutations T366S, L368A et Y407V (numérotation selon l'indice EU de Kabat) ; et
iii) au moins une paire formée d'un domaine variable de chaîne légère d'anticorps et d'un domaine variable de chaîne lourde d'anticorps dans une cellule eucaryote,
comprenant les étapes suivantes, à savoir :
- la culture d'une cellule eucaryote comprenant un ou plusieurs acides nucléiques codant pour le complexe, et
- la récupération du complexe à partir de la cellule ou du milieu de culture,
le complexe comprenant exactement un polypeptide de fusion d'un peptide provoquant la réponse des lymphocytes T, une β2-microglobuline et les domaines extracellulaires α1, α2, et α3 d'une molécule CMH de classe I, et
le polypeptide de fusion étant
- lié par covalence soit à l'extrémité C-terminale soit à l'extrémité N-terminale de l'une des chaînes polypeptidiques à liaison disulfure, ou
- lié par covalence à l'extrémité N-terminale d'un domaine variable d'anticorps qui est le domaine variable de chaîne lourde ou légère complémentaire de celui compris dans la première chaîne polypeptidique à liaison disulfure, ou
- lié par covalence à l'extrémité C-terminale d'un domaine constant d'anticorps qui est le domaine constant de chaîne lourde ou légère complémentaire de celui compris dans la première chaîne polypeptidique à liaison disulfure.

2. Procédé selon la revendication 1, **caractérisé en ce que** le complexe est obtenu à une concentration de 1 mg/ml ou plus dans le milieu de culture.

3. Procédé selon l'une quelconque des revendications précédentes, **caractérisé en ce que** la cellule eucaryote est une cellule de mammifère.

4. Procédé selon la revendication 3, **caractérisé en ce que** la cellule de mammifère est une cellule rénale embryonnaire humaine, une cellule ovarienne de hamster chinois, une cellule rénale de hamster nouveau-né ou une cellule de myélome de souris.

5. Procédé selon l'une quelconque des revendications précédentes, **caractérisé en ce que** les chaînes polypeptidiques à liaison disulfure dérivées d'une région charnière d'anticorps sont i) liées par une ou plusieurs liaisons disulfure, ii) la première chaîne polypeptidique à liaison disulfure comprend dans le sens N- à C-terminal un domaine variable de chaîne lourde ou légère d'immunoglobuline, un domaine constant de chaîne lourde ou légère d'immunoglobuline et un polypeptide de région charnière de chaîne lourde d'anticorps, et la deuxième chaîne polypeptidique à liaison disulfure comprend un polypeptide de région charnière de chaîne lourde d'anticorps.

6. Procédé selon l'une quelconque des revendications précédentes, **caractérisé en ce que** le peptide provoquant la réponse des lymphocytes T est un peptide dérivé de virus.

7. Procédé selon l'une quelconque des revendications précédentes, **caractérisé en ce que** le polypeptide de fusion comprend dans le sens N- à C-terminal
(i) un peptide dérivé de virus qui a une séquence d'acides aminés choisie parmi les SEQ ID NO : 01 à SEQ ID NO : 09,
(ii) un premier peptide lieur qui a une séquence d'acides aminés choisie parmi les SEQ ID NO : 16, 17, 18, 21, 22 et 23,
(iii) une β2-microglobuline qui a une séquence d'acides aminés SEQ ID NO : 10,
(iv) un deuxième peptide lieur qui a une séquence d'acides aminés choisie parmi les SEQ ID NO : 16, 17, 18, 21, 22 et 23,
(v) les domaines extracellulaires α1, α2 et α3 d'une molécule du CMH de classe I qui a une séquence d'acides aminés SEQ ID NO : 11, et
(vi) un troisième peptide lieur qui a une séquence d'acides aminés choisie parmi les SEQ ID NO : 12, 16, 17, 18, 21, 22 et 23.

8. Procédé selon l'une quelconque des revendications précédentes, **caractérisé en ce que** la première chaîne polypeptidique à liaison disulfure et la seconde chaîne polypeptidique à liaison disulfure comprennent i) un domaine CH2 d'IgG1 humaine comprenant une séquence d'acides aminés choisie parmi les SEQ ID NO : 31, 32 et 33, et un domaine CH3 d'IgG1 humaine comprenant une séquence d'acides aminés choisie parmi les SEQ ID NO : 34, 35 et 36.

9. Procédé selon l'une quelconque des revendications 7 à 8, **caractérisé en ce que** le complexe comprend i) un premier peptide lieur qui a la séquence d'acides aminés SEQ ID NO : 21, et/ou ii) un deuxième peptide lieur qui a la séquence d'acides aminés SEQ ID NO : 22, et/ou iii) un troisième peptide lieur qui a la séquence d'acides aminés SEQ ID NO : 12, et/ou iv) un domaine CH2 d'IgG1 humaine qui a la séquence d'acides aminés SEQ ID NO : 32 ou 33, et/ou v) dans le premier polypeptide à liaison disulfure, un domaine CH3 d'IgG1 humaine qui a la séquence d'acides aminés SEQ ID NO : 35 et dans le deuxième polypeptide à liaison disulfure, un domaine CH3 d'IgG1 humaine qui a la séquence d'acides aminés SEQ ID NO : 36.

10. Complexe, **caractérisé en ce qu'**il comprend
- un polypeptide de fusion qui comprend dans le sens N- à C-terminal
(i) un peptide provoquant la réponse des lymphocytes T,
(ii) une β2-microglobuline, et
iii) les domaines extracellulaires α1, α2, et α3 d'une molécule CMH de classe I présente à une fréquence relative de 1 % ou plus,
et
- deux chaînes polypeptidiques qui sont liées par une ou plusieurs liaisons disulfure, la première chaîne polypeptidique à liaison disulfure comprenant dans le sens N- à C-terminal
(i) un domaine variable de chaîne lourde ou légère d'immunoglobuline,
(ii) un domaine constant de chaîne lourde ou légère d'immunoglobuline, et
(iii) un polypeptide de région charnière de chaîne lourde d'anticorps,
et
la deuxième chaîne polypeptidique à liaison disulfure comprenant un polypeptide de région charnière de chaîne lourde d'anticorps, la première chaîne polypeptidique à liaison disulfure et la deuxième chaîne polypeptidique à liaison disulfure comprenant toutes deux un domaine CH2 et un domaine CH3 d'origine humaine, un domaine CH3 comprenant la mutation T366W et l'autre domaine CH3 comprenant les mutations T366S, L368A et Y407V (numérotation selon l'indice EU de Kabat) ;
le polypeptide de fusion étant
- lié par covalence soit à l'extrémité C-terminale soit à l'extrémité N-terminale de l'une des chaînes polypeptidiques à liaison disulfure, ou
- lié par covalence à l'extrémité N-terminale d'un domaine variable d'anticorps qui est le domaine variable de chaîne lourde ou légère complémentaire de celui compris dans la première chaîne polypeptidique à liaison disulfure, ou
- lié par covalence à l'extrémité C-terminale d'un domaine constant d'anticorps qui est le domaine constant de chaîne lourde ou légère complémentaire de celui compris dans la première chaîne polypeptidique à liaison disulfure
à condition que le complexe comprenne exactement un polypeptide de fusion.

11. Complexe selon la revendication 10, **caractérisé en ce que** le peptide provoquant la réponse des lymphocytes T est un peptide dérivé de virus.

12. Complexe selon l'une quelconque des revendications 10 ou 11, **caractérisé en ce que** le polypeptide de fusion comprend, dans le sens N- à C-terminal,
(i) un peptide dérivé de virus qui a une séquence d'acides aminés choisie parmi les SEQ ID NO : 01 à SEQ ID NO : 09,
(ii) un premier peptide lieur qui a une séquence d'acides aminés choisie parmi les SEQ ID NO : 16, 17, 18, 21, 22 et 23.
(iii) une β2-microglobuline qui a une séquence d'acides aminés SEQ ID NO : 10,
(iv) un deuxième peptide lieur qui a une séquence d'acides aminés choisie parmi les SEQ ID NO : 16, 17, 18, 21, 22 et 23.
(v) les domaines extracellulaires α1, α2 et α3 d'une molécule du CMH de classe I qui a une séquence d'acides aminés SEQ ID NO : 11, et
(vi) un troisième peptide lieur qui a une séquence d'acides aminés choisie parmi les SEQ ID NO : 12, 16, 17, 18, 21, 22 et 23.

13. Complexe selon l'une quelconque des revendications 10 à 12, **caractérisé en ce que** le premier polypeptide à liaison disulfure et le deuxième polypeptide à liaison disulfure comprennent en outre
- un domaine CH2 d'IgG1 humaine comprenant une séquence d'acides aminés choisie parmi les SEQ ID NO : 31, 32, et 33, et
- un domaine CH3 d'IgG1 humaine comprenant une séquence d'acides aminés choisie parmi les SEQ ID NO : 34, 35, et 36.

14. Complexe selon l'une quelconque des revendications 10 à 12, **caractérisé en ce que**
- le premier peptide lieur a la séquence d'acides aminés SEQ ID NO : 21, et/ou
- le deuxième peptide lieur a la séquence d'acides aminés SEQ ID NO : 22, et/ou
- le troisième peptide lieur a la séquence d'acides aminés SEQ ID NO : 12, et/ou
- le domaine CH2 d'IgG1 humaine a la séquence d'acides aminés SEQ ID NO : 32 ou 33, et/ou
- le domaine CH3 d'IgG1 humaine d'un polypeptide à liaison disulfure a la séquence d'acides aminés SEQ ID NO : 35 et le domaine CH3 d'IgG1 humaine de l'autre polypeptide à liaison disulfure a la séquence d'acides aminés SEQ ID NO : 36.

15. Acide nucléique codant pour le complexe selon l'une quelconque des revendications 10 à 14.

16. Cellule hôte comprenant l'acide nucléique selon la revendication 15.

17. Formulation pharmaceutique comprenant le complexe selon l'une quelconque des revendications 10 à 14 et éventuellement un véhicule pharmaceutiquement acceptable.

18. Complexe selon l'une quelconque des revendications 10 à 14, destiné à être utilisé comme médicament.

19. Complexe selon l'une quelconque des revendications 10 à 14 destiné à être utilisé dans le traitement du cancer ou d'une infection virale chronique.

20. Complexe selon l'une quelconque des revendications 10 à 14 destiné à être utilisé en thérapie en attirant vers une cible les lymphocytes T cytotoxiques spécifiques aux virus d'un individu.

21. Complexe selon l'une quelconque des revendications 10 à 14 destiné à être utilisé en thérapie en éliminant les cellules cancéreuses ou les cellules infectées par un virus.


### SEQUENCE LISTING

<110> F. Hoffmann-La Roche AG
<120> Removal of target cells by circulating virus-specific cytotoxic T-cells using MHC class I comprising complexes
<130> 27493
<150> EP11171027.3
   <151> 2011-06-22
<160> 112
<170> PatentIn version 3.5
<210> 1
   <211> 9
   <212> PRT
   <213> Human cytomegalovirus
<400> 1
   Asn Leu Val Pro Met Val Ala Thr Val
   1               5
<210> 2
   <211> 9
   <212> PRT
   <213> Human immunodeficiency virus
<400> 2
   Ser Leu Tyr Asn Thr Val Ala Thr Leu
   1               5
<210> 3
   <211> 9
   <212> PRT
   <213> Human herpesvirus 4
<400> 3
   Gly Leu Cys Thr Leu Val Ala Met Leu
   1               5
<210> 4
   <211> 9
   <212> PRT
   <213> Influenza A virus
<400> 4
   Gly Ile Leu Gly Phe Val Phe Thr Leu
   1               5
<210> 5
   <211> 9
   <212> PRT
   <213> Hepatitis B virus
<400> 5
   Ser Thr Asn Arg Gln Ser Gly Arg Gln
   1               5
<210> 6
   <211> 9
   <212> PRT
   <213> Human T-cell lymphotropic virus type 1
<400> 6
   Leu Leu Phe Gly Tyr Pro Val Tyr Val
   1               5
<210> 7
   <211> 9
   <212> PRT
   <213> Artificial Sequence
<220>
   <223> V-jun Sarcoma Virus 17 Oncogene Homolog (JUN)
<400> 7
   Phe Ala Glu Gly Phe Val Arg Ala Leu
   1               5
<210> 8
   <211> 9
   <212> PRT
   <213> Human adenovirus type 3
<400> 8
   Leu Ile Val Ile Gly Ile Leu Ile Leu
   1               5
<210> 9
   <211> 8
   <212> PRT
   <213> Hepatitis C virus
<400> 9
   Ile Leu His Thr Pro Gly Cys Val
   1               5
<210> 10
   <211> 99
   <212> PRT
   <213> Homo sapiens
<400> 10
<210> 11
   <211> 274
   <212> PRT
   <213> Homo sapiens
<400> 11
<210> 12
   <211> 2
   <212> PRT
   <213> Artificial Sequence
<220>
   <223> linker peptide 1
<400> 12
   Gly Ser
   1
<210> 13
   <211> 3
   <212> PRT
   <213> Artificial Sequence
<220>
   <223> linker peptide 2
<400> 13
   Gly Gly Ser
   1
<210> 14
   <211> 4
   <212> PRT
   <213> Artificial Sequence
<220>
   <223> linker peptide 3
<400> 14
   Gly Gly Gly Ser
   1
<210> 15
   <211> 8
   <212> PRT
   <213> Artificial Sequence
<220>
   <223> linker peptide 4
<400> 15
   Gly Gly Gly Ser Gly Gly Gly Ser
   1               5
<210> 16
   <211> 12
   <212> PRT
   <213> Artificial Sequence
<220>
   <223> linker peptide 5
<400> 16
   Gly Gly Gly Ser Gly Gly Gly Ser Gly Gly Gly Ser
   1               5                   10
<210> 17
   <211> 16
   <212> PRT
   <213> Artificial Sequence
<220>
   <223> linker peptide 6
<400> 17
   Gly Gly Gly Ser Gly Gly Gly Ser Gly Gly Gly Ser Gly Gly Gly Ser
   1               5                   10                  15
<210> 18
   <211> 20
   <212> PRT
   <213> Artificial Sequence
<220>
   <223> linker peptide 7
<400> 18
   Gly Gly Gly Ser Gly Gly Gly Ser Gly Gly Gly Ser Gly Gly Gly Ser
   1               5                   10                  15
   Gly Gly Gly Ser
               20
<210> 19
   <211> 5
   <212> PRT
   <213> Artificial Sequence
<220>
   <223> linker peptide 8
<400> 19
   Gly Gly Gly Gly Ser
   1               5
<210> 20
   <211> 10
   <212> PRT
   <213> Artificial Sequence
<220>
   <223> linker peptide 9
<400> 20
   Gly Gly Gly Gly Ser Gly Gly Gly Gly Ser
   1               5                   10
<210> 21
   <211> 15
   <212> PRT
   <213> Artificial Sequence
<220>
   <223> linker peptide 10
<400> 21
   Gly Gly Gly Gly Ser Gly Gly Gly Gly Ser Gly Gly Gly Gly Ser
   1               5                   10                  15
<210> 22
   <211> 20
   <212> PRT
   <213> Artificial Sequence
<220>
   <223> linker peptide 11
<400> 22
   Gly Gly Gly Gly Ser Gly Gly Gly Gly Ser Gly Gly Gly Gly Ser Gly
   1               5                   10                  15
   Gly Gly Gly Ser
               20
<210> 23
   <211> 25
   <212> PRT
   <213> Artificial Sequence
<220>
   <223> linker peptide 12
<400> 23
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser
               20                  25
<210> 24
   <211> 15
   <212> PRT
   <213> Homo sapiens
<400> 24
   Glu Pro Lys Ser Cys Asp Lys Thr His Thr Cys Pro Pro Cys Pro
   1               5                   10                  15
<210> 25
   <211> 15
   <212> PRT
   <213> Artificial Sequence
<220>
   <223> human IgG1 heavy chain hinge region polypeptide variant
<400> 25
   Glu Pro Lys Ser Ala Asp Lys Thr His Thr Cys Pro Pro Cys Pro
   1               5                   10                  15
<210> 26
   <211> 12
   <212> PRT
   <213> Homo sapiens
<400> 26
   Glu Arg Lys Cys Cys Val Glu Cys Pro Pro Cys Pro
   1               5                   10
<210> 27
   <211> 12
   <212> PRT
   <213> Artificial Sequence
<220>
   <223> human IgG2 heavy chain hinge region polypeptide variant 1
<400> 27
   Glu Arg Lys Cys Ala Val Glu Cys Pro Pro Cys Pro
   1               5                   10
<210> 28
   <211> 12
   <212> PRT
   <213> Artificial Sequence
<220>
   <223> human IgG2 heavy chain hinge region polypeptide variant 2
<400> 28
   Glu Arg Lys Ala Cys Val Glu Cys Pro Pro Cys Pro
   1               5                   10
<210> 29
   <211> 62
   <212> PRT
   <213> Homo sapiens
<400> 29
   Glu Leu Lys Thr Pro Leu Gly Asp Thr Thr His Thr Cys Pro Arg Cys
   1               5                   10                  15
   Pro Glu Pro Lys Ser Cys Asp Thr Pro Pro Pro Cys Pro Arg Cys Pro
               20                  25                  30
   Glu Pro Lys Ser Cys Asp Thr Pro Pro Pro Cys Pro Arg Cys Pro Glu
           35                  40                  45
   Pro Lys Ser Cys Asp Thr Pro Pro Pro Cys Pro Arg Cys Pro
       50                  55                  60
<210> 30
   <211> 12
   <212> PRT
   <213> Homo sapiens
<400> 30
   Glu Ser Lys Tyr Gly Pro Pro Cys Pro Ser Cys Pro
   1               5                   10
<210> 31
   <211> 107
   <212> PRT
   <213> Homo sapiens
<400> 31
   Leu Leu Gly Gly Pro Ser Val Phe Leu Phe Pro Pro Lys Pro Lys Asp
   1               5                   10                  15
   Thr Leu Met Ile Ser Arg Thr Pro Glu Val Thr Cys Val Val Val Asp
               20                  25                  30
   Val Ser His Glu Asp Pro Glu Val Lys Phe Asn Trp Tyr Val Asp Gly
           35                  40                  45
   Val Glu Val His Asn Ala Lys Thr Lys Pro Arg Glu Glu Gln Tyr Asn
       50                  55                  60
   Ser Thr Tyr Arg Val Val Ser Val Leu Thr Val Leu His Gln Asp Trp
   65                  70                  75                  80
   Leu Asn Gly Lys Glu Tyr Lys Cys Lys Val Ser Asn Lys Ala Leu Pro
                   85                  90                  95
   Ala Pro Ile Glu Lys Thr Ile Ser Lys Ala Lys
               100                 105
<210> 32
   <211> 107
   <212> PRT
   <213> Artificial Sequence
<220>
   <223> huamn IgG1 CH2 domain with the mutations L234A and L235A
<400> 32
   Ala Ala Gly Gly Pro Ser Val Phe Leu Phe Pro Pro Lys Pro Lys Asp
   1               5                   10                  15
   Thr Leu Met Ile Ser Arg Thr Pro Glu Val Thr Cys Val Val Val Asp
               20                  25                  30
   Val Ser His Glu Asp Pro Glu Val Lys Phe Asn Trp Tyr Val Asp Gly
           35                  40                  45
   Val Glu Val His Asn Ala Lys Thr Lys Pro Arg Glu Glu Gln Tyr Asn
       50                  55                  60
   Ser Thr Tyr Arg Val Val Ser Val Leu Thr Val Leu His Gln Asp Trp
   65                  70                  75                  80
   Leu Asn Gly Lys Glu Tyr Lys Cys Lys Val Ser Asn Lys Ala Leu Pro
                   85                  90                  95
   Ala Pro Ile Glu Lys Thr Ile Ser Lys Ala Lys
               100                 105
<210> 33
   <211> 107
   <212> PRT
   <213> Artificial Sequence
<220>
   <223> human IgG CH2 domain with the mutations L234A, L235A and P329G
<400> 33
   Ala Ala Gly Gly Pro Ser Val Phe Leu Phe Pro Pro Lys Pro Lys Asp
   1               5                   10                  15
   Thr Leu Met Ile Ser Arg Thr Pro Glu Val Thr Cys Val Val Val Asp
               20                  25                  30
   Val Ser His Glu Asp Pro Glu Val Lys Phe Asn Trp Tyr Val Asp Gly
           35                  40                  45
   Val Glu Val His Asn Ala Lys Thr Lys Pro Arg Glu Glu Gln Tyr Asn
       50                  55                  60
   Ser Thr Tyr Arg Val Val Ser Val Leu Thr Val Leu His Gln Asp Trp
   65                  70                  75                  80
   Leu Asn Gly Lys Glu Tyr Lys Cys Lys Val Ser Asn Lys Ala Leu Gly
                   85                  90                  95
   Ala Pro Ile Glu Lys Thr Ile Ser Lys Ala Lys
               100                 105
<210> 34
   <211> 105
   <212> PRT
   <213> Homo sapiens
<400> 34
   Pro Arg Glu Pro Gln Val Tyr Thr Leu Pro Pro Ser Arg Asp Glu Leu
   1               5                   10                  15
   Thr Lys Asn Gln Val Ser Leu Thr Cys Leu Val Lys Gly Phe Tyr Pro
               20                  25                  30
   Ser Asp Ile Ala Val Glu Trp Glu Ser Asn Gly Gln Pro Glu Asn Asn
           35                  40                  45
   Tyr Lys Thr Thr Pro Pro Val Leu Asp Ser Asp Gly Ser Phe Phe Leu
       50                  55                  60
   Tyr Ser Lys Leu Thr Val Asp Lys Ser Arg Trp Gln Gln Gly Asn Val
   65                  70                  75                  80
   Phe Ser Cys Ser Val Met His Glu Ala Leu His Asn His Tyr Thr Gln
                   85                  90                  95
   Lys Ser Leu Ser Leu Ser Pro Gly Lys
               100                 105
<210> 35
   <211> 105
   <212> PRT
   <213> Artificial Sequence
<220>
   <223> human IgG1 CH3 domain knob variant
<400> 35
   Pro Arg Glu Pro Gln Val Tyr Thr Leu Pro Pro Cys Arg Asp Glu Leu
   1               5                   10                  15
   Thr Lys Asn Gln Val Ser Leu Trp Cys Leu Val Lys Gly Phe Tyr Pro
               20                  25                  30
   Ser Asp Ile Ala Val Glu Trp Glu Ser Asn Gly Gln Pro Glu Asn Asn
           35                  40                  45
   Tyr Lys Thr Thr Pro Pro Val Leu Asp Ser Asp Gly Ser Phe Phe Leu
       50                  55                  60
   Tyr Ser Lys Leu Thr Val Asp Lys Ser Arg Trp Gln Gln Gly Asn Val
   65                  70                  75                  80
   Phe Ser Cys Ser Val Met His Glu Ala Leu His Asn His Tyr Thr Gln
                   85                  90                  95
   Lys Ser Leu Ser Leu Ser Pro Gly Lys
               100                 105
<210> 36
   <211> 105
   <212> PRT
   <213> Artificial Sequence
<220>
   <223> human IgG1 CH3 domain hole variant
<400> 36
   Pro Arg Glu Pro Gln Val Cys Thr Leu Pro Pro Ser Arg Asp Glu Leu
   1               5                   10                  15
   Thr Lys Asn Gln Val Ser Leu Ser Cys Ala Val Lys Gly Phe Tyr Pro
               20                  25                  30
   Ser Asp Ile Ala Val Glu Trp Glu Ser Asn Gly Gln Pro Glu Asn Asn
           35                  40                  45
   Tyr Lys Thr Thr Pro Pro Val Leu Asp Ser Asp Gly Ser Phe Phe Leu
       50                  55                  60
   Val Ser Lys Leu Thr Val Asp Lys Ser Arg Trp Gln Gln Gly Asn Val
   65                  70                  75                  80
   Phe Ser Cys Ser Val Met His Glu Ala Leu His Asn His Tyr Thr Gln
                   85                  90                  95
   Lys Ser Leu Ser Leu Ser Pro Gly Lys
               100                 105
<210> 37
   <211> 232
   <212> PRT
   <213> Homo sapiens
<400> 37
   Glu Pro Lys Ser Cys Asp Lys Thr His Thr Cys Pro Pro Cys Pro Ala
   1               5                   10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65 70 75 80
   Tyr Asn Ser Thr Tyr Arg Val Val Ser Val Leu Thr Val Leu His Gln
   85 90 95
   Asp Trp Leu Asn Gly Lys Glu Tyr Lys Cys Lys Val Ser Asn Lys Ala
   100 105 110
   Leu Pro Ala Pro Ile Glu Lys Thr Ile Ser Lys Ala Lys Gly Gln Pro
   115 120 125
   Arg Glu Pro Gln Val Tyr Thr Leu Pro Pro Ser Arg Asp Glu Leu Thr
   130 135 140
   Lys Asn Gln Val Ser Leu Thr Cys Leu Val Lys Gly Phe Tyr Pro Ser
   145 150 155 160
   Asp Ile Ala Val Glu Trp Glu Ser Asn Gly Gln Pro Glu Asn Asn Tyr
   165 170 175
   Lys Thr Thr Pro Pro Val Leu Asp Ser Asp Gly Ser Phe Phe Leu Tyr
   180 185 190
   Ser Lys Leu Thr Val Asp Lys Ser Arg Trp Gln Gln Gly Asn Val Phe
   195 200 205
   Ser Cys Ser Val Met His Glu Ala Leu His Asn His Tyr Thr Gln Lys
   210 215 220
   Ser Leu Ser Leu Ser Pro Gly Lys
   225 230
<210> 38
   <211> 232
   <212> PRT
   <213> Artificial Sequence
<220>
   <223> human IgG1 heavy chain Fc-region with the mutations L234A and L235A
<400> 38
   Glu Pro Lys Ser Cys Asp Lys Thr His Thr Cys Pro Pro Cys Pro Ala
   1               5                   10                  15
   Pro Glu Ala Ala Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Ser Arg Asp Glu Leu Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 39
   <211> 232
   <212> PRT
   <213> Artificial Sequence
<220>
   <223> human IgG1 heavy chain Fc-region wiith mutations L234A and L235A as hole variant
<400> 39
   Glu Pro Lys Ser Cys Asp Lys Thr His Thr Cys Pro Pro Cys Pro Ala
   1               5                   10                  15
   Pro Glu Ala Ala Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Cys Thr Leu Pro Pro Ser Arg Asp Glu Leu Thr
       130                 135                 140
   Lys Asn Gln Val Ser Leu Ser Cys Ala Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Val
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 40
   <211> 232
   <212> PRT
   <213> Artificial Sequence
<220>
   <223> human IgG1 heavy chain Fc region with mutations L234A and L235A knob variant
<400> 40
   Glu Pro Lys Ser Cys Asp Lys Thr His Thr Cys Pro Pro Cys Pro Ala
   1               5                   10                  15
   Pro Glu Ala Ala Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Cys Arg Asp Glu Leu Thr
       130                 135                 140
   Lys Asn Gln Val Ser Leu Trp Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225 230
<210> 41
   <211> 215
   <212> PRT
   <213> Artificial Sequence
<220>
   <223> Ig Light Chain
<400> 41
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Val Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Lys Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Arg Ser Lys Trp Pro Pro
                   85                  90                  95
   Trp Thr Phe Gly Gln Gly Thr Lys Val Glu Ser Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
                 195                 200                 205
   Ser Phe Asn Arg Gly Glu Cys
       210                 215
<210> 42
   <211> 448
   <212> PRT
   <213> Artificial Sequence
<220>
   <223> Ig Heavy Chain (IgG1-L234A, L235A mutant)
<400> 42
   Gln Val Glu Leu Val Glu Ser Gly Gly Gly Val Val Gln Pro Gly Arg
   1               5                   10                  15
   Ser Gln Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Ile Ile Trp Phe Asp Gly Ser Ser Thr Tyr Tyr Ala Asp Ser Val
       50                  55                  60
   Arg Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Phe Cys
                   85                  90                  95
   Ala Arg Glu Leu Gly Arg Arg Tyr Phe Asp Leu Trp Gly Arg Gly Thr
               100                 105                 110
   Leu Val Ser Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
   195 200 205
   Asn Thr Lys Val Asp Lys Lys Val Glu Pro Lys Ser Cys Asp Lys Thr
   210 215 220
   His Thr Cys Pro Pro Cys Pro Ala Pro Glu Ala Ala Gly Gly Pro Ser
   225 230 235 240
   Val Phe Leu Phe Pro Pro Lys Pro Lys Asp Thr Leu Met Ile Ser Arg
   245 250 255
   Thr Pro Glu Val Thr Cys Val Val Val Asp Val Ser His Glu Asp Pro
   260 265 270
   Glu Val Lys Phe Asn Trp Tyr Val Asp Gly Val Glu Val His Asn Ala
   275 280 285
   Lys Thr Lys Pro Arg Glu Glu Gln Tyr Asn Ser Thr Tyr Arg Val Val
   290 295 300
   Ser Val Leu Thr Val Leu His Gln Asp Trp Leu Asn Gly Lys Glu Tyr
   305 310 315 320
   Lys Cys Lys Val Ser Asn Lys Ala Leu Pro Ala Pro Ile Glu Lys Thr
   325 330 335
   Ile Ser Lys Ala Lys Gly Gln Pro Arg Glu Pro Gln Val Tyr Thr Leu
   340 345 350
   Pro Pro Ser Arg Asp Glu Leu Thr Lys Asn Gln Val Ser Leu Thr Cys
   355 360 365
   Leu Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Tyr Ser Lys Leu Thr Val Asp Lys Ser
   405 410 415
   Arg Trp Gln Gln Gly Asn Val Phe Ser Cys Ser Val Met His Glu Ala
   420 425 430
   Leu His Asn His Tyr Thr Gln Lys Ser Leu Ser Leu Ser Pro Gly Lys
   435 440 445
<210> 43
   <211> 448
   <212> PRT
   <213> Artificial Sequence
<220>
   <223> Ig Heavy Chain (IgG1-L234A, L235A mutant with knob variation)
<400> 43
   Gln Val Glu Leu Val Glu Ser Gly Gly Gly Val Val Gln Pro Gly Arg
   1 5 10 15
   Ser Gln Arg Leu Ser Cys Ala Ala Ser Gly Phe Thr Phe Ser Ser Tyr
   20 25 30
   Gly Met His Trp Val Arg Gln Ala Pro Gly Lys Gly Leu Glu Trp Val
   35 40 45
   Ala Ile Ile Trp Phe Asp Gly Ser Ser Thr Tyr Tyr Ala Asp Ser Val
   50 55 60
   Arg Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Phe Cys
   85 90 95
   Ala Arg Glu Leu Gly Arg Arg Tyr Phe Asp Leu Trp Gly Arg Gly Thr
   100 105 110
   Leu Val Ser Val Ser Ser Ala Ser Thr Lys Gly Pro Ser Val Phe Pro
   115 120 125
   Leu Ala Pro Ser Ser Lys Ser Thr Ser Gly Gly Thr Ala Ala Leu Gly
   130 135 140
   Cys Leu Val Lys Asp Tyr Phe Pro Glu Pro Val Thr Val Ser Trp Asn
   145 150 155 160
   Ser Gly Ala Leu Thr Ser Gly Val His Thr Phe Pro Ala Val Leu Gln
   165 170 175
   Ser Ser Gly Leu Tyr Ser Leu Ser Ser Val Val Thr Val Pro Ser Ser
   180 185 190
   Ser Leu Gly Thr Gln Thr Tyr Ile Cys Asn Val Asn His Lys Pro Ser
   195 200 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Ala Ala Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Cys Arg Asp Glu Leu Thr Lys Asn Gln Val Ser Leu Trp Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Tyr Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly Lys
           435                 440                 445
<210> 44
   <211> 448
   <212> PRT
   <213> Artificial Sequence
<220>
   <223> Ig Heavy Chain (IgG1-L234A, L235A mutant with hole variation)
<400> 44
   Gln Val Glu Leu Val Glu Ser Gly Gly Gly Val Val Gln Pro Gly Arg
   1               5                   10                  15
   Ser Gln Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Ile Ile Trp Phe Asp Gly Ser Ser Thr Tyr Tyr Ala Asp Ser Val
       50                  55                  60
   Arg Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Phe Cys
                   85                  90                  95
   Ala Arg Glu Leu Gly Arg Arg Tyr Phe Asp Leu Trp Gly Arg Gly Thr
               100                 105                 110
   Leu Val Ser Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Ala Ala Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Cys Thr Leu
               340                 345                 350
   Pro Pro Ser Arg Asp Glu Leu Thr Lys Asn Gln Val Ser Leu Ser Cys
           355                 360                 365
   Ala Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Val Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly Lys
           435                 440                 445
<210> 45
   <211> 232
   <212> PRT
   <213> Artificial Sequence
<220>
   <223> Ig heavy chain Fc region (IgG1-L234A, L235A mutant Fc-region)
<400> 45
   Glu Pro Lys Ser Ala Asp Lys Thr His Thr Cys Pro Pro Cys Pro Ala
   1 5 10 15
   Pro Glu Ala Ala Gly Gly Pro Ser Val Phe Leu Phe Pro Pro Lys Pro
   20 25 30
   Lys Asp Thr Leu Met Ile Ser Arg Thr Pro Glu Val Thr Cys Val Val
   35 40 45
   Val Asp Val Ser His Glu Asp Pro Glu Val Lys Phe Asn Trp Tyr Val
   50 55 60
   Asp Gly Val Glu Val His Asn Ala Lys Thr Lys Pro Arg Glu Glu Gln
   65 70 75 80
   Tyr Asn Ser Thr Tyr Arg Val Val Ser Val Leu Thr Val Leu His Gln
   85 90 95
   Asp Trp Leu Asn Gly Lys Glu Tyr Lys Cys Lys Val Ser Asn Lys Ala
   100 105 110
   Leu Pro Ala Pro Ile Glu Lys Thr Ile Ser Lys Ala Lys Gly Gln Pro
   115 120 125
   Arg Glu Pro Gln Val Tyr Thr Leu Pro Pro Cys Arg Asp Glu Leu Thr
   130 135 140
   Lys Asn Gln Val Ser Leu Trp Cys Leu Val Lys Gly Phe Tyr Pro Ser
   145 150 155 160
   Asp Ile Ala Val Glu Trp Glu Ser Asn Gly Gln Pro Glu Asn Asn Tyr
   165 170 175
   Lys Thr Thr Pro Pro Val Leu Asp Ser Asp Gly Ser Phe Phe Leu Tyr
   180 185 190
   Ser Lys Leu Thr Val Asp Lys Ser Arg Trp Gln Gln Gly Asn Val Phe
   195 200 205
   Ser Cys Ser Val Met His Glu Ala Leu His Asn His Tyr Thr Gln Lys
   210 215 220
   Ser Leu Ser Leu Ser Pro Gly Lys
   225 230
<210> 46
   <211> 246
   <212> PRT
   <213> Artificial Sequence
<220>
   <223> scFv
<400> 46
   Gln Val Glu Leu Val Glu Ser Gly Gly Gly Val Val Gln Pro Gly Arg
   1               5                   10                  15
   Ser Gln Arg Leu Ser Cys Ala Ala Ser Gly Phe Thr Phe Ser Ser Tyr
               20                  25                  30
   Gly Met His Trp Val Arg Gln Ala Pro Gly Lys Cys Leu Glu Trp Val
           35                  40                  45
   Ala Ile Ile Trp Phe Asp Gly Ser Ser Thr Tyr Tyr Ala Asp Ser Val
       50                  55                  60
   Arg Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Phe Cys
                   85                  90                  95
   Ala Arg Glu Leu Gly Arg Arg Tyr Phe Asp Leu Trp Gly Arg Gly Thr
               100                 105                 110
   Leu Val Ser Val Ser Ser Gly Gly Gly Gly Ser Gly Gly Gly Gly Ser
           115                 120                 125
   Gly Gly Gly Gly Ser Gly Gly Gly Gly Ser Glu Ile Val Leu Thr Gln
       130                 135                 140
   Ser Pro Ala Thr Leu Ser Leu Ser Pro Gly Glu Arg Ala Thr Leu Ser
   145                 150                 155                 160
   Cys Arg Ala Ser Gln Ser Val Ser Ser Tyr Leu Ala Trp Tyr Gln Gln
                   165                 170                 175
   Lys Pro Gly Gln Ala Pro Arg Leu Leu Ile Tyr Asp Ala Ser Lys Arg
               180                 185                 190
   Ala Thr Gly Ile Pro Ala Arg Phe Ser Gly Ser Gly Ser Gly Thr Asp
           195                 200                 205
   Phe Thr Leu Thr Ile Ser Ser Leu Glu Pro Glu Asp Phe Ala Val Tyr
       210                 215                 220
   Tyr Cys Gln Gln Arg Ser Lys Trp Pro Pro Trp Thr Phe Gly Cys Gly
   225                 230                 235                 240
   Thr Lys Val Glu Ser Lys
                   245
<210> 47
   <211> 10
   <212> PRT
   <213> Homo sapiens
<400> 47
   Asp Lys Thr His Thr Cys Pro Pro Cys Pro
   1               5                   10
<210> 48
   <211> 7
   <212> PRT
   <213> Homo sapiens
<400> 48
   Val Glu Cys Pro Pro Cys Pro
   1               5
<210> 49
   <211> 8
   <212> PRT
   <213> Homo sapiens
<400> 49
   Ala Val Glu Cys Pro Pro Cys Pro
   1               5
<210> 50
   <211> 10
   <212> PRT
   <213> Homo sapiens
<400> 50
   Asp Thr Thr His Thr Cys Pro Arg Cys Pro
   1               5                   10
<210> 51
   <211> 7
   <212> PRT
   <213> Homo sapiens
<400> 51
   Pro Pro Cys Pro Ser Cys Pro
   1 5
<210> 52
   <211> 9
   <212> PRT
   <213> Dengue virus
<400> 52
   Trp Tyr Ala Gln Ile Gln Pro His Trp
   1 5
<210> 53
   <211> 9
   <212> PRT
   <213> Dengue virus
<400> 53
   Ala Phe Ser Gly Val Ser Trp Thr Met
   1 5
<210> 54
   <211> 9
   <212> PRT
   <213> Dengue virus
<400> 54
   Ile Leu Ile Gly Val Val Ile Thr Trp
   1 5
<210> 55
   <211> 9
   <212> PRT
   <213> Dengue virus
<400> 55
   Met Met Ile Pro Thr Val Val Ala Phe
   1 5
<210> 56
   <211> 9
   <212> PRT
   <213> Dengue virus
<400> 56
   Pro Phe Pro Gln Ser Asn Ala Pro Ile
   1 5
<210> 57
   <211> 9
   <212> PRT
   <213> Dengue virus
<400> 57
   Leu Leu Leu Thr Leu Leu Ala Thr Val
   1 5
<210> 58
   <211> 9
   <212> PRT
   <213> Dengue virus
<400> 58
   Ile Val Leu Glu His Gly Ser Cys Val
   1 5
<210> 59
   <211> 9
   <212> PRT
   <213> Dengue virus
<400> 59
   Leu Leu Phe Lys Thr Glu Asn Gly Val
   1 5
<210> 60
   <211> 9
   <212> PRT
   <213> Dengue virus
<400> 60
   Pro Leu Asn Glu Ala Ile Met Ala Val
   1 5
<210> 61
   <211> 9
   <212> PRT
   <213> Dengue virus
<400> 61
   Asn Leu Val Arg Leu Gln Ser Gly Val
   1 5
<210> 62
   <211> 9
   <212> PRT
   <213> Dengue virus
<400> 62
   Leu Val Ile Ser Gly Leu Phe Pro Val
   1 5
<210> 63
   <211> 9
   <212> PRT
   <213> Dengue virus
<400> 63
   Leu Leu Leu Val Ala His Tyr Ala Ile
   1 5
<210> 64
   <211> 9
   <212> PRT
   <213> Dengue virus
<400> 64
   Leu Ala Leu Leu Ala Ala Phe Lys Val
   1 5
<210> 65
   <211> 9
   <212> PRT
   <213> Dengue virus
<400> 65
   Val Ile Leu Ala Gly Pro Met Pro Val
   1 5
<210> 66
   <211> 9
   <212> PRT
   <213> Dengue virus
<400> 66
   His Val Leu Gly Arg Leu Ile Thr Val
   1 5
<210> 67
   <211> 9
   <212> PRT
   <213> Human cytomegalovirus
<400> 67
   Val Thr Glu His Asp Thr Leu Leu Tyr
   1 5
<210> 68
   <211> 9
   <212> PRT
   <213> Human cytomegalovirus
<400> 68
   Asn Thr Asp Phe Arg Val Leu Glu Leu
   1 5
<210> 69
   <211> 9
   <212> PRT
   <213> Human cytomegalovirus
<400> 69
   Cys Val Glu Thr Met Cys Asn Glu Tyr
   1 5
<210> 70
   <211> 9
   <212> PRT
   <213> Human cytomegalovirus
<400> 70
   Val Leu Glu Glu Thr Ser Val Met Leu
   1 5
<210> 71
   <211> 9
   <212> PRT
   <213> Human cytomegalovirus
<400> 71
   Asn Leu Val Pro Met Val Ala Thr Val
   1 5
<210> 72
   <211> 9
   <212> PRT
   <213> Human cytomegalovirus
<400> 72
   Arg Ile Phe Ala Glu Leu Glu Gly Val
   1 5
<210> 73
   <211> 9
   <212> PRT
   <213> Human cytomegalovirus
<400> 73
   Ile Ile Tyr Thr Arg Asn His Glu Val
   1 5
<210> 74
   <211> 9
   <212> PRT
   <213> Human cytomegalovirus
<400> 74
   Val Leu Ala Glu Leu Val Lys Gln Ile
   1               5
<210> 75
   <211> 9
   <212> PRT
   <213> Human cytomegalovirus
<400> 75
   Ala Val Gly Gly Ala Val Ala Ser Val
   1               5
<210> 76
   <211> 9
   <212> PRT
   <213> Human cytomegalovirus
<400> 76
   Thr Val Arg Ser His Cys Val Ser Lys
   1               5
<210> 77
   <211> 9
   <212> PRT
   <213> Human cytomegalovirus
<400> 77
   Ile Met Arg Glu Phe Asn Ser Tyr Lys
   1               5
<210> 78
   <211> 10
   <212> PRT
   <213> Human cytomegalovirus
<400> 78
   Gly Pro Ile Ser His Gly His Val Leu Lys
   1               5                   10
<210> 79
   <211> 9
   <212> PRT
   <213> Human cytomegalovirus
<400> 79
   Ala Thr Val Gln Gly Gln Asn Leu Lys
   1               5
<210> 80
   <211> 9
   <212> PRT
   <213> Human cytomegalovirus
<400> 80
   Val Tyr Ala Leu Pro Leu Lys Met Leu
   1 5
<210> 81
   <211> 10
   <212> PRT
   <213> Human cytomegalovirus
<400> 81
   Ala Tyr Ala Gln Lys Ile Phe Lys Ile Leu
   1 5 10
<210> 82
   <211> 9
   <212> PRT
   <213> Human cytomegalovirus
<400> 82
   Gln Tyr Asp Pro Val Ala Ala Leu Phe
   1 5
<210> 83
   <211> 9
   <212> PRT
   <213> Human cytomegalovirus
<400> 83
   Tyr Val Lys Val Tyr Leu Glu Ser Phe
   1 5
<210> 84
   <211> 9
   <212> PRT
   <213> Human cytomegalovirus
<400> 84
   Asp Ile Tyr Arg Ile Phe Ala Glu Leu
   1 5
<210> 85
   <211> 10
   <212> PRT
   <213> Human cytomegalovirus
<400> 85
   Val Phe Glu Thr Ser Gly Gly Leu Val Val
   1 5 10
<210> 86
   <211> 9
   <212> PRT
   <213> Human cytomegalovirus
<400> 86
   Lys Ala Arg Asp His Leu Ala Val Leu
   1               5
<210> 87
   <211> 9
   <212> PRT
   <213> Human cytomegalovirus
<400> 87
   Gln Ala Arg Leu Thr Val Ser Gly Leu
   1               5
<210> 88
   <211> 9
   <212> PRT
   <213> Human cytomegalovirus
<400> 88
   Lys Ala Arg Ala Lys Lys Asp Glu Leu
   1               5
<210> 89
   <211> 9
   <212> PRT
   <213> Human cytomegalovirus
<400> 89
   Gln Ile Lys Val Arg Val Asp Met Val
   1               5
<210> 90
   <211> 9
   <212> PRT
   <213> Human cytomegalovirus
<400> 90
   Arg Arg Arg His Arg Gln Asp Ala Leu
   1               5
<210> 91
   <211> 9
   <212> PRT
   <213> Human cytomegalovirus
<400> 91
   Ala Arg Val Tyr Glu Ile Lys Cys Arg
   1               5
<210> 92
   <211> 9
   <212> PRT
   <213> Human cytomegalovirus
<400> 92
   Lys Met Gln Val Ile Gly Asp Gln Tyr
   1               5
<210> 93
   <211> 9
   <212> PRT
   <213> Human cytomegalovirus
<400> 93
   Asn Val Arg Arg Ser Trp Glu Glu Leu
   1               5
<210> 94
   <211> 12
   <212> PRT
   <213> Human cytomegalovirus
<400> 94
   Cys Pro Ser Gln Glu Pro Met Ser Ile Tyr Val Tyr
   1               5                   10
<210> 95
   <211> 13
   <212> PRT
   <213> Human cytomegalovirus
<400> 95
   Lys Pro Gly Lys Ile Ser His Ile Met Leu Asp Val Ala
   1               5                   10
<210> 96
   <211> 9
   <212> PRT
   <213> Human cytomegalovirus
<400> 96
   Glu Leu Arg Arg Lys Met Met Tyr Met
   1               5
<210> 97
   <211> 9
   <212> PRT
   <213> Human cytomegalovirus
<400> 97
   Ile Pro Ser Ile Asn Val His His Tyr
   1 5
<210> 98
   <211> 9
   <212> PRT
   <213> Human cytomegalovirus
<400> 98
   Phe Glu Gln Pro Thr Glu Thr Pro Pro
   1 5
<210> 99
   <211> 9
   <212> PRT
   <213> Human cytomegalovirus
<400> 99
   Tyr Ala Tyr Ile Tyr Thr Thr Tyr Leu
   1 5
<210> 100
   <211> 11
   <212> PRT
   <213> Human cytomegalovirus
<400> 100
   Gln Glu Phe Phe Trp Asp Ala Asn Asp Ile Tyr
   1 5 10
<210> 101
   <211> 9
   <212> PRT
   <213> Human cytomegalovirus
<400> 101
   Tyr Glu Gln His Lys Ile Thr Ser Tyr
   1 5
<210> 102
   <211> 9
   <212> PRT
   <213> Human cytomegalovirus
<400> 102
   Gln Glu Pro Met Ser Ile Tyr Val Tyr
   1 5
<210> 103
   <211> 10
   <212> PRT
   <213> Human cytomegalovirus
<400> 103
   Ser Glu His Pro Thr Phe Thr Ser Gln Tyr
   1 5 10
<210> 104
   <211> 9
   <212> PRT
   <213> Human cytomegalovirus
<400> 104
   Gln Ala Ile Arg Glu Thr Val Glu Leu
   1 5
<210> 105
   <211> 9
   <212> PRT
   <213> Human cytomegalovirus
<400> 105
   Thr Arg Ala Thr Lys Met Gln Val Ile
   1 5
<210> 106
   <211> 8
   <212> PRT
   <213> Human cytomegalovirus
<400> 106
   Asp Ala Leu Pro Gly Pro Cys Ile
   1 5
<210> 107
   <211> 9
   <212> PRT
   <213> Human cytomegalovirus
<400> 107
   Cys Glu Asp Val Pro Ser Gly Lys Leu
   1 5
<210> 108
   <211> 9
   <212> PRT
   <213> Human cytomegalovirus
<400> 108
   His Glu Arg Asn Gly Phe Thr Val Leu
   1 5
<210> 109
   <211> 13
   <212> PRT
   <213> Human cytomegalovirus
<400> 109
   Pro Thr Phe Thr Ser Gln Tyr Arg Ile Gln Gly Lys Leu
   1               5                   10
<210> 110
   <211> 9
   <212> PRT
   <213> Human cytomegalovirus
<400> 110
   Gln Met Trp Gln Ala Arg Leu Thr Val
   1               5
<210> 111
   <211> 12
   <212> PRT
   <213> Human cytomegalovirus
<400> 111
   His Glu Leu Leu Val Leu Val Lys Lys Ala Gln Leu
   1               5                   10
<210> 112
   <211> 12
   <212> PRT
   <213> Human cytomegalovirus
<400> 112
   Asp Asp Tyr Ser Asn Thr His Ser Thr Arg Tyr Val
   1               5                   10